# EUROPEAN PATENT APPLICATION

(11) **EP 4 293 027 A1**
(43) Date of publication of application: **20.12.2023**
(21) Application number: 22752307.3
(22) Date of filing: 09.02.2022
(51) Int. Cl.: C07D 519/00, A61K 31/55, A61K 31/439, A61P 35/00

(54) **PYRIMIDINE AROMATIC RING COMPOUNDS**

(30) Priority: 09.02.2021 CN 202110182357; 08.03.2021 CN 202110251656; 08.04.2021 CN 202110379326; 30.04.2021 CN 202110485837; 21.07.2021 CN 202110825879; 24.08.2021 CN 202110975205; 27.09.2021 CN 202111136266; 01.11.2021 CN 202111283561; 21.01.2022 CN 202210072243; 29.01.2022 CN 202210113080
(71) Applicant: Medshine Discovery Inc., Nanjing, Jiangsu 210032 (CN)
(72) Inventor: ZHANG, Yang, Shanghai 200131 (CN); WU, Wentao, Shanghai 200131 (CN); GENG, Kaijun, Shanghai 200131 (CN); XU, Yangyang, Shanghai 200131 (CN); LI, Zhixiang, Shanghai 200131 (CN); CHEN, Shuhui, Shanghai 200131 (CN)
(74) Representative: D Young & Co LLP
(86) International application number: PCT/CN2022/075732
(87) International publication number: WO 2022/171147

(57) **Abstract**

Provided are pyrimidine aromatic ring compounds. Specifically provided is a compound as represented by formula (II) or a pharmaceutically acceptable salt thereof.

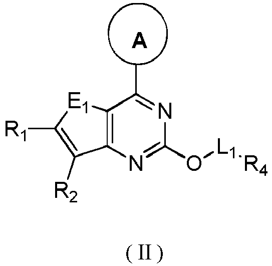

## Description

This application claims the priority of:
CN202110182357.6, filed on February 9, 2021;
CN202110251656.0, filed on March 8, 2021;
CN202110379326.X, filed on April 8, 2021;
CN202110485837.X, filed on April 30, 2021;
CN202110825879.3, filed on July 21, 2021;
CN202110975205.1, filed on August 24, 2021;
CN202111136266.5, filed on September 27, 2021;
CN202111283561.3, filed on November 01, 2021;
CN202210072243.0, filed on January 21, 2022;
CN202210113080.6, filed on January 29, 2022.

### FIELD OF THE INVENTION

The present disclosure relates to a class of pyrimidoaromatic ring compounds, in particular to a compound represented by formula (II) or a pharmaceutically acceptable salt thereof.

### BACKGROUND OF THE INVENTION

RAS oncogene mutations are the most common activating mutations in human cancers, and RAS mutations are present in about 30% of human tumors. The RAS gene family includes three subtypes (KRAS, HRAS, and NRAS), of which 85% of RAS-driven cancers are caused by mutations in the KRAS subtype.

KRAS is a rat sarcoma viral oncogene and an important member of the RAS protein. KRAS is like a molecular switch, once it is turned on, it will activate a variety of division and proliferation factors, such as c-RAF, PI3K and so on. Under normal circumstances, KRAS binds to GTP, and cuts off one phosphate group at the end of GTP to turn GTP into GDP. After GTP is turned into GDP, KRAS is closed. Under normal circumstances, KRAS can regulate the path of cell growth; after KRAS gene mutation, KRAS protein continues to remain activated, and can independently transmit growth and proliferation signals to downstream pathways independent of upstream growth factor receptor signals, resulting in uncontrolled cell growth and tumor progression.

KRAS mutations are commonly found in solid tumors such as lung adenocarcinoma, pancreatic ductal carcinoma, and colorectal cancer, etc. In KRAS-mutated tumors, 80% of oncogenic mutations occur at codon 12, with the most common mutations including: p.G12D (41%), p.G12V (28%), and p.G12C (14%). At the same time, whether the KRAS gene has mutation or not is also an important indicator of tumor prognosis.

At present, small molecules that directly target KRAS mutations are mainly focused in the field of KRAS^{G12C}, including Amgen's AMG510 and Mirati Therapeutics' MRTX849. Clinical results show that these two compounds have shown good therapeutic effects on patients with KRAS^{G12C}-mutated tumor. However, no small molecule targeting KRAS^{G12D} has entered a clinical research stage so far, and there is a huge unmet need for inhibitors of KRAS^{G12D} mutation in clinical practice.

### SUMMARY OF THE INVENTION

The present disclosure provides a compound represented by formula (II) or a pharmaceutically acceptable salt thereof 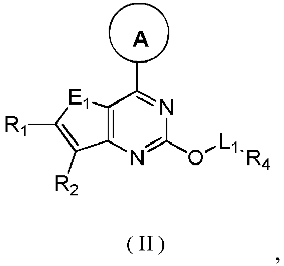 wherein
E₁ is selected from S and -CR₃ = CH-;
L₁ is selected from -CH₂- and a bond;
Ring A is selected from 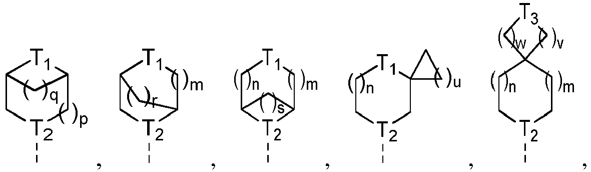 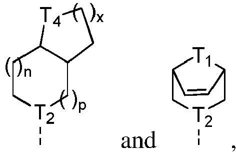 wherein the 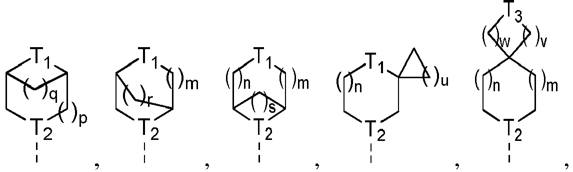 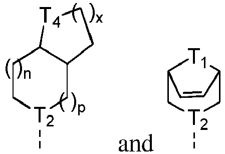 are optionally substituted with 1, 2 or 3 Rₐ;
T₁ is selected from CH₂, NH and O;
T₂ is selected from CH and N;
T₃ and T₄ are each independently selected from CH₂ and NH;
m, n, p and x are each independently selected from 0, 1 and 2;
r, v and w are each independently selected from 1 and 2;
q, s and u are each independently selected from 1, 2 and 3;
R₁ is selected from C₆₋₁₀ aryl and 5- to 10-membered heteroaryl, wherein the C₆₋₁₀ aryl and 5- to 10-membered heteroaryl are optionally substituted with 1, 2, 3, 4 or 5 R_{b};
R₂ is selected from H, F, Cl, CN, NH₂, C₁₋₃ alkyl and C₁₋₃ alkoxy, wherein the C₁₋₃ alkyl and C₁₋₃ alkoxy are optionally substituted with 1, 2 or 3 halogens;
R₃ is selected from H, F, Cl, C₁₋₃ alkyl, C₁₋₃ alkoxy, C₂₋₄ alkenyl and cyclopropyl, wherein the C₁₋₃ alkyl, C₁₋₃ alkoxy, C₂₋₄ alkenyl and cyclopropyl are optionally substituted with 1, 2 or 3 halogens;
R₄ is selected from 4- to 8-membered heterocycloalkyl and 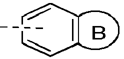 , wherein the 4- to 8-membered heterocycloalkyl and 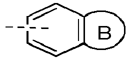 are optionally substituted with 1, 2 or 3 Rₑ;
the structural moiety 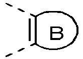 is 5- to 6-membered heterocycloalkenyl;
each Rₐ is independently selected from F, Cl, Br, I and CH₃;
each R_{b} is independently selected from F, Cl, Br, I, OH, NH₂, CN, C₁₋₃ alkyl, C₁₋₃ alkoxy, C₂₋₄ alkenyl and C₂₋₄ alkynyl, wherein the C₁₋₃ alkyl, C₁₋₃ alkoxy, C₂₋₄ alkenyl and C₂₋₄ alkynyl are optionally substituted with 1, 2 or 3 halogens;
each Rₑ is independently selected from H, F, Cl, Br, OH, CN, C₁₋₃ alkyl, C₁₋₃ alkoxy and -C₁₋₃ alkyl-O-CO-C₁₋₃ alkylamino.

In some embodiments of the present disclosure, disclosed is the compound or a pharmaceutically acceptable salt thereof, wherein Ring A is selected from 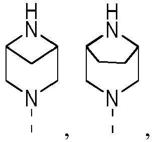 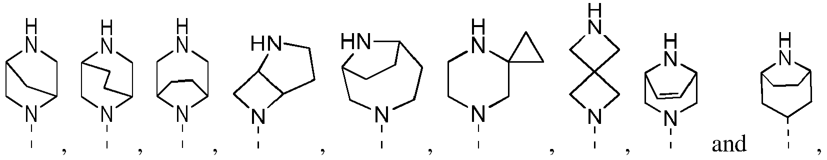 wherein the 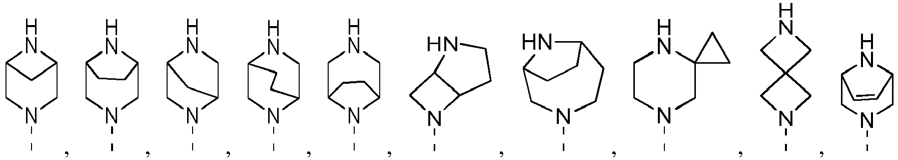 and 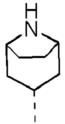 are optionally substituted with 1, 2 or 3 Rₐ, and other variables are as defined herein.

In some embodiments of the present disclosure, disclosed is the compound or a pharmaceutically acceptable salt thereof, wherein Ring A is selected from 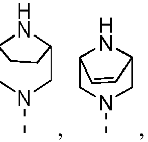 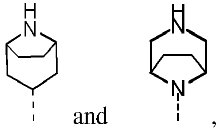 and other variables are as defined herein.

In some embodiments of the present disclosure, disclosed is the compound or a pharmaceutically acceptable salt thereof, wherein each R_{b} is independently selected from F, Cl, Br, I, OH, NH₂, CN, CH₃, CH₂CH₃, OCH₃, OCH₂CH₃, -CH = CH₂, -CH₂-CH = CH₂ and -C≡CH, wherein the CH₃, CH₂CH₃, OCH₃, OCH₂CH₃, -CH = CH₂, -CH₂-CH = CH₂ and -C≡CH are optionally substituted with 1, 2 or 3 halogens, and other variables are as defined herein.

In some embodiments of the present disclosure, the each R_{b} is independently selected from F, Cl, OH, NH₂, CN, CH₃, CF₃, CH₂CH₃ and -C≡CH , and other variables are as defined herein.

In some embodiments of the present disclosure, the R₁ is selected from phenyl, pyridyl, naphthyl, quinolyl, benzothiazolyl and benzothienyl, wherein the phenyl, pyridyl, naphthyl, quinolyl, benzothiazolyl and benzothienyl are optionally substituted with 1, 2, 3, 4 or 5 R_{b}, and other variables are as defined herein.

In some embodiments of the present disclosure, the R₁ is selected from 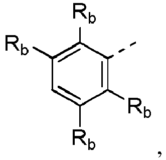 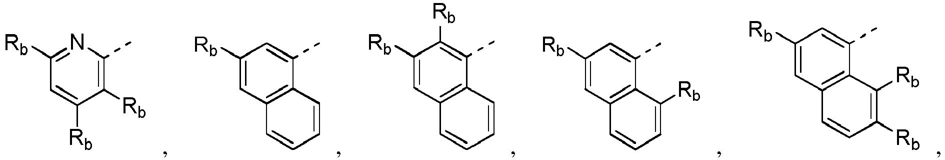 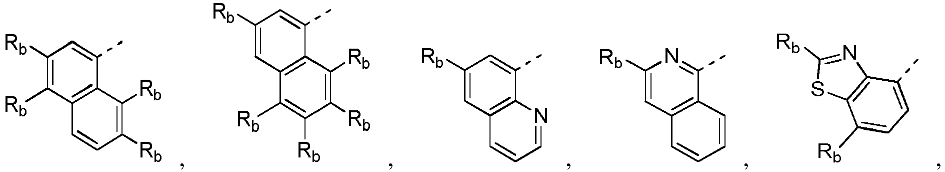 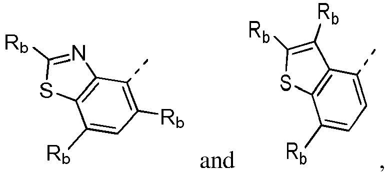 and other variables are as defined herein.

In some embodiments of the present disclosure, the R₁ is selected from 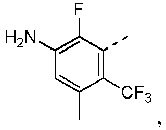 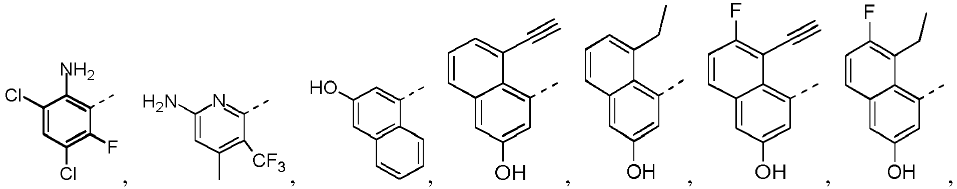 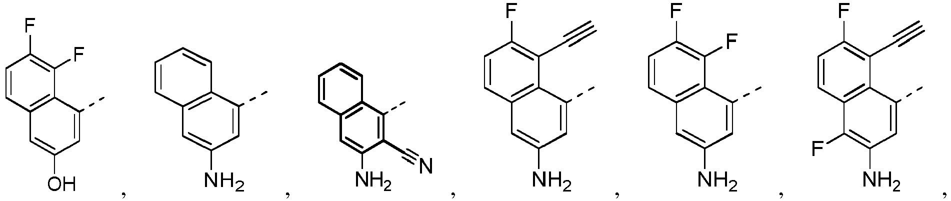 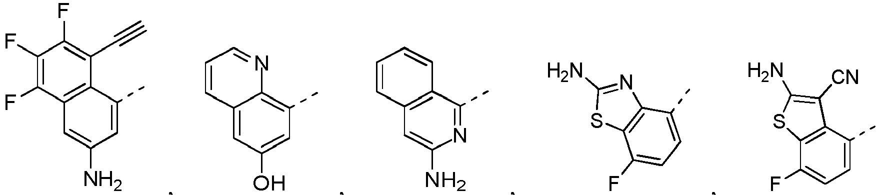 and 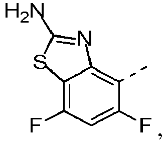 and other variables are as defined herein.

In some embodiments of the present disclosure, the R₂ is selected from H, F, Cl, CH₃ and OCH₃, wherein the CH₃ and OCH₃ are optionally substituted with 1, 2 or 3 halogens, and other variables are as defined herein.

In some embodiments of the present disclosure, the R₂ is selected from H, F, Cl, OCH₃ and OCHF₂, and other variables are as defined herein.

In some embodiments of the present disclosure, the R₃ is selected from H, F, Cl, CH₃, OCH₃, -CH = CH₂ and cyclopropyl, wherein the CH₃, OCH₃, -CH = CH₂ and cyclopropyl are optionally substituted with 1, 2 or 3 halogens, and other variables are as defined herein.

In some embodiments of the present disclosure, the R₃ is selected from H, F, Cl, OCHF₂, -CH = CH₂ and cyclopropyl, and other variables are as defined herein.

In some embodiments of the present disclosure, the each Rₑ is independently selected from H, F, Cl, Br, OH, CN, CH₃, CH₂CH₃, OCH₃ and 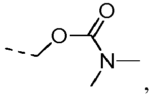 and other variables are as defined herein.

In some embodiments of the present disclosure, the R₄ is selected from tetrahydropyrrolyl and hexahydro-1H-pyrrolizinyl, wherein the tetrahydropyrrolyl and hexahydro-1H-pyrrolizinyl are substituted with 1, 2 or 3 Rₑ, and other variables are as defined herein.

In some embodiments of the present disclosure, the R₄ is selected from 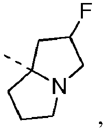 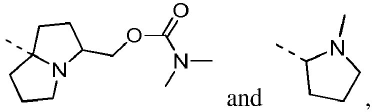 and other variables are as defined herein.

The present disclosure provides a compound represented by formula (II) or a pharmaceutically acceptable salt thereof 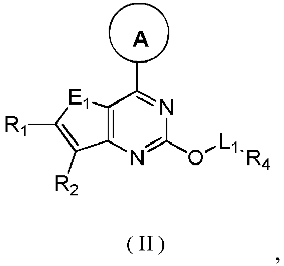 wherein
E₁ is selected from S and -CR₃ = CH-;
L₁ is selected from -CH₂- and a bond;
Ring A is selected from 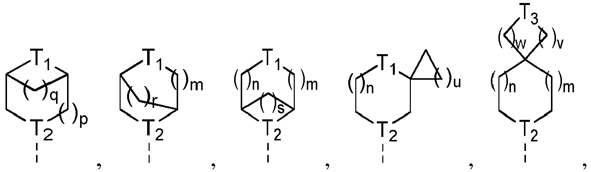 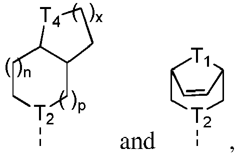 wherein the 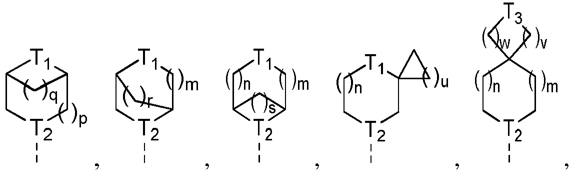 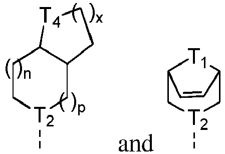 are optionally substituted with 1, 2 or 3 Rₐ;
T₁ is selected from CH₂, NH and O;
T₂ is selected from CH and N;
T₃ and T₄ are each independently selected from CH₂ and NH;
m, n, p and x are each independently selected from 0, 1 and 2;
r, v and w are each independently selected from 1 and 2;
q, s and u are each independently selected from 1, 2 and 3;
R₁ is selected from C₆₋₁₀ aryl and 5- to 10-membered heteroaryl, wherein the C₆₋₁₀ aryl and 5- to 10-membered heteroaryl are optionally substituted with 1, 2, 3, 4 or 5 R_{b};
R₂ is selected from H, F, Cl, CN, NH₂, C₁₋₃ alkyl and C₁₋₃ alkoxy, wherein the C₁₋₃ alkyl and C₁₋₃ alkoxy are optionally substituted with 1, 2 or 3 halogens;
R₃ is selected from H, F, Cl, C₁₋₃ alkyl, C₁₋₃ alkoxy, C₂₋₄ alkenyl and cyclopropyl, wherein the C₁₋₃ alkyl, C₁₋₃ alkoxy, C₂₋₄ alkenyl and cyclopropyl are optionally substituted with 1, 2 or 3 halogens;
R₄ is selected from 4- to 8-membered heterocycloalkyl and 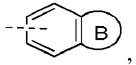 wherein the 4- to 8-membered heterocycloalkyl and 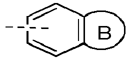 are optionally substituted with 1, 2 or 3 Rₑ;
the structural moiety 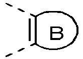 is 5- to 6-membered heterocycloalkenyl;
each Rₐ is independently selected from F, Cl, Br, I and CH₃;
each R_{b} is independently selected from F, Cl, Br, I, OH, NH₂, CN, C₁₋₃ alkyl, C₁₋₃ alkoxy, C₂₋₄ alkenyl and C₂₋₄ alkynyl, wherein the C₁₋₃ alkyl, C₁₋₃ alkoxy, C₂₋₄ alkenyl and C₂₋₄ alkynyl are optionally substituted with 1, 2 or 3 halogens;
each Rₑ is independently selected from H, F, Cl, Br, OH, CN, C₁₋₃ alkyl, C₁₋₃ alkoxy and -C₁₋₃ alkyl-O-CO-C₁₋₃ alkylamino.

In some embodiments of the present disclosure, disclosed is the compound or a pharmaceutically acceptable salt thereof, wherein Ring A is selected from 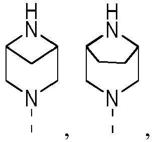 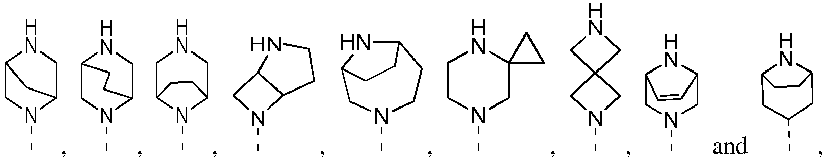 wherein the 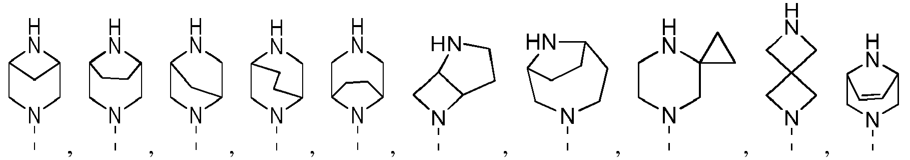 and 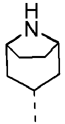 are optionally substituted with 1, 2 or 3 Rₐ, and other variables are as defined herein.

In some embodiments of the present disclosure, disclosed is the compound or a pharmaceutically acceptable salt thereof, wherein Ring A is selected from 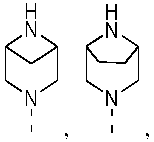 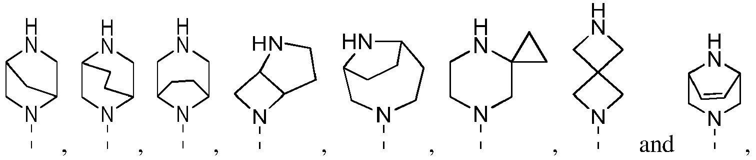 wherein the 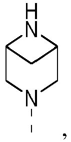 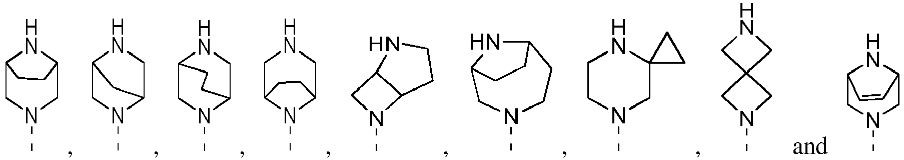 are optionally substituted with 1, 2 or 3 Rₐ, and other variables are as defined herein.

In some embodiments of the present disclosure, disclosed is the compound or a pharmaceutically acceptable salt thereof, wherein Ring A is selected from 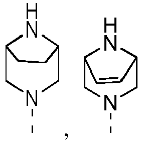 and 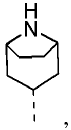 and other variables are as defined herein.

In some embodiments of the present disclosure, disclosed is the compound or a pharmaceutically acceptable salt thereof, wherein Ring A is selected from 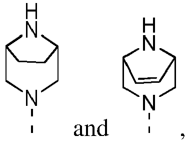 and other variables are as defined herein.

In some embodiments of the present disclosure, disclosed is the compound or a pharmaceutically acceptable salt thereof, wherein each R_{b} is independently selected from F, Cl, Br, I, OH, NH₂, CN, CH₃, CH₂CH₃, OCH₃, OCH₂CH₃, -CH = CH₂, -CH₂-CH = CH₂ and -C≡CH, wherein the CH₃, CH₂CH₃, OCH₃, OCH₂CH₃, -CH = CH₂, -CH₂-CH = CH₂ and -C≡CH are optionally substituted with 1, 2 or 3 halogens, and other variables are as defined herein.

In some embodiments of the present disclosure, the each R_{b} is independently selected from F, OH, NH₂, CN, CH₃, CF₃, CH₂CH₃ and -C≡CH, and other variables are as defined herein.

In some embodiments of the present disclosure, the R₁ is selected from phenyl, naphthyl, benzothiazolyl and benzothienyl, wherein the phenyl, naphthyl, benzothiazolyl and benzothienyl are optionally substituted with 1, 2, 3, 4 or 5 R_{b}, and other variables are as defined herein.

In some embodiments of the present disclosure, the R₁ is selected from 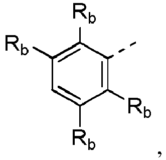 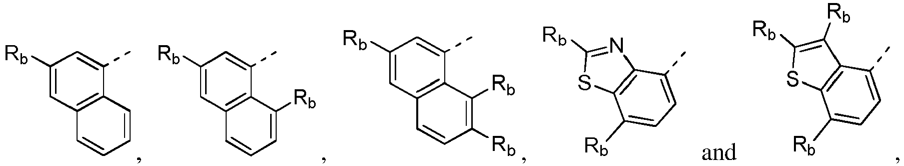 and other variables are as defined herein.

In some embodiments of the present disclosure, the R₁ is selected from 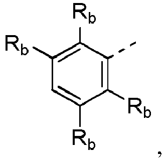 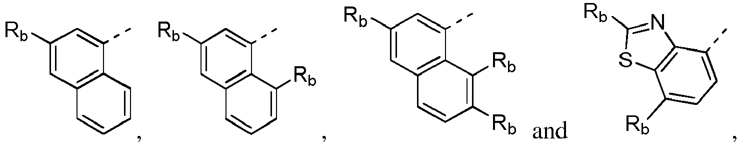 and other variables are as defined herein.

In some embodiments of the present disclosure, the R₁ is selected from 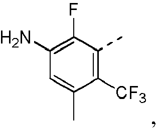 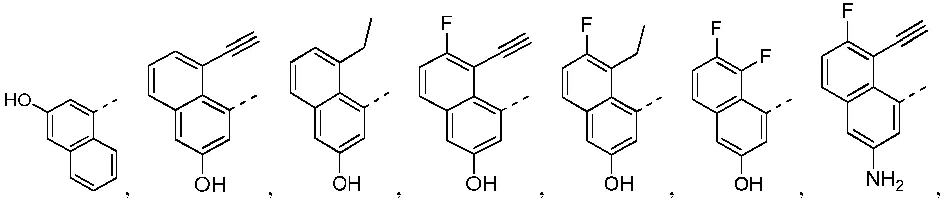 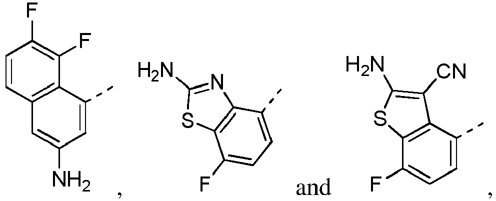 and other variables are as defined herein.

In some embodiments of the present disclosure, the R₁ is selected from 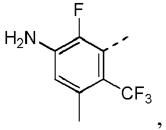 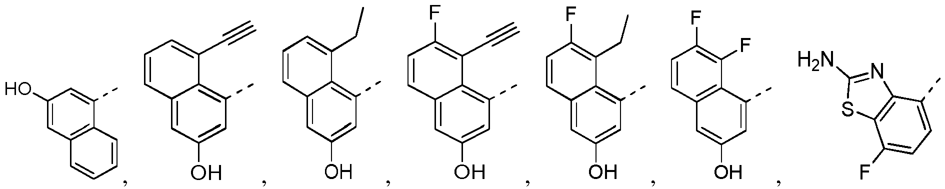 and 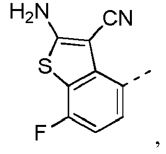 and other variables are as defined herein.

In some embodiments of the present disclosure, the R₁ is selected from 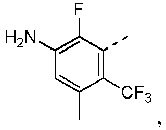 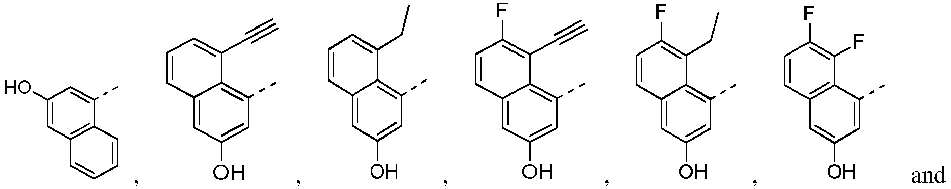 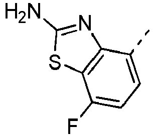 , and other variables are as defined herein.

In some embodiments of the present disclosure, the R₂ is selected from H, F, Cl, CH₃ and OCH₃, wherein the CH₃ and OCH₃ are optionally substituted with 1, 2 or 3 halogens, and other variables are as defined herein.

In some embodiments of the present disclosure, the R₂ is selected from H, F, Cl, OCH₃ and OCHF₂, and other variables are as defined herein.

In some embodiments of the present disclosure, the R₃ is selected from H, F, Cl, CH₃, OCH₃, -CH = CH₂ and cyclopropyl, wherein the CH₃, OCH₃, -CH = CH₂ and cyclopropyl are optionally substituted with 1, 2 or 3 halogens, and other variables are as defined herein.

In some embodiments of the present disclosure, the R₃ is selected from H, Cl, OCHF₂, -CH = CH₂ and cyclopropyl, and other variables are as defined herein.

In some embodiments of the present disclosure, the each Rₑ is independently selected from H, F, Cl, Br, OH, CN, CH₃, CH₂CH₃, OCH₃ and 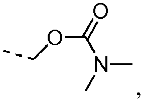 and other variables are as defined herein.

In some embodiments of the present disclosure, the R₄ is selected from tetrahydropyrrolyl and hexahydro-1H-pyrrolizinyl, wherein the tetrahydropyrrolyl and hexahydro-1H-pyrrolizinyl are substituted with 1, 2 or 3 Rₑ, and other variables are as defined herein.

In some embodiments of the present disclosure, the R₄ is selected from 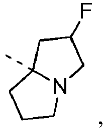 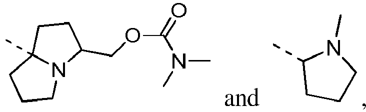 and other variables are as defined herein.

The present disclosure provides a compound represented by formula (II) or a pharmaceutically acceptable salt thereof 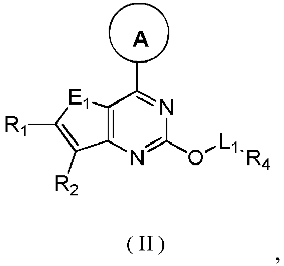 wherein
E₁ is selected from S and -CR₃ = CH-;
L₁ is selected from -CH₂- and a bond;
Ring A is selected from 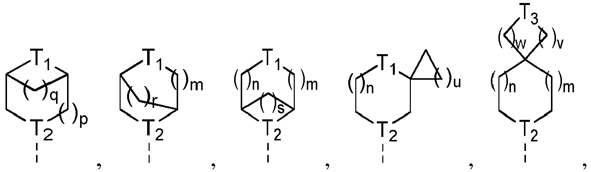 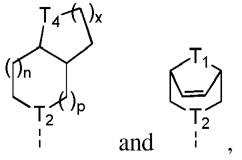 wherein the 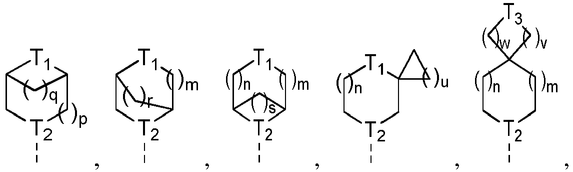 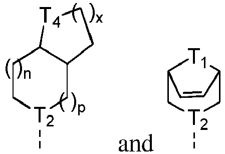 are optionally substituted with 1, 2 or 3 Rₐ;
T₁ is selected from CH₂, NH and O;
T₂ is selected from CH and N;
T₃ and T₄ are each independently selected from CH₂ and NH;
m, n, p and x are each independently selected from 0, 1 and 2;
r, v and w are each independently selected from 1 and 2;
q, s and u are each independently selected from 1, 2 and 3;
R₁ is selected from C₆₋₁₀ aryl and 5- to 10-membered heteroaryl, wherein the C₆₋₁₀ aryl and 5- to 10-membered heteroaryl are optionally substituted with 1, 2, 3, 4 or 5 R_{b};
R₂ is selected from H, F, Cl, CN, NH₂, C₁₋₃ alkyl and C₁₋₃ alkoxy, wherein the C₁₋₃ alkyl and C₁₋₃ alkoxy are optionally substituted with 1, 2, or 3 R_{c};
R₃ is selected from H, F, Cl, C₁₋₃ alkyl, C₁₋₃ alkoxy, C₂₋₄ alkenyl and cyclopropyl, wherein the C₁₋₃ alkyl, C₁₋₃ alkoxy, C₂₋₄ alkenyl and cyclopropyl are optionally substituted with 1, 2 or 3 R_{d};
R₄ is selected from 4- to 8-membered heterocycloalkyl and 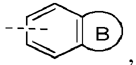 wherein the 4- to 8-membered heterocycloalkyl and 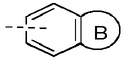 are optionally substituted with 1, 2 or 3 Rₑ;
the structural moiety 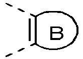 is 5- to 6-membered heterocycloalkenyl;
each Rₐ is independently selected from F, Cl, Br, I and CH₃;
each R_{b} is independently selected from F, Cl, Br, I, OH, NH₂, CN, C₁₋₃ alkyl, C₁₋₃ alkoxy, C₂₋₄ alkenyl and C₂₋₄ alkynyl, wherein the C₁₋₃ alkyl, C₁₋₃ alkoxy, C₂₋₄ alkenyl and C₂₋₄ alkynyl are optionally substituted with 1, 2 or 3 R;
each R_{c} is independently selected from F, Cl, Br and I;
each R_{d} is independently selected from F, Cl, Br and I;
each Rₑ is independently selected from H, F, Cl, Br, OH, CN, C₁₋₃ alkyl, C₁₋₃ alkoxy and -C₁₋₃ alkyl-O-CO-C₁₋₃ alkylamino;
each R is independently selected from F, Cl, Br, and I.

In some embodiments of the present disclosure, disclosed is the compound or a pharmaceutically acceptable salt thereof, wherein Ring A is selected from 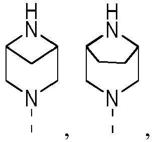 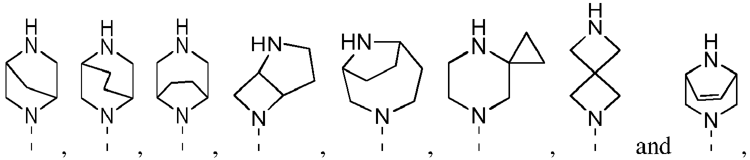 wherein the 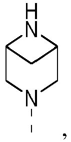 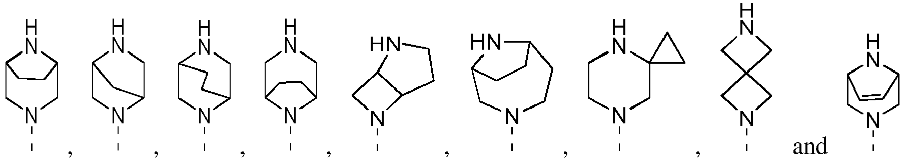 are optionally substituted with 1, 2 or 3 Rₐ, and other variables are as defined herein.

In some embodiments of the present disclosure, disclosed is the compound or a pharmaceutically acceptable salt thereof, wherein Ring A is selected from 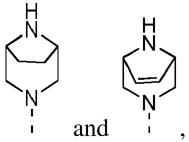 and other variables are as defined herein.

In some embodiments of the present disclosure, disclosed is the compound or a pharmaceutically acceptable salt thereof, wherein each R_{b} is independently selected from F, Cl, Br, I, OH, NH₂, CN, CH₃, CH₂CH₃, OCH₃, OCH₂CH₃, -CH = CH₂, -CH₂-CH = CH₂ and -C≡CH, wherein the CH₃, CH₂CH₃, OCH₃, OCH₂CH₃, -CH = CH₂, -CH₂-CH = CH₂ and -C≡CH are optionally substituted with 1, 2 or 3 R, and other variables are as defined herein.

In some embodiments of the present disclosure, the each R_{b} is independently selected from F, OH, NH₂, CH₃, CF₃, CH₂CH₃ and -C≡CH, and other variables are as defined herein.

In some embodiments of the present disclosure, the R₁ is selected from 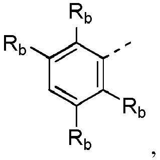 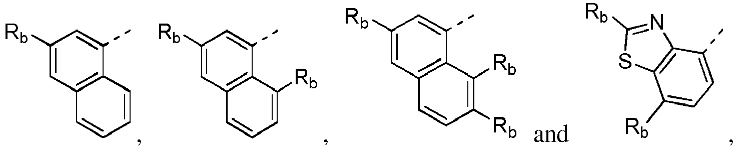 and other variables are as defined herein.

In some embodiments of the present disclosure, the R₁ is selected from 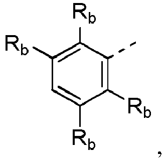 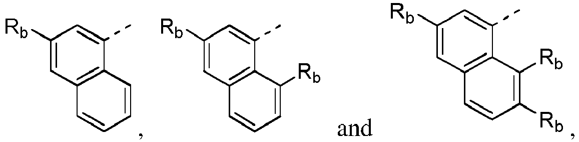 and other variables are as defined herein.

In some embodiments of the present disclosure, the R₁ is selected from 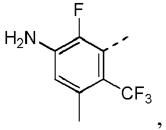 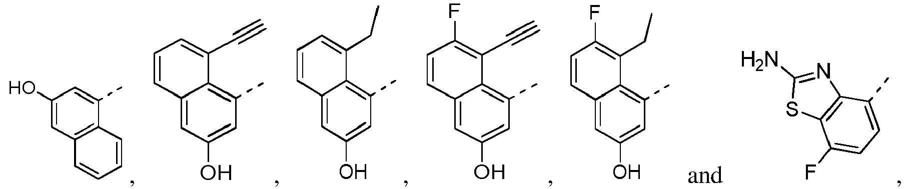 and other variables are as defined herein.

In some embodiments of the present disclosure, the R₁ is selected from 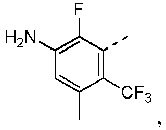 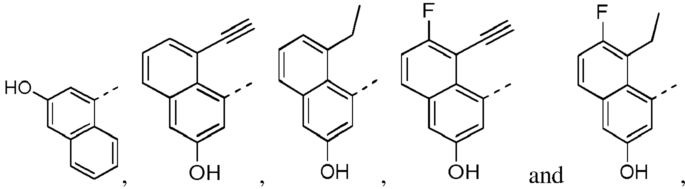 and other variables are as defined herein.

In some embodiments of the present disclosure, the R₂ is selected from H, F, CH₃ and OCH₃, wherein the CH₃ and OCH₃ are optionally substituted with 1, 2, or 3 R_{c}, and other variables are as defined herein.

In some embodiments of the present disclosure, the R₂ is selected from H, F, OCH₃ and OCHF₂, and other variables are as defined herein.

In some embodiments of the present disclosure, the R₃ is selected from H, F, Cl, CH₃, OCH₃, -CH = CH₂ and cyclopropyl, wherein the CH₃, OCH₃, -CH = CH₂ and cyclopropyl are optionally substituted with 1, 2 or 3 R_{d}, and other variables are as defined herein.

In some embodiments of the present disclosure, the R₃ is selected from H, Cl, OCHF₂, -CH = CH₂ and cyclopropyl, and other variables are as defined herein.

In some embodiments of the present disclosure, the each Rₑ is independently selected from H, F, Cl, Br, OH, CN, CH₃, CH₂CH₃, CH₂CF₃, OCH₃, OCF₃ and 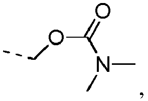 and other variables are as defined herein.

In some embodiments of the present disclosure, the each Rₑ is independently selected from H, F, Cl, Br, OH, CN, CH₃, CH₂CH₃, OCH₃ and 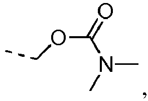 and other variables are as defined herein.

In some embodiments of the present disclosure, the R₄ is selected from tetrahydropyrrolyl and hexahydro-1H-pyrrolizinyl, wherein the tetrahydropyrrolyl and hexahydro-1H-pyrrolizinyl are substituted with 1, 2 or 3 Rₑ, and other variables are as defined herein.

In some embodiments of the present disclosure, the R₄ is selected from 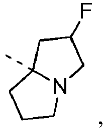 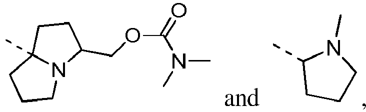 and other variables are as defined herein.

The present disclosure provides a compound represented by formula (I) or a pharmaceutically acceptable salt thereof 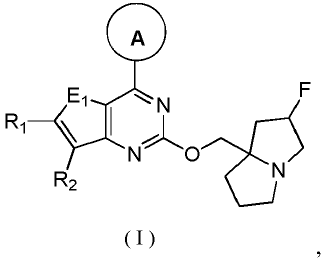 wherein
E₁ is selected from S and -CR₃ = CH-;
Ring A is selected from 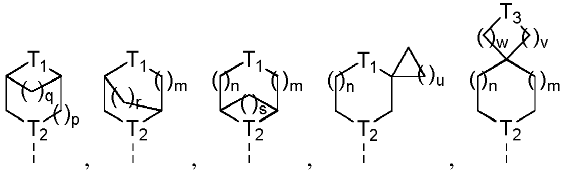 and 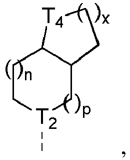 wherein the 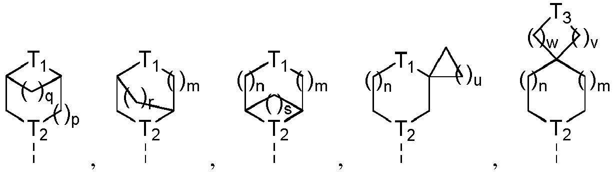 and 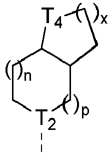 are optionally substituted with 1, 2 or 3 Rₐ;
T₁, T₂, T₃ and T₄ are each independently selected from CH and N;
m, n, p and x are each independently selected from 0, 1 and 2;
r, v and w are each independently selected from 1 and 2;
q, s and u are each independently selected from 1, 2 and 3;
R₁ is selected from phenyl and naphthyl, wherein the phenyl and naphthyl are optionally substituted with 1, 2, 3, 4 or 5 R_{b};
R₂ is selected from H, F, Cl, CN, NH₂, C₁₋₃ alkyl and C₁₋₃ alkoxy, wherein the C₁₋₃ alkyl and C₁₋₃ alkoxy are optionally substituted with 1, 2, or 3 R_{c};
R₃ is selected from H, F, Cl, C₁₋₃ alkyl, C₁₋₃ alkoxy, C₂₋₄ alkenyl and cyclopropyl, wherein the C₁₋₃ alkyl, C₁₋₃ alkoxy, C₂₋₄ alkenyl and cyclopropyl are optionally substituted with 1, 2 or 3 R_{d};
each Rₐ is independently selected from F, Cl, Br, I and CH₃;
each R_{b} is independently selected from F, Cl, Br, I, OH, NH₂, CN, CH₃, CF₃ and OCH₃;
each R_{c} is independently selected from F, Cl, Br and I;
each R_{d} is independently selected from F, Cl, Br and I.

In some embodiments of the present disclosure, disclosed is the compound or a pharmaceutically acceptable salt thereof, wherein Ring A is selected from 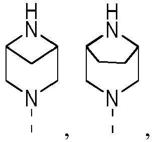 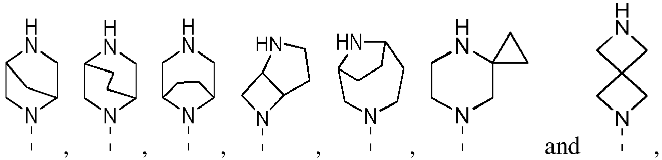 wherein the 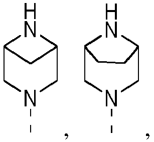 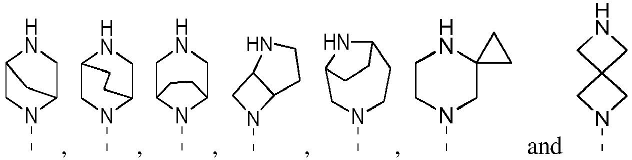 are optionally substituted with 1, 2 or 3 Rₐ, and other variables are as defined herein.

In some embodiments of the present disclosure, disclosed is the compound or a pharmaceutically acceptable salt thereof, wherein Ring A is 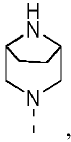 and other variables are as defined herein.

In some embodiments of the present disclosure, the R₁ is selected from 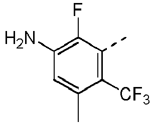 and 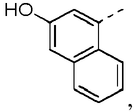 and other variables are as defined herein.

In some embodiments of the present disclosure, the R₂ is selected from H, F, CH₃ and OCH₃, wherein the CH₃ and OCH₃ are optionally substituted with 1, 2, or 3 R_{c}, and other variables are as defined herein.

In some embodiments of the present disclosure, the R₂ is selected from H, F, OCH₃ and OCHF₂, and other variables are as defined herein.

In some embodiments of the present disclosure, the R₃ is selected from H, F, Cl, CH₃, OCH₃, -CH = CH₂ and cyclopropyl, wherein the CH₃, OCH₃, -CH = CH₂ and cyclopropyl are optionally substituted with 1, 2 or 3 R_{d}, and other variables are as defined herein.

In some embodiments of the present disclosure, the R₃ is selected from H, Cl, OCHF₂, -CH = CH₂ and cyclopropyl, and other variables are as defined herein.

In some embodiments of the present disclosure, disclosed is the compound or a pharmaceutically acceptable salt thereof, wherein the compound is selected from 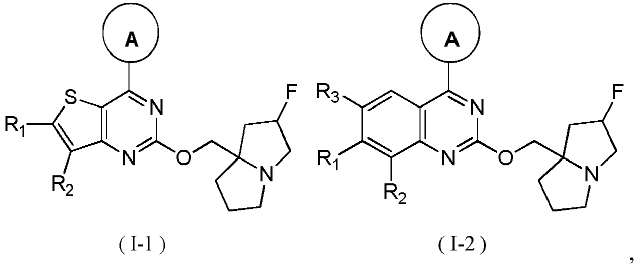 wherein
Ring A, R₁, R₂ and R₃ are as defined herein.

In some embodiments of the present disclosure, disclosed is the compound or a pharmaceutically acceptable salt thereof, wherein the compound is selected from 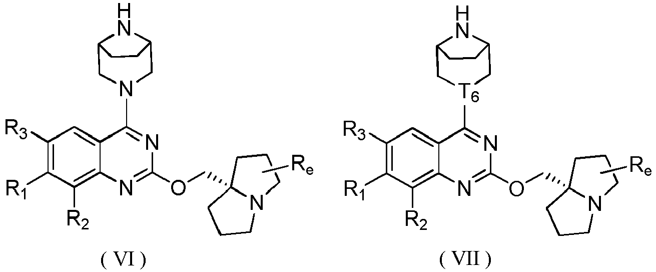 wherein
T₆ is selected from CH and N;
R₁, R₂, R₃ and Rₑ are as defined herein.

In some embodiments of the present disclosure, disclosed is the compound or a pharmaceutically acceptable salt thereof, wherein the compound is selected from 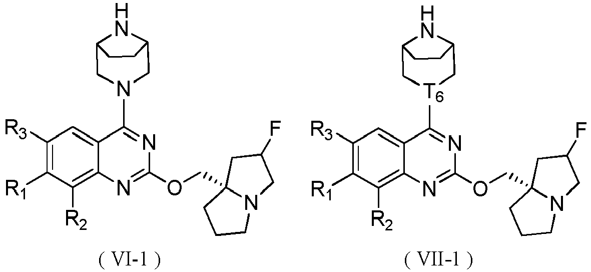 wherein T₆, R₁, R₂ and R₃ are as defined herein.

In some embodiments of the present disclosure, disclosed is the compound or a pharmaceutically acceptable salt thereof, wherein the compound is selected from 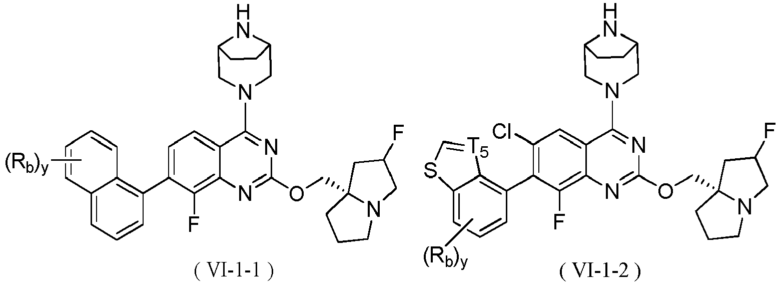 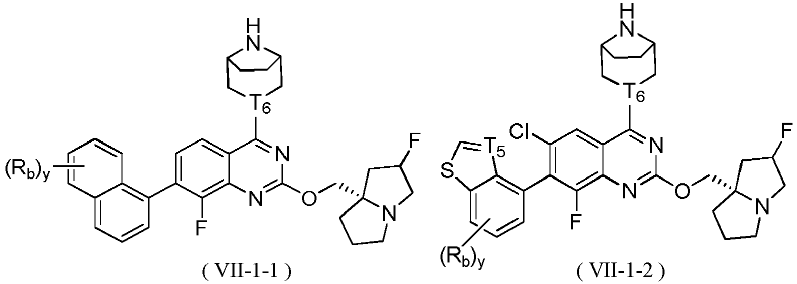 wherein
T₅ and T₆ are each independently selected from CH and N;
y is selected from 0, 1, 2, 3, 4 and 5;
R_{b} is as defined herein.

In some embodiments of the present disclosure, disclosed is the compound or a pharmaceutically acceptable salt thereof, wherein the compound is selected from 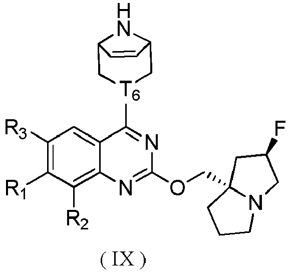 wherein
T₆ is selected from CH and N;
R₁, R₂ and R₃ are as defined herein.

In some embodiments of the present disclosure, disclosed is the compound or a pharmaceutically acceptable salt thereof, wherein the compound is selected from 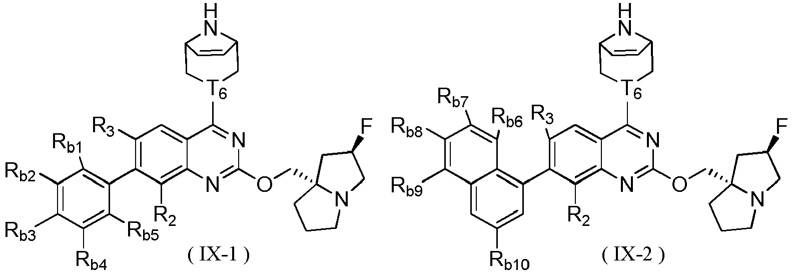 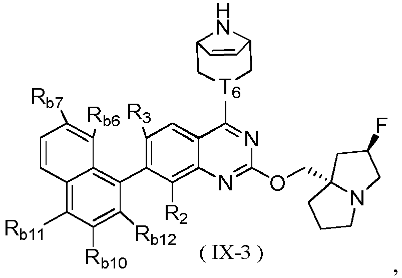 wherein
T₆ is selected from CH and N;
R₂ and R₃ are as defined herein.
each R_{b1}, R_{b2}, R_{b3}, R_{b4}, R_{b5}, R_{b6}, R_{b7}, R_{b8}, R_{b9}, R_{b10} and R_{b11} are each independently selected from H, F, Cl, Br, I, OH, NH₂, CN, C₁₋₃ alkyl, C₁₋₃ alkoxy, C₂₋₄ alkenyl and C₂₋₄ alkynyl, wherein the C₁₋₃ alkyl, C₁₋₃ alkoxy, C₂₋₄ alkenyl and C₂₋₄ alkynyl are optionally substituted with 1, 2 or 3 halogens.

In some embodiments of the present disclosure, the R_{b1}, R_{b2}, R_{b3}, R_{b4}, R_{b5}, R_{b6}, R_{b7}, R_{b8}, R_{b9}, R_{b10} and R_{b11} are each independently selected from F, Cl, Br, I, OH, NH₂, CN, CH₃, CH₂CH₃, OCH₃, OCH₂CH₃, -CH = CH₂, -CH₂-CH = CH₂ and -C≡CH, wherein the CH₃, CH₂CH₃, OCH₃, OCH₂CH₃, -CH = CH₂, -CH₂-CH = CH₂ and -C≡CH are optionally substituted with 1, 2 or 3 halogens, and other variables are as defined herein.

In some embodiments of the present disclosure, the R_{b1}, R_{b2}, R_{b3}, R_{b4}, R_{b5}, R_{b6}, R_{b7}, R_{b8}, R_{b9}, R_{b10} and R_{b11} are each independently selected from F, Cl, OH, NH₂, CN, CH₃, CF₃, CH₂CH₃ and -C≡CH, and other variables are as defined herein.

The present disclosure also includes some embodiments obtained by any combination of the above variables.

The present disclosure also provides a compound of the following formula or a pharmaceutically acceptable salt thereof, wherein the compound is selected from

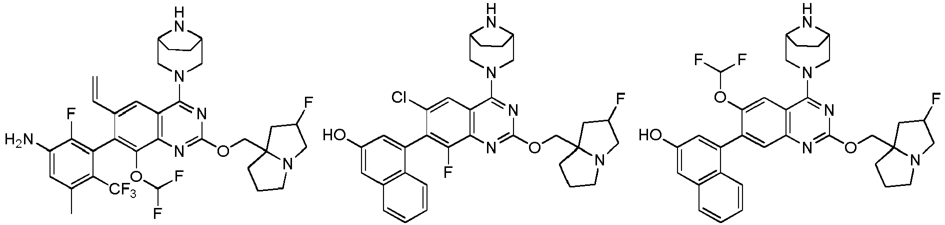

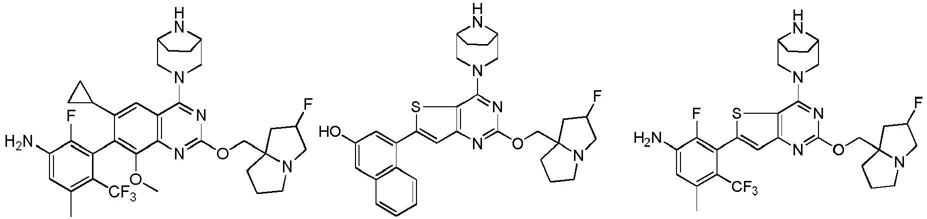

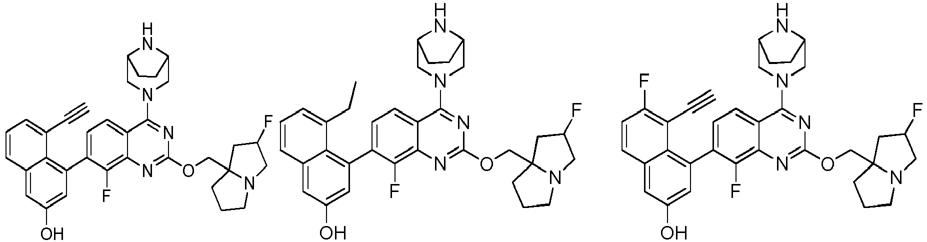

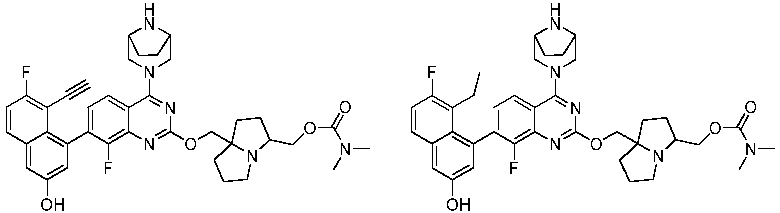

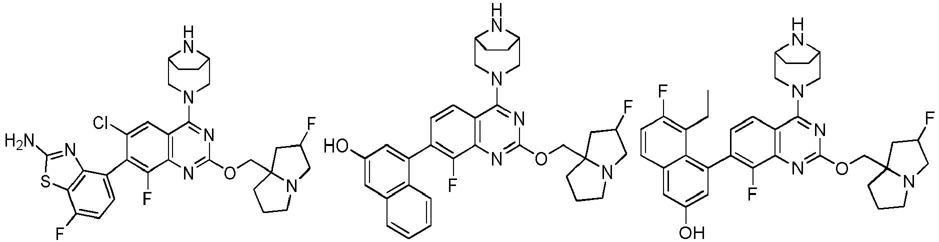

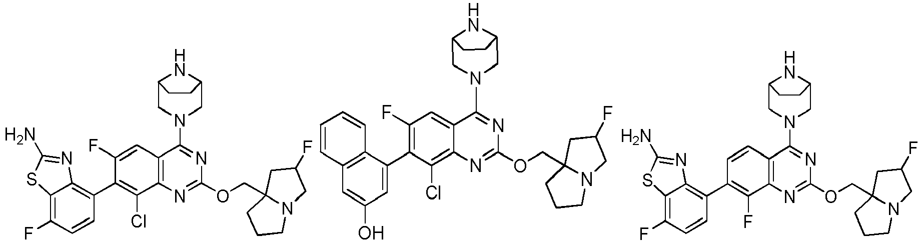

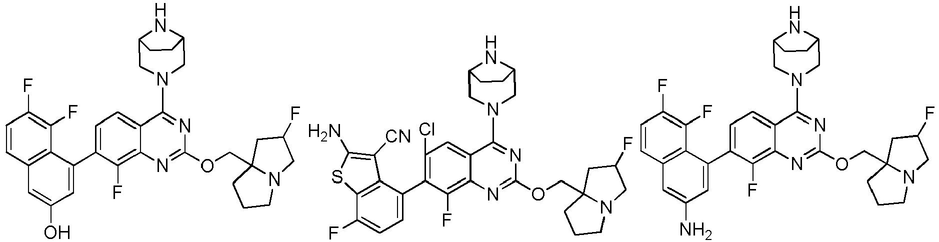

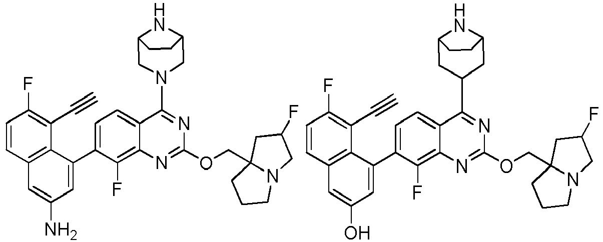

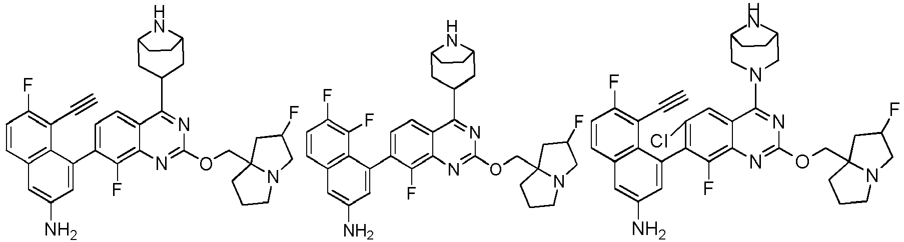

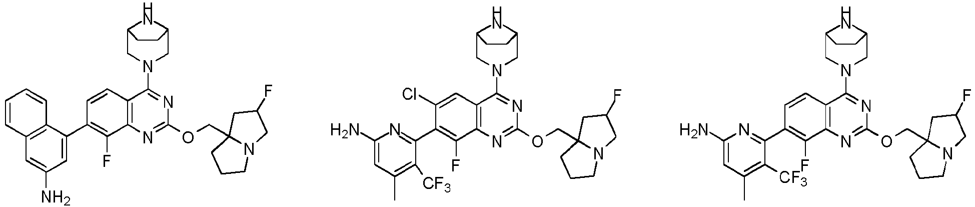

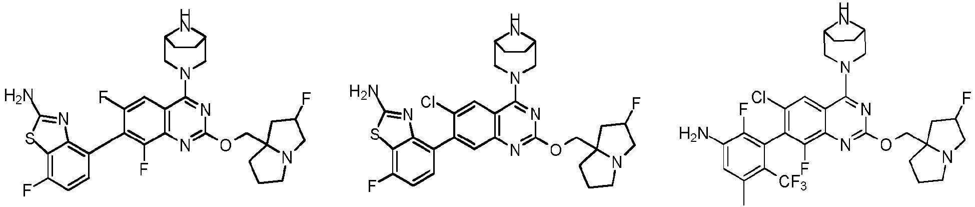

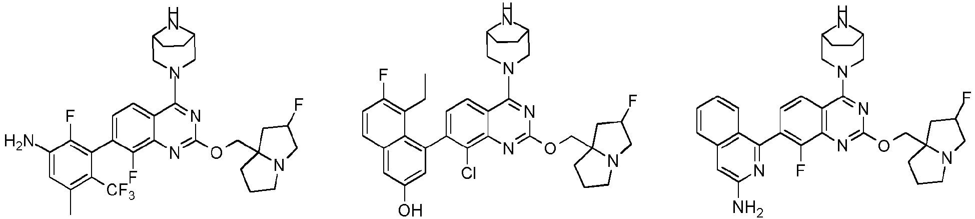

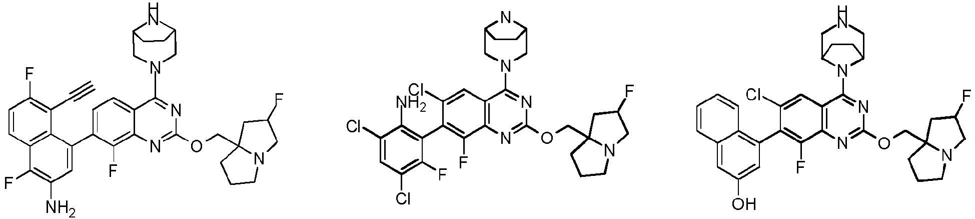

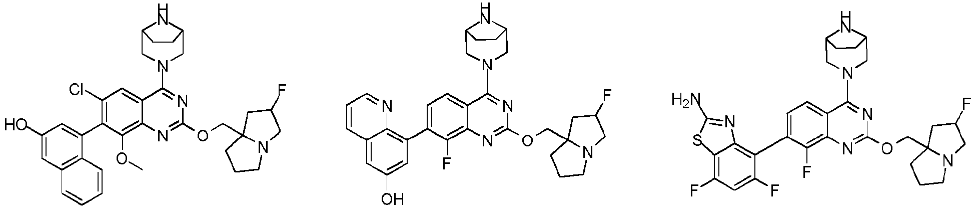

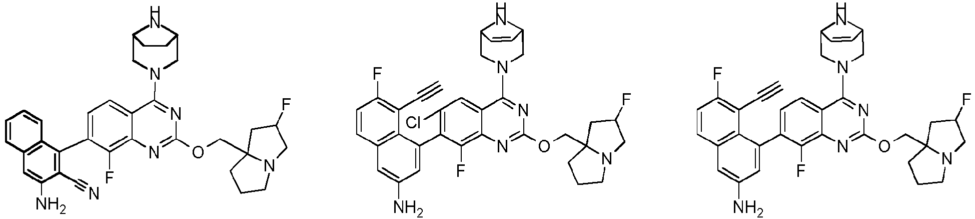

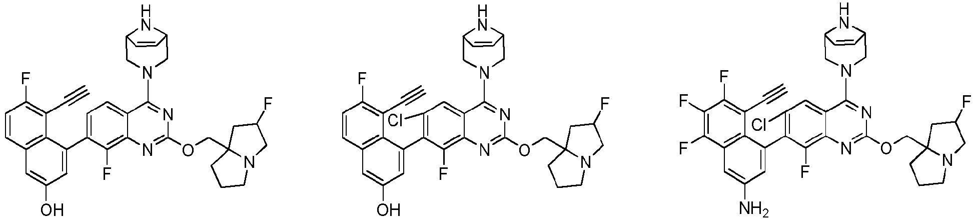

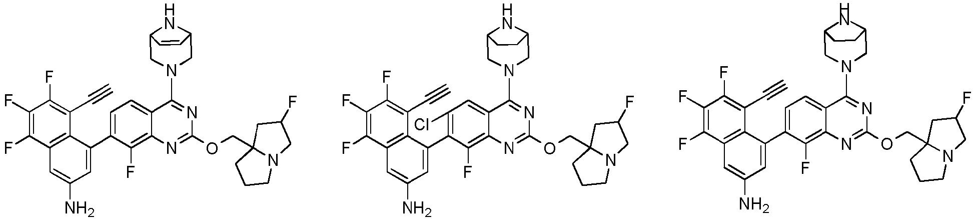

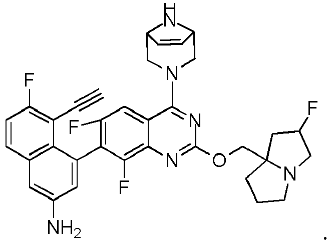

In some embodiments of the present disclosure, disclosed is the compound or a pharmaceutically acceptable salt thereof, wherein the compound is selected from

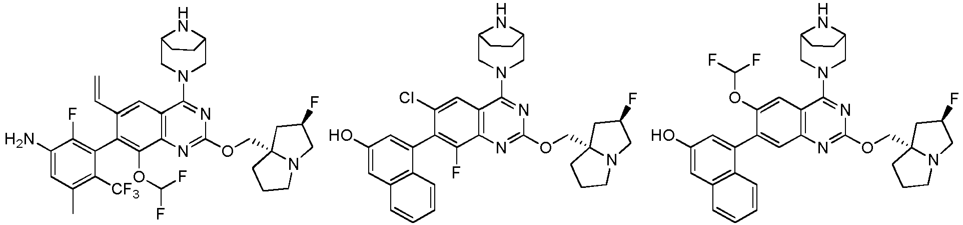

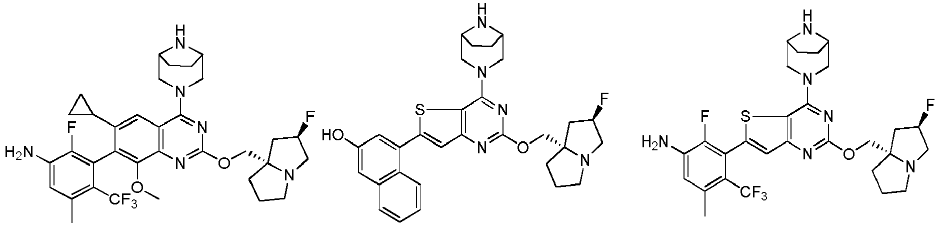

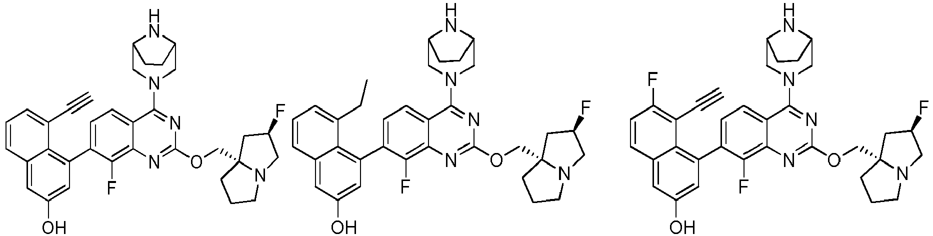

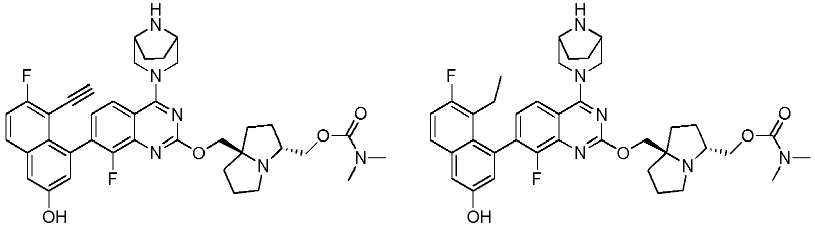

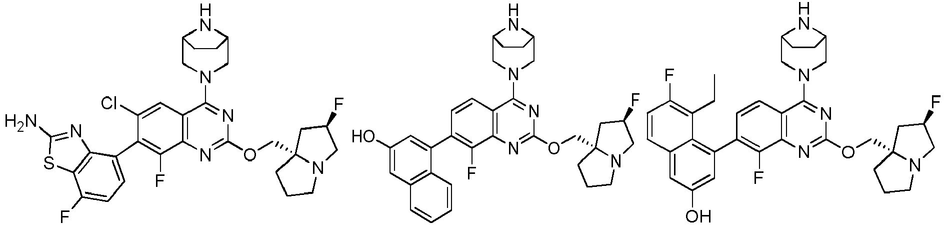

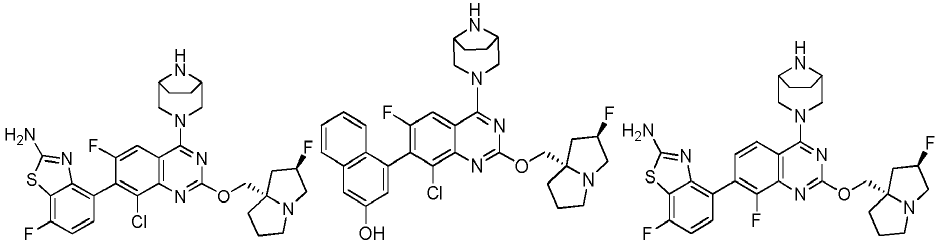

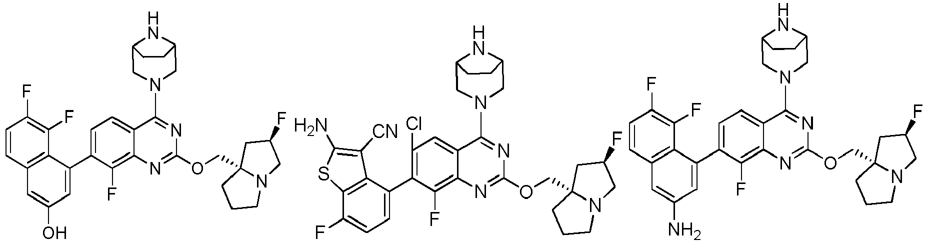

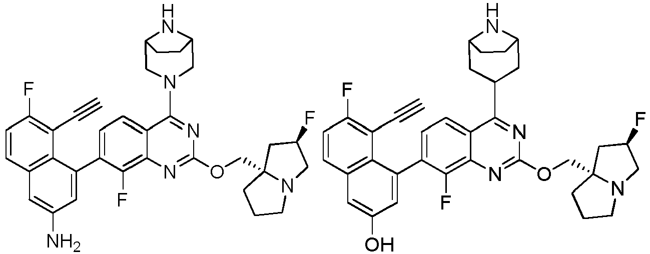

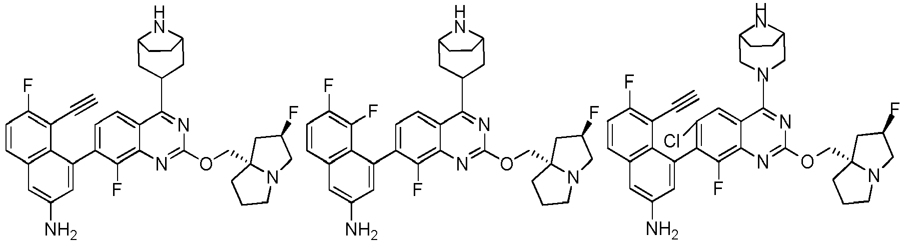

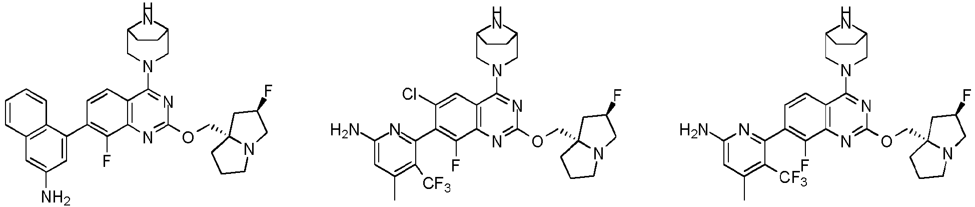

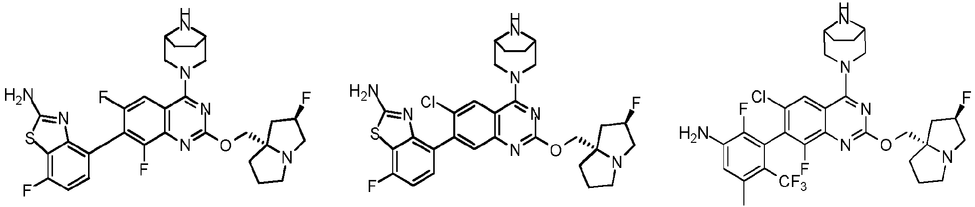

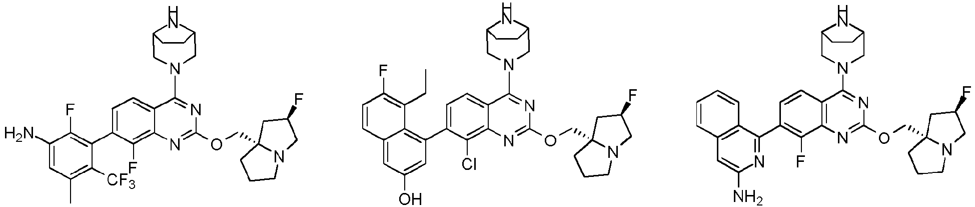

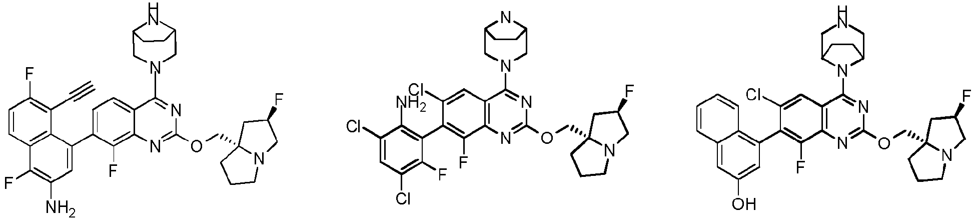

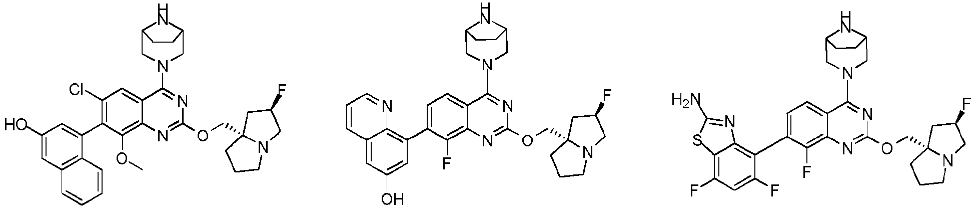

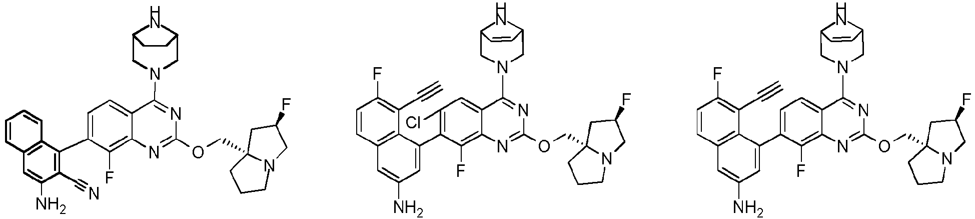

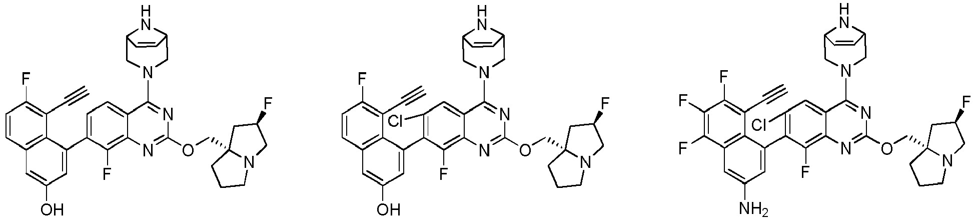

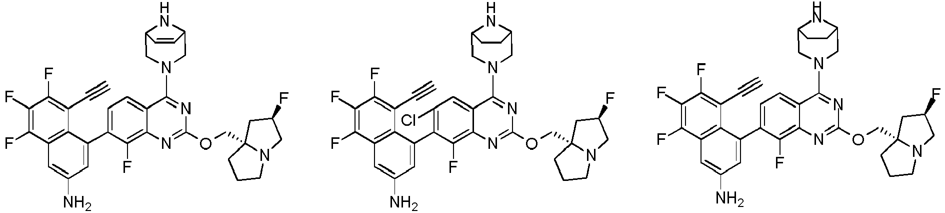

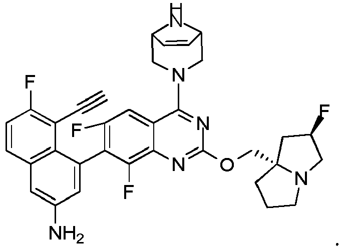

In some embodiments of the present disclosure, disclosed is the compound or a pharmaceutically acceptable salt thereof, wherein the compound is selected from

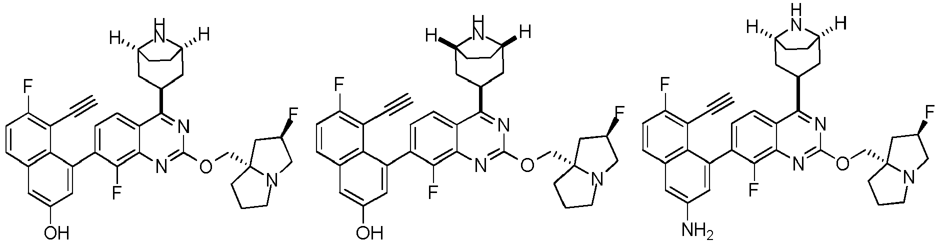

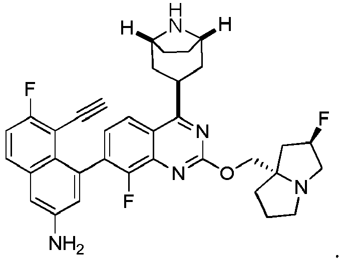

The present disclosure also provides use of the compound or a pharmaceutically acceptable salt thereof in the manufacture of a medicament for treating KRAS^{G12D} mutationrelated tumors.

In some embodiments of the present disclosure, the tumors refer to colorectal cancer and pancreatic cancer.

The present disclosure also provides the following synthetic methods:
Method 1: 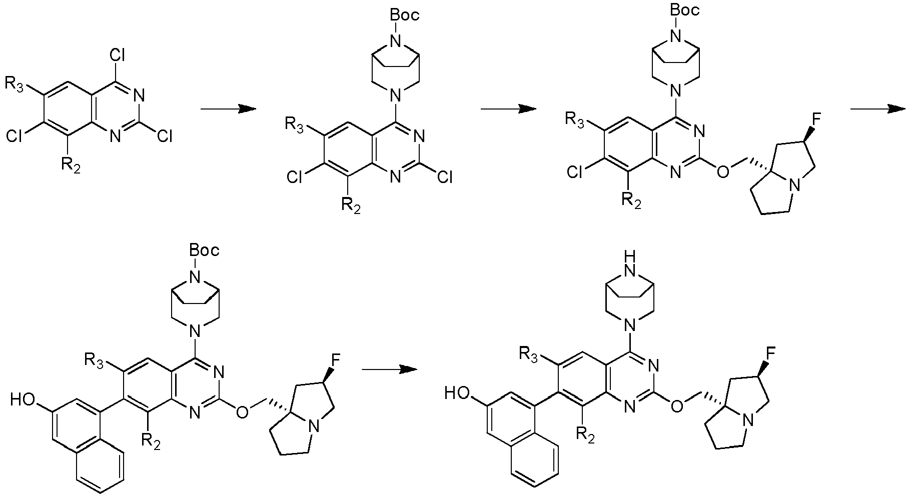 wherein R₂ and R₃ are as defined herein.
Method 2: 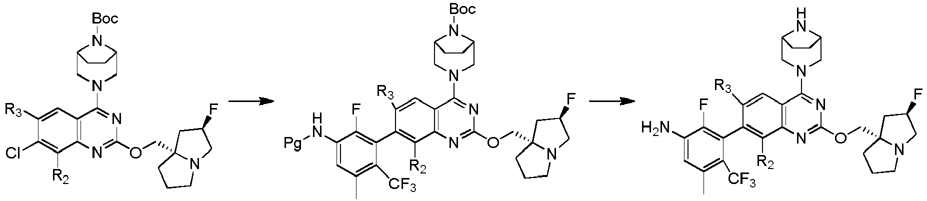 wherein R₂ and R₃ are as defined herein.
Method 3: 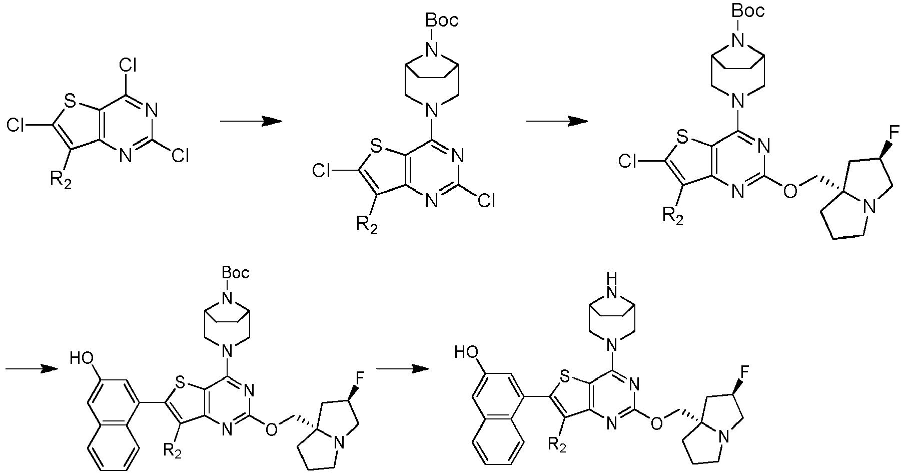 wherein R₂ is as defined herein.
Method 4: 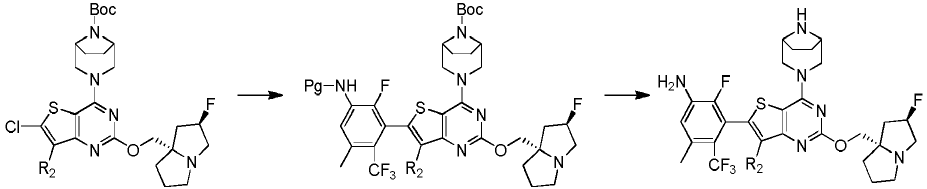 wherein R₂ is as defined herein.

### Assay method 2. Anti-cell proliferation effects of compounds in tumor cell lines AsPC-1 and GP2D

### Research purpose

In this assay, the inhibitory effect of the compound on cell proliferation was studied by detecting the effect of the compound on the *in vitro* cell activity in the tumor cell lines AsPC-1 and GP2D.

### Assay materials

**Table 1. Assay materials**

| Cell line | Tumor type | Growth characteristics | Culture method |
|---|---|---|---|
| AsPC-1 | Pancreatic cancer | Adherent growth | RPMI 1640 + 10% FBS |
| GP2D | Colon cancer | Adherent growth | DMEM + 10% FBS+ 2 mM L-glutamine |

Ultra Low Cluster-96-well plate (Corning-7007)
Greiner CELLSTAR 96-well plate (# 655090)
Promega CellTiter-Glo 3D Luminescence Cell Viability Assay Kit (Promega-G9683)
2104-10 EnVision plate reader, PerkinElmer
RPMI 1640, DMEM, PBS (phosphate buffered saline), FBS (fetal bovine serum), Antibiotic-antimycotic, L-glutamine, DMSO (dimethyl sulfoxide)
Assay method and step

### Cell culture

The tumor cell lines were cultured in a 37°C, 5% CO2 incubator according to the culture conditions indicated in the culture method. The cells were periodically passaged, and cells in logarithmic growth phase were taken for plating.

### Cell plating

Cells were stained with trypan blue and viable cells were counted.

The cell concentration was adjusted to an appropriate concentration.

**Table 2. Cell density**

| Cell line | Density (per well) |
|---|---|
| AsPC-1 | 7000 cells |
| GP2D | 8000 cells |

135 µL of cell suspension was added to each well of the ULA culture plate, and the same volume of culture medium without cells was added to the blank control well.

Immediately after plating, the ULA plate was centrifuged at 1000 rpm for 10 minutes at room temperature. Note: After centrifugation, be careful not to cause unnecessary shocks in subsequent operations.

The plate was incubated overnight in an incubator at 37°C, 5% CO₂, and 100% relative humidity.

### Preparation of 10X compound working solution and treatment of cells with compounds (day 1)

After preparing the 10X compound working solution (10X working solution in DMSO), 15 µL of the 10X compound working solution was added to the ULA culture plate respectively, and 15 µL of DMSO-cell culture solution mixture was added to the vehicle control and blank control.

The 96-well cell plate was placed back into the incubator and cultured for 120 hours.

Cell spheroidization was observed every day until the end of the assay. CellTiter-Glo Luminescence Assay for Cell Viability (Day 5)

The following steps were performed according to the instructions of the Promega CellTiter-Glo 3D Luminescence Cell Viability Assay Kit (Promega #G9683).

150 µL (equivalent to the volume of cell medium in each well) of CellTiter-Glo 3D reagent was added to each well. The cell plate was wrapped in aluminum foil to be protected from light.

The culture plate was shaken on an orbital shaker for 5 minutes.

The mixture in wells was mixed well by carefully pipetting up and down 10 times. Ensure that the cell spheres were sufficiently detached before proceeding to the next step.

The solution in the ULA culture plate was then transferred to a black bottom culture plate (#655090) and left at room temperature for 25 minutes to stabilize the luminescence signal. Luminescence signals were detected on a 2104EnVision plate reader.

### Data analysis

The inhibition rate (IR) of the assayed compound was calculated by the following formula: IR (%) = (1 - (RLU of compound - RLU of blank control) / (RLU of vehicle control - RLU of blank control)) * 100%. The inhibition rates of different concentrations of compounds were calculated in Excel, and then the GraphPad Prism software was used to plot the inhibition curves and calculate the relevant parameters, including the minimum inhibition rate, the maximum inhibition rate, and IC₅₀.

### Technical Effect

The compounds of the present disclosure have good binding effect and inhibitory effect on KRAS^{G12D} protein, can effectively inhibit the downstream signal p-ERK, have good cell proliferation inhibitory activity on KRAS^{G12D} mutated cells, and have a significant inhibitory effect on tumors. In addition, the compounds of the present disclosure have good pharmacokinetic properties.

### Related definitions

Unless otherwise specified, the following terms and phrases used herein are intended to have the following meanings. A specific term or phrase should not be considered indefinite or unclear in the absence of a particular definition, but should be understood in the conventional sense. When a trade name appears herein, it is intended to refer to its corresponding commodity or active ingredient thereof.

The term "pharmaceutically acceptable" is used herein in terms of those compounds, materials, compositions, and/or dosage forms, which are suitable for use in contact with human and animal tissues within the scope of reliable medical judgment, with no excessive toxicity, irritation, allergic reaction or other problems or complications, commensurate with a reasonable benefit/risk ratio.

The term "pharmaceutically acceptable salt" means a salt of compounds disclosed herein that is prepared by reacting the compound having a specific substituent disclosed herein with a relatively non-toxic acid or base. When compounds disclosed herein contain a relatively acidic functional group, a base addition salt can be obtained by bringing the compound into contact with a sufficient amount of base in a pure solution or a suitable inert solvent. The pharmaceutically acceptable base addition salt includes a salt of sodium, potassium, calcium, ammonium, organic amine or magnesium or similar salts. When compounds disclosed herein contain a relatively basic functional group, an acid addition salt can be obtained by bringing the compound into contact with a sufficient amount of acid in a pure solution or a suitable inert solvent. Examples of the pharmaceutically acceptable acid addition salt include an inorganic acid salt, wherein the inorganic acid includes, for example, hydrochloric acid, hydrobromic acid, nitric acid, carbonic acid, bicarbonate, phosphoric acid, monohydrogen phosphate, dihydrogen phosphate, sulfuric acid, hydrogen sulfate, hydroiodic acid, phosphorous acid, and the like; and an organic acid salt, wherein the organic acid includes, for example, acetic acid, propionic acid, isobutyric acid, maleic acid, malonic acid, benzoic acid, succinic acid, suberic acid, fumaric acid, lactic acid, mandelic acid, phthalic acid, benzenesulfonic acid, p-toluenesulfonic acid, citric acid, tartaric acid, and methanesulfonic acid, and the like; and an salt of amino acid (such as arginine and the like), and a salt of an organic acid such as glucuronic acid and the like. Certain specific compounds disclosed herein contain both basic and acidic functional groups and can be converted to any base or acid addition salt.

The pharmaceutically acceptable salt disclosed herein can be prepared from the parent compound that contains an acidic or basic moiety by conventional chemical methods. Generally, such salt can be prepared by reacting the free acid or base form of the compound with a stoichiometric amount of an appropriate base or acid in water or an organic solvent or a mixture thereof.

Compounds disclosed herein may be present in a specific geometric or stereoisomeric form. The present disclosure contemplates all such compounds, including cis and trans isomers, (-)- and (+)-enantiomers, (R)- and (S)-enantiomers, diastereoisomer, (D)-isomer, (L)-isomer, and a racemic mixture and other mixtures, for example, a mixture enriched in enantiomer or diastereoisomer, all of which are encompassed within the scope disclosed herein. The substituent such as alkyl may have an additional asymmetric carbon atom. All these isomers and mixtures thereof are encompassed within the scope disclosed herein.

Compounds disclosed herein may contain an unnatural proportion of atomic isotopes at one or more of the atoms that make up the compounds. For example, a compound may be labeled with a radioisotope such as tritium (³H), iodine-125 (¹²⁵I) or C-14(¹⁴C). For another example, hydrogen can be replaced by heavy hydrogen to form a deuterated drug. The bond between deuterium and carbon is stronger than that between ordinary hydrogen and carbon. Compared with undeuterated drugs, deuterated drugs have advantages of reduced toxic side effects, increased drug stability, enhanced efficacy, and prolonged biological half-life of drugs. All changes in the isotopic composition of compounds disclosed herein, regardless of radioactivity, are included within the scope of the present disclosure.

The term "optional" or "optionally" means that the subsequent event or condition may occur but not requisite, that the term includes the instance in which the event or condition occurs and the instance in which the event or condition does not occur.

The term "substituted" means one or more than one hydrogen atom(s) on a specific atom are substituted by a substituent, including deuterium and hydrogen variants, as long as the valence of the specific atom is normal and the substituted compound is stable. When the substituent is oxo (i.e., = O), it means two hydrogen atoms are substituted. Positions on an aromatic ring cannot be substituted by oxo. The term "optionally substituted" means an atom can be substituted by a substituent or not, unless otherwise specified, the species and number of the substituent may be arbitrary so long as being chemically achievable.

When any variable (such as R) occurs in the constitution or structure of the compound more than once, the definition of the variable at each occurrence is independent. Thus, for example, if a group is substituted by 0 to 2 R, the group can be optionally substituted by up to two R, wherein the definition of R at each occurrence is independent. Moreover, a combination of the substituent and/or the variant thereof is allowed only when the combination results in a stable compound.

When the number of a linking group is 0, such as -(CRR)₀-, it means that the linking group is a single bond.

When one of variables is a single bond, it means that the two groups linked by the single bond are connected directly. For example, when L in A-L-Z represents a single bond, the structure of A-L-Z is actually A-Z.

When an enumerated linking group does not indicate its linking direction, its linking direction is read in the order from left to right as shown in the plane. For example, when the linking group L in 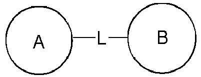 is -M-W-, the -M-W- is linked to the ring A and the ring B in the same direction as the reading order from left to right to constitute 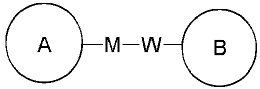 . A combination of the linking groups, substituents and/or variants thereof is allowed only when such combination can result in a stable compound.

Unless otherwise specified, when a group has one or more connectable sites, any one or more sites of the group can be connected to other groups through chemical bonds. Where the connection position of the chemical bond is variable, and there is H atom(s) at a connectable site(s), when the connectable site(s) having H atom(s) is connected to the chemical bond, the number of H atom(s) at this site will correspondingly decrease as the number of the connected chemical bond increases, and the group will become a group of corresponding valence. The chemical bond between the site and other groups can be represented by a straight solid bond 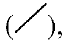 a straight dashed bond 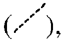 or a wavy line 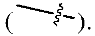 For example, the straight solid bond in -OCH₃ indicates that the group is connected to other groups through the oxygen atom in the group; the straight dashed bond in 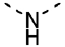 indicates that the group is connected to other groups through two ends of the nitrogen atom in the group; the wavy line in 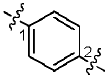 indicates that the group is connected to other groups through the 1- and 2-carbon atoms in the phenyl group; 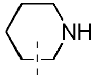 indicates that any connectable site on the piperidinyl group can be connected to other groups through one chemical bond, including at least four connection ways, 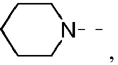 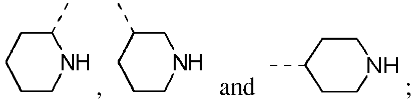 even if a H atom is drawn on -N-, 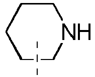 still includes the connection way of 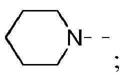 it's just that when one chemical bond is connected, the H at this site will be reduced by one, and the group will become the corresponding monovalent piperidinyl group. 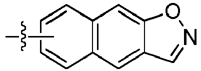 indicates that any connectable site on this naphtho[2,3-d]isoxazolyl group can be connected to other groups through one chemical bond, including at least 7 connection ways:

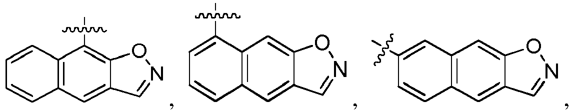

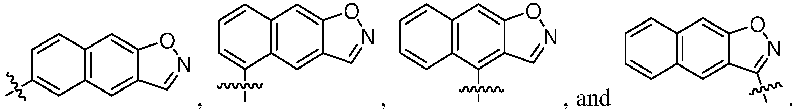

Unless otherwise specified, a wedged solid bond ( 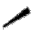 ) and a wedged dashed bond ( 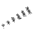 ) indicate the absolute configuration of a stereocenter; a straight solid bond ( 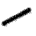 ) and a straight dashed bond ( 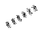 ) indicate the relative configuration of a stereocenter; a wavy line ( 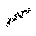 ) indicates a wedged solid bond ( 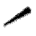 ) or a wedged dashed bond ( 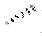 ); or a wavy line ( 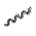 ) indicates a straight solid bond ( 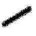 ) or a straight dashed bond ( 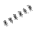 ).

Unless otherwise specified, the term "halo" or "halogen" by itself or as part of another substituent represents a fluorine (F), chlorine (Cl), bromine (Br) or iodine (I) atom.

Unless otherwise specified, the term "C₁₋₃ alkyl" is used to indicate a linear or branched saturated hydrocarbon group consisting of 1 to 3 carbon atoms. The C₁₋₃ alkyl group includes C₁₋₂ and C₂₋₃ alkyl groups and the like. It may be monovalent (e.g., methyl), divalent (e.g., methylene) or multivalent (e.g., methenyl). Examples of C₁₋₃ alkyl group include, but are not limited to, methyl (Me), ethyl (Et), propyl (including n-propyl and isopropyl), and the like.

Unless otherwise specified, the term "C₁₋₃ alkoxy" means an alkyl group containing 1 to 3 carbon atoms and attached to the remainder of a molecule by an oxygen atom. The C₁₋₃ alkoxy group includes C₁₋₂, C₂₋₃, C₃ and C₂ alkoxy groups, and the like. Examples of C₁₋₃ alkoxy groups include, but are not limited to, methoxy, ethoxy, propoxy (including n-propoxy and isopropoxy), and the like.

Unless otherwise specified, the term "C₁₋₃ alkylamino" means an alkyl group containing 1 to 3 carbon atoms and attached to the remainder of a molecule by an amino group. The C₁₋₃ alkylamino group includes C₁₋₂, C₃ and C₂ alkylamino groups and the like. Examples of C₁₋₃ alkylamino groups include, but are not limited to -NHCH₃, -N(CH₃)₂, -NHCH₂CH₃, - N(CH₃)CH₂CH₃, -NHCH₂CH₂CH₃, -NHCH₂(CH₃)₂, and the like.

Unless otherwise specified, "C₂₋₄ alkenyl" is used to represent a linear or branched hydrocarbon group composed of 2 to 4 carbon atoms containing at least one carbon-carbon double bond, wherein the carbon-carbon double bond can be located at any position of the group. The C₂₋₄ alkenyl includes C₂₋₃, C₄, C₃ and C₂ alkenyl. The C₂₋₄ alkenyl may be monovalent, divalent or multivalent. Examples of the C₂₋₄ alkenyl include, but are not limited to, vinyl, propenyl, butenyl, butadienyl and the like.

Unless otherwise specified, "C₂₋₃ alkenyl" is used to represent a linear or branched hydrocarbon group composed of 2 to 3 carbon atoms containing at least one carbon-carbon double bond, wherein the carbon-carbon double bond can be located at any position of the group. The C₂₋₃ alkenyl includes C₃ and C₂ alkenyl. The C₂₋₃ alkenyl may be monovalent, divalent or multivalent. Examples of the C₂₋₃ alkenyl include, but are not limited to, vinyl, propenyl, and the like.

Unless otherwise specified, "C₂₋₄ alkynyl" is used to represent a linear or branched hydrocarbon group composed of 2 to 4 carbon atoms containing at least one carbon-carbon triple bond, wherein the carbon-carbon triple bond can be located at any position of the group. The C₂₋₄ alkynyl includes C₂₋₃, C₄, C₃ and C₂ alkynyl, etc. It may be monovalent, divalent or multivalent. Examples of the C₂₋₄ alkynyl include, but are not limited to, ethynyl, propynyl, butynyl, and the like.

Unless otherwise specified, "C₂₋₃ alkynyl" is used to represent a linear or branched hydrocarbon group composed of 2 to 3 carbon atoms containing at least one carbon-carbon triple bond, wherein the carbon-carbon triple bond can be located at any position of the group. It may be monovalent, divalent or multivalent. The C₂₋₃ alkynyl includes C₃ and C₂ alkynyl. Examples of the C₂₋₃ alkynyl include, but are not limited to, ethynyl, propynyl, and the like.

Unless otherwise specified, the terms "C₆₋₁₀ aromatic ring" and "C₆₋₁₀ aryl" may be used interchangeably in this disclosure. The term"C₆₋₁₀ aromatic ring" or "C₆₋₁₀ aryl" means a cyclic hydrocarbon group having a conjugated pi electron system and composed of 6 to 10 carbon atoms. It may be a monocyclic, fused bicyclic or fused tricyclic ring system, wherein each ring is aromatic. It may be monovalent, divalent or multivalent. The C₆₋₁₀ aryl includes C₆₋₉, C₉, C₁₀ and C₆ aryl, etc. Examples of C₆₋₁₀ aryl include, but are not limited to, phenyl, naphthyl (including 1-naphthyl and 2-naphthyl, etc.).

Unless otherwise specified, the terms "5- to 10-membered heteroaromatic ring" and "5- to 10-membered heteroaryl" may be used interchangeably. The term "5- to 10-membered heteroaryl" means a cyclic group having a conjugated pi electron system and composed of 5 to 10 ring atoms, in which 1, 2, 3 or 4 ring atoms are heteroatoms independently selected from O, S and N, and the remainder is carbon atoms. It may be a monocyclic, fused bicyclic or fused tricyclic ring system, wherein each ring is aromatic, and wherein the nitrogen atom is optionally quaternized and the nitrogen and sulfur heteroatoms are optionally oxidized (i.e., NO and S(O)ₚ, p is 1 or 2). A 5- to 10-membered heteroaryl can be attached to the remainder of the molecule through a heteroatom or a carbon atom. The 5- to 10-membered heteroaryl group includes 5-to 8-membered, 5- to 7-membered, 5- to 6-membered, 5-membered and 6-membered heteroaryl groups. Examples of the 5-10 membered heteroaryl include, but are not limited to, pyrrolyl (including N-pyrrolyl, 2-pyrrolyl, 3-pyrrolyl, and the like), pyrazolyl (including 2-pyrazolyl and 3-pyrazolyl, and the like), imidazolyl (including N-imidazolyl, 2-imidazolyl, 4-imidazolyl, and 5-imidazolyl, and the like), oxazolyl (including 2-oxazolyl, 4-oxazolyl, and 5-oxazolyl, and the like), triazolyl (1H-1,2,3-triazolyl, 2H-1,2,3-triazolyl, 1H-1,2,4-triazolyl and 4H-1,2,4-triazolyl, and the like), tetrazolyl, isoxazolyl (3-isoxazolyl, 4-isoxazolyl and 5-isoxazolyl, and the like), thiazolyl (including 2-thiazolyl, 4-thiazolyl and 5-thiazolyl, and the like), furyl (including 2-furyl and 3-furyl, and the like), thienyl (including 2-thienyl and 3-thienyl, and the like), pyridyl (including 2-pyridyl, 3-pyridyl and 4-pyridyl, and the like), pyrazinyl or pyrimidinyl (including 2-pyrimidinyl and 4-pyrimidinyl, and the like), benzothiazolyl (including 5-benzothiazolyl, and the like), purinyl, benzimidazolyl (including 2-benzimidazolyl, and the like), benzoxazolyl, indolyl (including 5-indolyl, and the like), isoquinolyl (including 1-isoquinolyl, 5-isoquinolyl, and the like), quinoxalinyl (including 2-quinoxalinyl, 5-quinoxalinyl, and the like) or quinolyl (including 3-quinolyl, 6-quinolyl, and the like).

Unless otherwise specified, the term "4- to 8-membered heterocycloalkyl" alone or in combination with other terms respectively represents a saturated cyclic group composed of 4 to 8 ring atoms, in which 1, 2, 3 or 4 ring atoms are heteroatoms independently selected from O, S and N, and the remainder is carbon atoms, wherein the nitrogen atom is optionally quaternized, and the nitrogen and sulfur heteroatoms are optionally oxidized (i.e., NO and S(O)ₚ, p is 1 or 2). The ring comprises monocyclic and bicyclic ring systems, wherein the bicyclic ring systems comprise spiro, fused, and bridged cyclic rings. In addition, with respect to the "4- to 8-membered heterocycloalkyl", the heteroatom may be present on the position of attachment of the heterocycloalkyl group to the remainder of a molecule. The 4- to 8-membered heterocycloalkyl includes 4-6 membered, 5-6 membered, 4 membered, 5 membered, and 6 membered heterocycloalkyl, etc. Examples of the 4- to 8-membered heterocycloalkyl include, but are not limited to, azetidinyl, oxetanyl, thietanyl, pyrrolidinyl, pyrazolidinyl, imidazolidinyl, tetrahydrothienyl (including tetrahydrothien-2-yl and tetrahydrothien-3-yl and the like), tetrahydrofuranyl (including tetrahydrofuran-2-yl and the like), tetrahydropyranyl, piperidinyl (including 1-piperidinyl, 2-piperidinyl and 3-piperidinyl and the like), piperazinyl (including 1-piperazinyl and 2-piperazinyl and the like), morpholinyl (including 3-morpholinyl and 4-morpholinyl and the like), dioxanyl, dithianyl, isoxazolidinyl, isothiazolidinyl, 1,2-oxazinyl, 1,2-thiazinyl, hexahydropyridazinyl, homopiperazinyl, homopiperidinyl or dioxepanyl, and the like.

Unless otherwise specified, the term "5- to 6-membered heterocycloalkenyl" alone or in combination with other terms each means a partially unsaturated cyclic group containing at least one carbon-carbon double bond and consisting of 5 to 6 ring atoms, of which 1, 2, 3 or 4 ring atoms are heteroatoms independently selected from O, S and N, and the remainder atoms are carbon atoms, wherein the nitrogen atom is optionally quaternized and the nitrogen and sulfur heteroatoms may be optionally oxidized (i.e., NO and S(O)ₚ, wherein p is 1 or 2). It comprises a single ring system and a double ring system, wherein the double ring system comprises a sprio-ring, a fused-ring and a bridged-ring, and any ring of the system is nonaromatic. In addition, with respect to the "5- to 6-membered heterocycloalkenyl", the heteroatom may be present on the position of attachment of the heterocycloalkenyl group to the remainder of a molecule. The 5- to 6-membered heterocycloalkenyl group includes 5-membered and 6-membered heterocycloalkenyl groups and the like. Examples of 5- to 6-membered heterocycloalkenyl groups include, but are not limited to

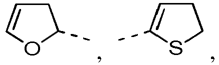

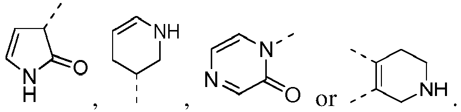

Unless otherwise specified, Cₙ₋ₙ₊ₘ or Cₙ-Cₙ₊ₘ includes any specific case of n to n+m carbons, for example, C₁₋₁₂ includes C₁, C₂, C₃, C₄, C₅, C₆, C₇, C₈, C₉, C₁₀, C₁₁ and C₁₂, also includes any range from n to n+m, for example, C₁₋₁₂ includes C₁₋₃, C₁₋₆, C₁₋₉, C₃₋₆, C₃₋₉, C₃₋₁₂, C₆₋₉, C₆₋₁₂ and C₉₋₁₂, etc.; similarly, n membered to n+m membered indicates that the number of atoms on a ring is n to n+m, for example, 3-12 membered ring includes 3 membered ring, 4 membered ring, 5 membered ring, 6 membered ring, 7 membered ring, 8 membered ring, 9 membered ring, 10 membered ring, 11 membered ring, and 12 membered ring, also includes any range from n to n+m, for example, 3-12 membered ring includes 3-6 membered ring, 3-9 membered ring, 5-6 membered ring, 5-7 membered ring, 6-7 membered ring, 6-8 membered ring, and 6-10 membered ring, and the like.

Compounds disclosed herein can be prepared by a variety of synthetic methods well known to those skilled in the art, including the following enumerated embodiment, the embodiment formed by the following enumerated embodiment in combination with other chemical synthesis methods, and equivalent replacement well known to those skilled in the art. Alternative embodiments include, but are not limited to the embodiment disclosed herein.

The structures of compounds disclosed herein can be confirmed by conventional methods well known to those skilled in the art. If the present disclosure relates to an absolute configuration of a compound, the absolute configuration can be confirmed by conventional techniques in the art, such as single crystal X-Ray diffraction (SXRD). In the single crystal X-Ray diffraction (SXRD), the diffraction intensity data of the cultivated single crystal is collected using a Bruker D8 venture diffractometer with a light source of CuKα radiation in a scanning mode of ϕ/ω scan; after collecting the relevant data, the crystal structure is further analyzed by the direct method (Shelxs97) to confirm the absolute configuration.

Solvents used in the present disclosure are commercially available.

Compounds are named according to general naming principles in the art or by ChemDraw^{®} software, and commercially available compounds are named with their vendor directory names.

### BRIEF DESCRIPTION OF THE DRAWINGS

FIG. 1. Binding pattern of compound A and KRAS^{G12D} protein;
FIG. 2. Binding pattern of compound B and KRAS^{G12D} protein;
FIG. 3. Binding pattern of compound C and KRAS^{G12D} protein;
FIG. 4. Binding pattern of compound D and KRAS^{G12D} protein;
FIG. 5. Binding pattern of compound E and KRAS^{G12D} protein;
FIG. 6. Binding pattern of compound F and KRAS^{G12D} protein;
FIG. 7. Binding pattern of compound G and KRAS^{G12D} protein;
FIG. 8. Binding pattern of compound H and KRAS^{G12D} protein;
FIG. 9. Binding pattern of compound I and KRAS^{G12D} protein;
FIG. 10. Binding pattern of compound J and KRAS^{G12D} protein;
FIG. 11. Binding pattern of compound K and KRAS^{G12D} protein.

### DETAILED DESCRIPTION OF THE INVENTION

The present disclosure is described in detail below by means of examples. However, it is not intended that these examples have any disadvantageous limitations to the present disclosure. The present disclosure has been described in detail herein, and embodiments are also disclosed herein. It will be apparent to those skilled in the art that various changes and modifications may be made to the embodiments disclosed herein without departing from the spirit and scope disclosed herein.

### Calculation example 1

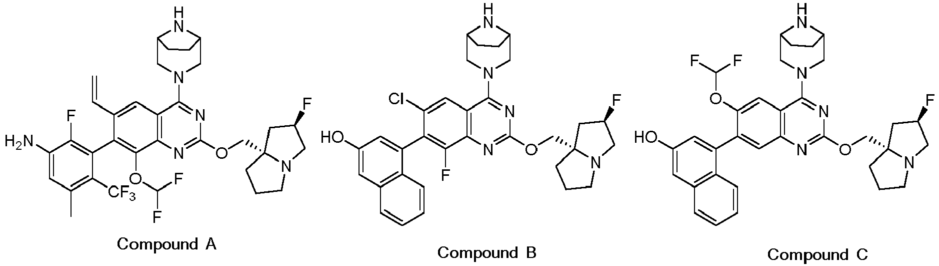

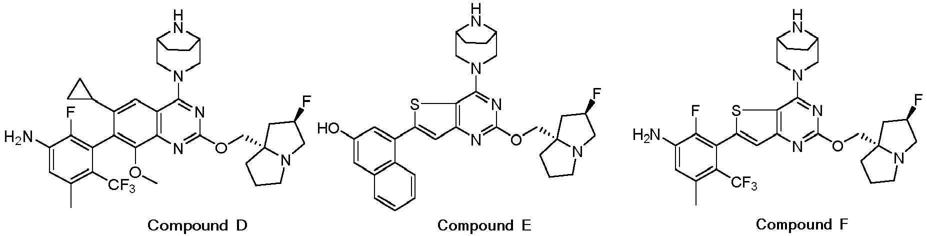

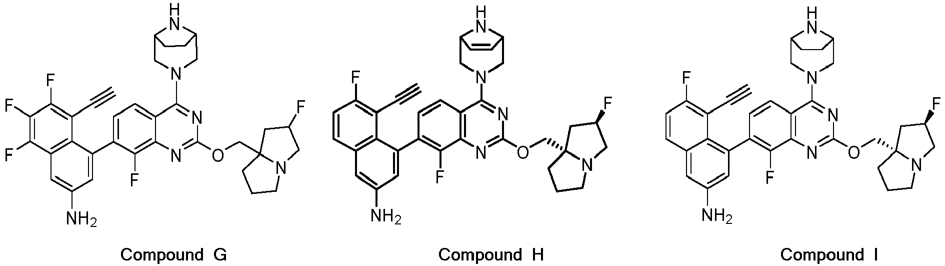

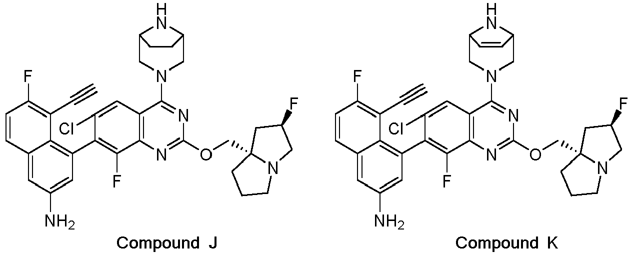

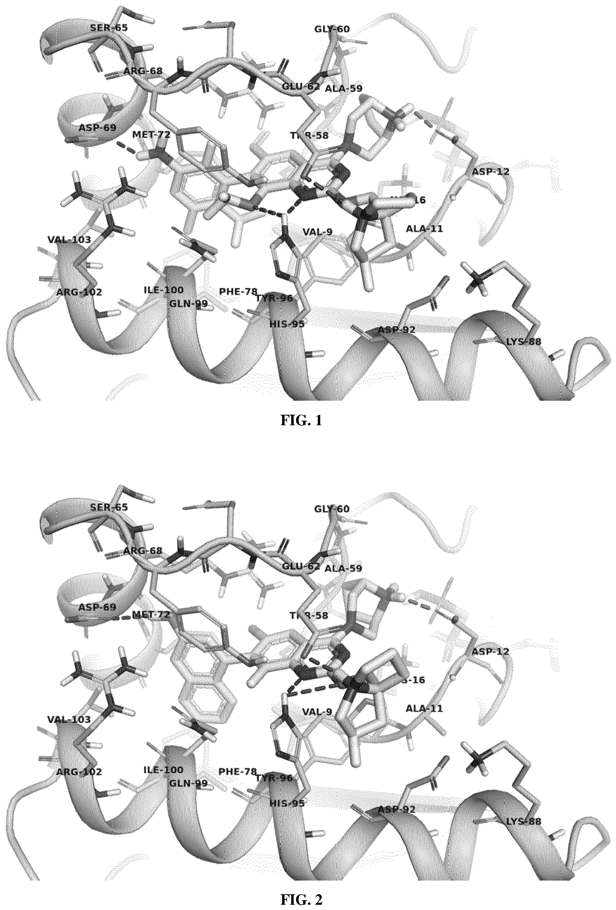

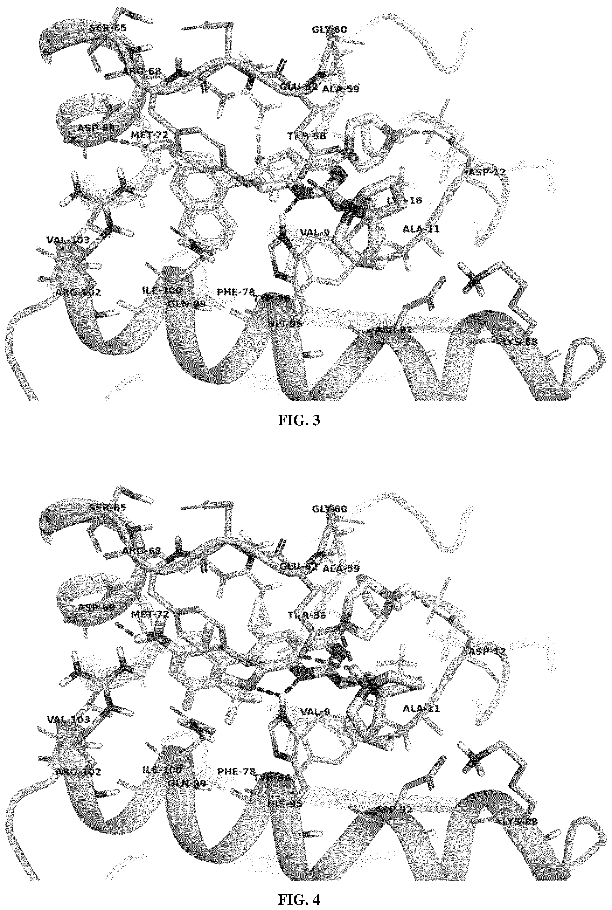

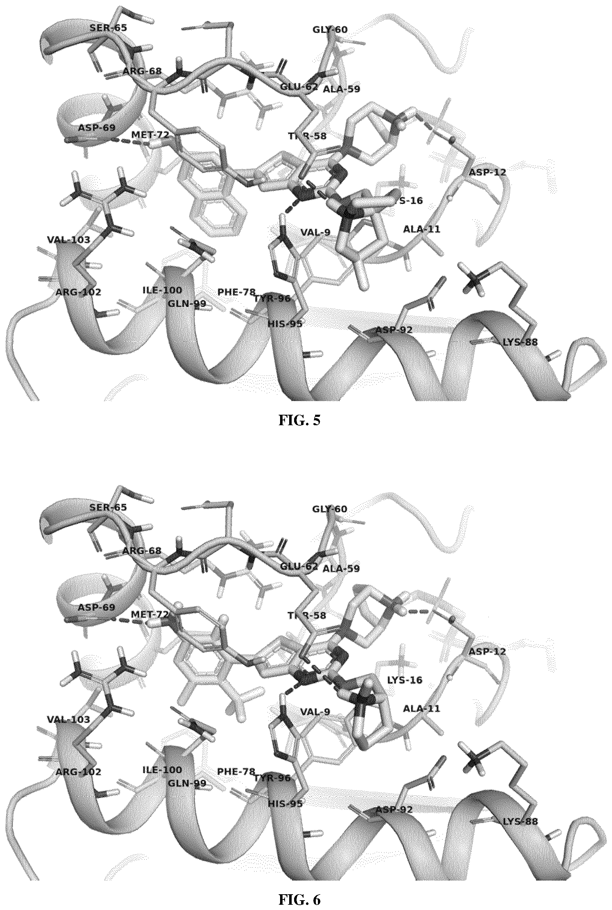

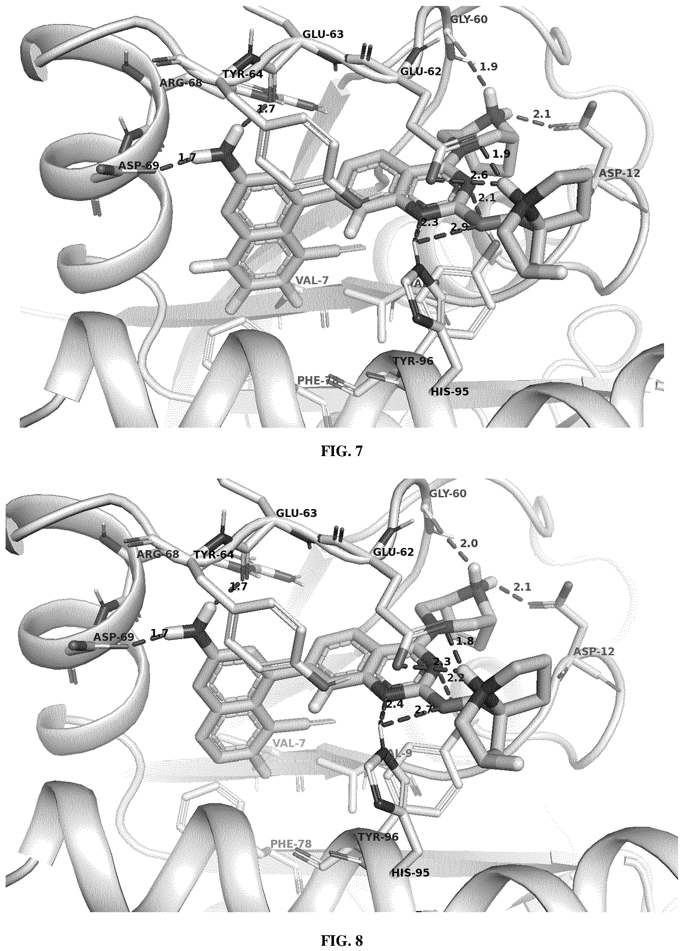

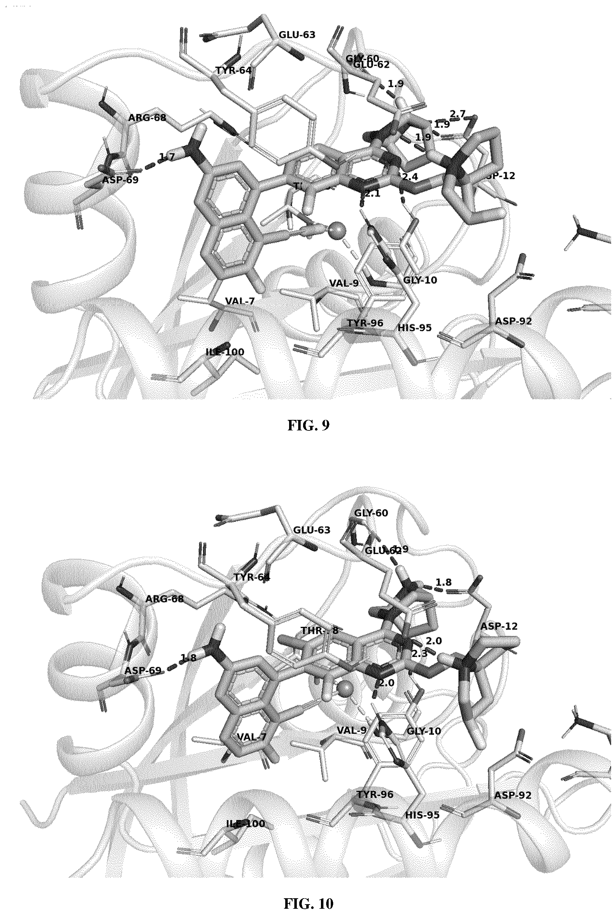

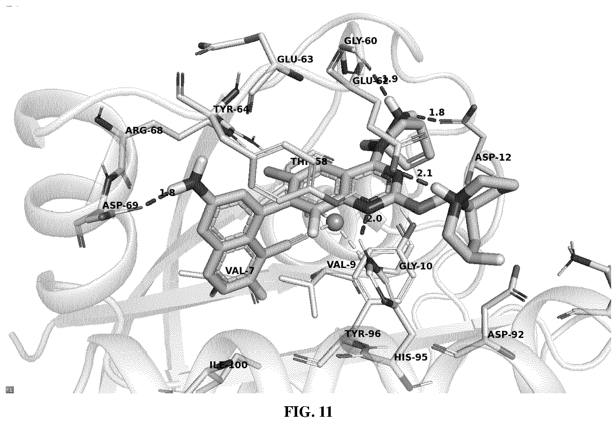

The molecular docking process was performed by using Glide SP^{[1]} in Maestro (Schrödinger version 2017-2) with default options. The crystal structure PDB: 6UT0 of KRAS_G12C in the PDB database was selected, and Cys12 was simulated to be mutated to Asp12, which was used as the docking template after energy optimization. To prepare the protein, hydrogen atoms were added using the Protein Preparation Wizard module of Maestro^{[2]} and the OPLS3 force field was used. For ligand preparation, the 3D structure of the molecule was generated using LigPrep and energy minimization was performed^{[3]}. The small molecule conformation was sampled using the confgen module. A cubic docking mesh with side length of 25 Å *25 Å *25 Å was generated with the ligand of 6UT0 as the centroid. Reference compound was placed during molecular docking. The type of interaction of the protein receptor with the ligand was analyzed, and the type of interaction of the protein receptor with the ligand was analyzed. A reasonable docking conformation was selected and saved according to the calculated docking scrore and binding pattern, as shown in FIG. 1 to FIG. 11.
[1]Glide, Schrödinger, LLC, New York, NY, 2017.
[2] Maestro, Schrödinger, LLC, New York, NY, 2017.
[3] LigPrep, Schrödinger, LLC, New York, NY, 2017.

Conclusion: The compounds of the present disclosure have good binding with KRAS^{G12D}.

### Example 1

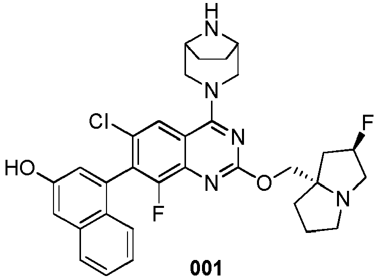

### Synthetic route:

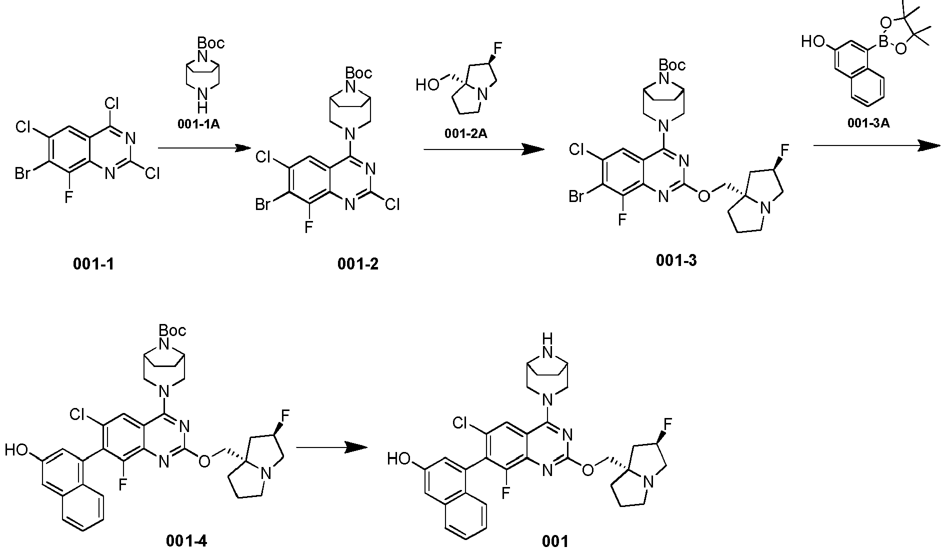

### Step 1: Synthesis of compound 001-2

Compound **001-1** (3.95 g, 11.96 mmol, 1 eq) and compound **001-1A** (2.54 g, 11.96 mmol, 1 eq) were dissolved in N,N-dimethylformamide (100 mL), and diisopropylethylamine (3.87 g, 29.91 mmol, 5.21 mL, 2.5 eq) was added. The mixture was reacted at 20 °C for 16 h. 200 mL of water was added and the mixture was extracted with ethyl acetate 3 times, 100 mL each time. The organic phases were combined, dried over anhydrous sodium sulfate, and rotary-evaporated to dryness to give a crude product. The crude product was separated on silica gel column (petroleum ether : ethyl acetate = 20:1-10:1-5:1) to give compound **001-2.** LCMS:(ESI)m/z: 506.8 [M+H]⁺.

### Step 2: Synthesis of compound 001-3

Compound **001-2** (1.5 g, 2.96 mmol, 1 eq) was dissolved in tetrahydrofuran (6 mL) and N,N-dimethylformamide (6 mL), and **001-2A** (707.64 mg, 4.44 mmol, 1.5 eq), cesium carbonate (2.90 g, 8.89 mmol, 3 eq) and triethylenediamine (33.24 mg, 296.33 µmol, 32.59 µL, 0.1 eq) were added sequentially under nitrogen. The mixture was then reacted at 20 °C for 16 h. To the reaction solution was added water, and the mixture was extracted with ethyl acetate (10 mL*3), and washed with water. The organic phase was concentrated by rotary-evaporation to dryness to give a crude product. The crude product was purified by column chromatography (PE:EA = 10:1) to give compound **001-3.** LCMS:(ESI)m/z: 628.1 [M+H]⁺.

### Step 3: Synthesis of compound 001-4

Compound **001-3** (0.05 g, 79.50 µmol, 1 eq), compound **001-3A** (25.77 mg, 95.40 µmol, 1.2 eq), and sodium carbonate (25.28 mg, 238.50 µmol, 3 eq) were dissolved in 1,4-dioxane (2 mL) and water (0.5 mL), and Pd(dppf)Cl₂.DCM (5.82 mg, 7.95 µmol, 0.1 eq) was added under nitrogen. The mixture was then reacted at 100 °C for 16 h. The reaction solution was concentrated to give a crude product. The crude product was purified by column chromatography (PE:EA= 10:1) to give compound **001-4.** LCMS:(ESI)m/z: 692.2 [M+H]⁺.

### Step 4: Synthesis of compound 001 formate

Compound **001-4** (0.05 g, 72.23 µmol, 1 *eq*) was dissolved in dichloromethane (2 mL) and trifluoroacetic acid (2.57 g, 22.51 mmol, 1.67 mL, 311.63 *eq)* was added. The mixture was then reacted at 20 °C for 1 hr. The reaction solution was concentrated by rotary-evaporation to dryness to give a crude product. The crude product was purified by machine separation (column: Phenomenex Gemini-NX C18 75*30mm*3µm; mobile phase: [water (0.225% formic acid)-acetonitrile]; acetonitrile %: 0%-30%, 7 min) to give compound **001** formate. LCMS:(ESI)m/z: 592.2 [M+H] ⁺. ¹H NMR (CD₃OD, 400MHz): δ = 8.50 (s, 1H), 8.02 (s, 1H), 7.78 (d, J = 8.3 Hz, 1H), 7.41-7.46 (m, 1H), 7.29 (d, J = 2.3 Hz, 1H), 7.19-7.25 (m, 2H), 7.05 (d, J = 1.5 Hz, 1H), 5.38-5.58 (m, 1H), 4.69 (br d, J = 13.6 Hz, 2H), 4.49-4.60 (m, 2H), 4.08 (br s, 2H), 3.86 (br d, J = 13.3 Hz, 2H), 3.59-3.81 (m, 3H), 3.25-3.33 (m, 3H), 2.44-2.65 (m, 2H), 2.29-2.38 (m, 1H), 2.20-2.28 (m, 2H), 2.07-2.13 ppm (m, 3H).

### Example 4

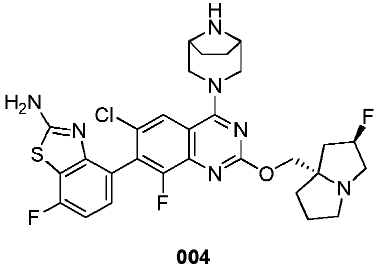

### Synthetic route:

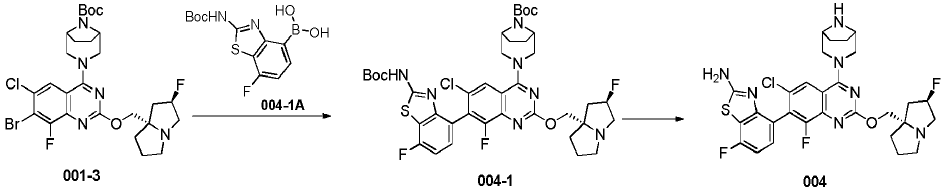

### Step 1: Synthesis of compound 004-1

Compounds **001-3** (500 mg, 794.99 µmol, 1 *eq)* and **004-1A** (297.76 mg, 953.99 µmol, *1.2 eq*) and cesium carbonate (518.05 mg, 1.59 mmol, 2 *eq*) were added to 1,4-dioxane (5 mL) and water (1 mL). The atmosphere was replaced with nitrogen, then tetrakis(triphenylphosphine)palladium (91.87 mg, 79.50 µmol, 0.1 *eq*) was added and the mixture was stirred to react in an oil bath at 100 °C for 15 h. After the reaction was completed, the reaction solution was rotary-evaporated to dryness and purified on silica gel column (ethyl acetate/petroleum ether = 0-100%) to give compound **004-1.** LCMS:(ESI)m/z: 816.5 [M+H]⁺.

### Step 2: Synthesis of compound 004

Compound **004-1** (530 mg, 649.25 µmol, 1 *eq*) was added to trifluoroacetic acid (5 mL) and the mixture was stirred to react at room temperature (20°C) for 30 min. After the reaction was completed, the reaction solution was directly concentrated and purified by a preparative chromatography column: Phenomenex C18 80*40 mm*3 µm; mobile phase: [water (ammonia)-acetonitrile]; acetonitrile %: 44%-74%, 8 min, to give compound **004.** LCMS:(ESI)m/z: 616.2 [M+H]⁺.

### Example 5

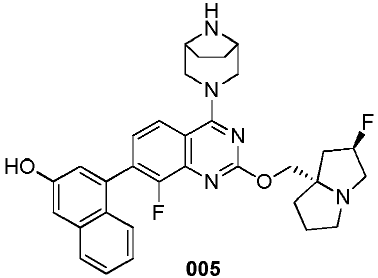

### Synthetic route:

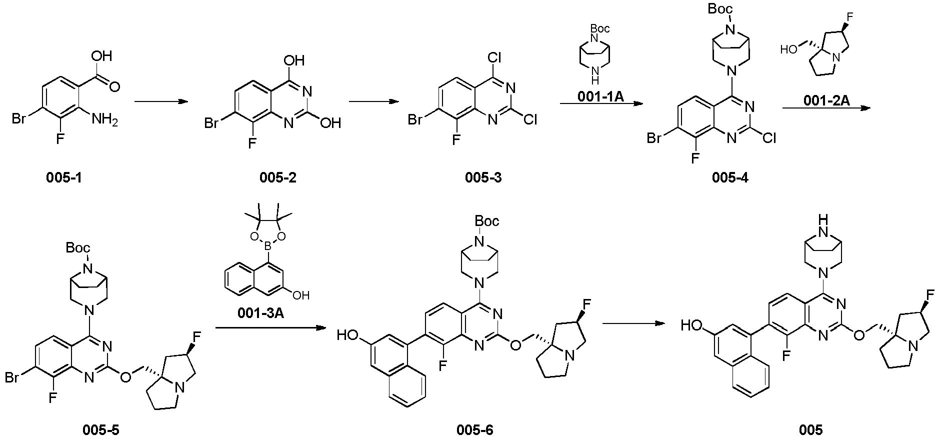

### Step 1: Synthesis of compound 005-2

Compound **005-1** (4 g, 17.09 mmol, 1 *eq)* and urea (10.27 g, 170.92 mmol, 9.17 mL, 10 *eq)* were added to a flask. The mixture was reacted at 200 °C for 1.5 h. After the reaction was completed, the reaction was cooled down to room temperature. The reaction solid was slurried with 30 mL of ethyl acetate for 1 h and filtered. The filter cake was concentrated by rotary-evaporation to dryness, then slurried with 30 mL of water for 1 h, and filtered. The filter cake was rotary-evaporated to dryness to give compound **005-2.** ¹H NMR (400 MHz, DMSO-*d*₆) δ = 11.45 - 11.06 (m, 2H), 7.67 - 7.56 (m, 1H), 7.45 - 7.35 (m, 1H).

### Step 2: Synthesis of compound 005-3

Compound **005-2** (2.5 g, 9.65 mmol, 1 *eq*) was added to phosphorus oxychloride (20 mL), and diisopropylethylenediamine (3.74 g, 28.95 mmol, 5.04 mL, 3 *eq*) was added. The mixture was reacted at 100 °C for 3 h. After the reaction was completed, the reaction solution was concentrated directly, and the concentrated product was slowly added to 20 mL of ice water and extracted with 10 mL*2 of ethyl acetate. The organic phases were combined, washed respectively with 20 mL of saturated ammonium chloride and saturated brine, dried with anhydrous sodium sulfate and filtered. The filtrate was concentrated to give compound **005-3.** ¹H NMR (400 MHz, DMSO- *d₆*) δ = 8.81 - 8.59 (m, 2H); LCMS:(ESI)m/z: 296.8 [M+H] ⁺.

### Step 3: Synthesis of compound 005-4

Compound **005-3** (1.5 g, 5.07 mmol, 1 *eq*) was added to anhydrous dichloromethane (20 mL), and triethylamine (1.54 g, 15.21 mmol, 2.12 mL, 3 *eq)* and compound **001-1A** (1.29 g, 6.08 mmol, 1.2 *eq*) were added. The mixture was reacted at 20 °C for 2 h. After the reaction was completed, the crude product was purified using a column (petroleum ether:ethyl acetate = 100:0-1:1) to give compound **005-4.** ¹H NMR (400 MHz, DMSO- *d₆*) δ = 7.83 (d, *J* = 0.8Hz, 1H), 7.71 - 7.63 (m, 1H), 4.42 - 4.30 (m, 2H), 4.23 (s, 2H), 3.58 (d, *J* = 1.2Hz, 2H), 1.84 - 1.74 (m, 2H), 1.68 - 1.59 (m, 2H), 1.45 (s, 9H); LCMS:(ESI)m/z: 473.0[M+H]⁺.

### Step 4: Synthesis of compound 005-5

Compound **005-4** (0.7 g, 1.48 mmol, 1 eq) was added to N,N-dimethylformamide (7 mL) and anhydrous tetrahydrofuran (7 mL), and compound **001-2A** (354.34 mg, 2.23 mmol, 1.5 eq), cesium carbonate (1.45 g, 4.45 mmol, 3 eq), and triethylenediamine (16.64 mg, 148.38 µmol, 16.32 µL, 0.1 eq) were added. The mixture was reacted at 20°C for 20 h. After the reaction was completed, 20 mL of water was added to the reaction solution, and the mixture was extracted with 10 mL*2 of ethyl acetate. The organic phases were combined, washed with 20 mL of saturated saline, dried with anhydrous sodium sulfate and filtered. The filtrate was concentrated, and the crude product was purified using a column (dichloromethane:methanol = 100:0-10:1) to give compound **005-5.** ¹H NMR (400 MHz, CDCl₃) δ = 7.45 - 7.39 (m, 1H), 7.32 - 7.28 (m, 1H), 5.40 - 5.13 (m, 1H), 4.45 - 4.28 (m, 4H), 4.26 - 4.21 (m, 1H), 4.18 - 4.05 (m, 1H), 3.69 - 3.42 (m, 2H), 3.33 - 3.10 (m, 3H), 3.05 - 2.92 (m, 1H), 2.30 - 2.07 (m, 3H), 2.00 - 1.84 (m, 5H), 1.81 - 1.70 (m, 2H), 1.52 (s, 9H); LCMS:(ESI)m/z: 594.1[M+H]⁺.

### Step 5: Synthesis of compound 005-6

Compound **005-5** (0.1 g, 168.21 µmol, 1 *eq*) and compound **001-3A** (68.16 mg, 252.32 µmol, 1.5 *eq)* and sodium carbonate (35.66 mg, 336.42 µmol, 2 *eq*) were added to 1,4-dioxane (2 mL) and water (0.4 mL). The atmosphere was replaced with nitrogen three times, and [1,1-bis(diphenylphosphino)ferrocene]palladium dichloride dichloromethane (13.74 mg, 16.82 µmol, 0.1 *eq)* was added. The mixture was reacted at 100 °C for 1 h. The reaction solution was diluted using 3 mL of ethyl acetate, washed using 2 mL of water and 5 mL of saturated brine, dried with anhydrous sodium sulfate and filtered. The filtrate was concentrated to give compound **005-6.** LCMS:(ESI)m/z: 658.2[M+H]⁺.

### Step 6: Synthesis of compound 005 hydrochloride

Compound **005-6** (70.00 mg, 106.42 µmol, 1 *eq)* was added to anhydrous dichloromethane (2 mL) and trifluoroacetic acid (0.4 mL) was added. The mixture was reacted at 20 °C for 2 h. The reaction solution was directly concentrated by rotary-evaporation to dryness, and the crude product was purified by preparative chromatography (column: Phenomenex luna C18 80*40 mm*3 µm; mobile phase: [water (HCl) - acetonitrile]; Acetonitrile %: 14%-34%, 7 min) to give compound **005** hydrochloride. ¹H NMR (400 MHz, DMSO- *d₆*) δ = 11.50 (s, 1H), 10.05 (d, *J* = 0.8 Hz, 1H ), 9.87 - 9.63 (m, 1H), 7.97 (d, *J* = 0.8Hz, 1H), 7.81 (m, *J* = 0.8 Hz, 1H), 7.54 - 7.34 (m, 3H), 7.31 - 7.21 (m, 2H), 7.15 (m, *J* = 0.8 Hz, 1H), 5.67 - 5.62 (m, 1H), 4.69 - 4.59 (m, 2H), 4.52(t, *J* = 1.6 Hz, 2H), 4.17 (s, 2H), 3.95 (t, *J* = 1.2 Hz, 2H), 3.87 - 3.71 (m, 3H), 3.38 - 3.18 (m, 2H), 2.69 - 2.58 (m, 1H), 2.57 - 2.53 (m, 1H), 2.38 - 2.28 (m, 1H), 2.24 - 2.12 (m, 2H), 2.10 - 2.04 (m, 1H), 1.95-2.02 (m, 3H); LCMS:(ESI)m/z: 558.2 [M+H]⁺.

### Example 6

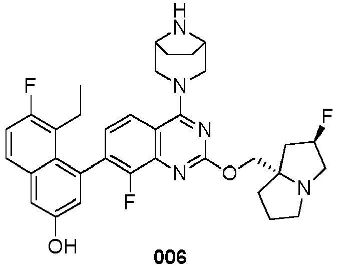

### Synthetic route:

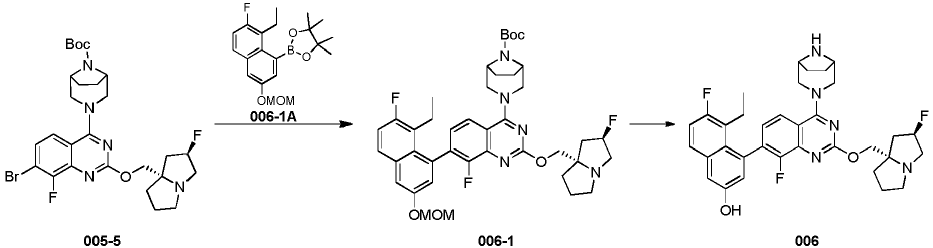

### Step 1: Synthesis of compound 006-1

Compound **005-5** (0.06 g, 100.93 µmol, 1 *eq)* and compound **006-1A** (43.63 mg, 121.11 µmol, 1.2 *eq)* and sodium carbonate (32.09 mg, 302.78 µmol, 3 *eq)* were added to 1,4-dioxane (1 mL) and water (0.2 mL). The atmosphere was replaced with nitrogen three times. [1,1-bis(diphenylphosphino)ferrocene]palladium dichloride (14.77 mg, 20.19 µmol, 0.2 eq) was added under nitrogen. The mixture was reacted at 100 °C for 2 hours. After the reaction was completed, 3 mL of water was added to the reaction solution. The mixture was extracted using 3 mL*2 of ethyl acetate. The organic phases were combined, washed using 5 mL of saturated brine, dried with anhydrous sodium sulfate and filtered. The filtrate was concentrated to give compound **006-1.** LCMS:(ESI)m/z: 748.4[M+H]⁺.

### Step 2: Synthesis of compound 006 hydrochloride

Compound **006-1** was added to acetonitrile (4 mL) and hydrochloric acid/dioxane (4 M, 802.31 µL, 30 *eq)* was added. The mixture was reacted at 20°C for 5 h. After the reaction was completed, the reaction solution was concentrated and the crude product was purified by preparative chromatography column: Phenomenex luna C18 80*40 mm*3 µm; mobile phase: [water (HCl)-acetonitrile]; acetonitrile %: 17%-44%, 7 min, to give compound **006** hydrochloride. ¹H NMR (400 MHz, CD₃OD) δ = 8.11 ((d, *J* = 0.8 Hz, 1H), 7.76 - 7.59 (m, 2H), 7.35 - 7.20 (m, 2H), 6.97 (s, 1H), 5.73 - 5.50 (m, 1H), 5.15 - 4.94 (m, 5H), 4.44 - 4.29 (m, 2H), 4.27 - 4.10 (m, 2H), 4.06 - 3.81 (m, 3H), 3.50 - 3.42 (m, 1H), 2.97 - 2.58 (m, 3H), 2.57 - 2.31 (m, 6H), 2.30 - 2.08 (m, 5H); LCMS:(ESI)m/z: 604.2 [M+H]⁺.

### Example 7

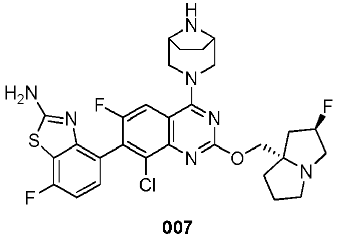

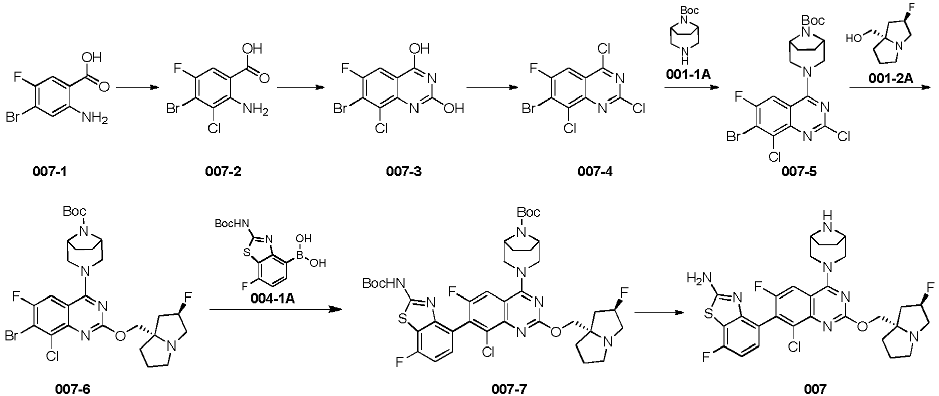

### Step 1: Synthesis of compound 007-2

Compound **007-1** (2.5 g, 10.68 mmol, 1 *eq)* was added to N,N-dimethylformamide (25 mL), and N-chlorosuccinimide (1.57 g, 11.75 mmol, 1.1 *eq)* was added. The mixture was reacted at 50°C for 2 h. After the reaction was completed, 20 mL of water was added to the reaction solution and a solid was precipitated. The solid was filtered and rotary-evaporated to dryness. To the crude product was added 30 mL of dichloromethane and the mixture was slurried with stirring for 1 h. The mixture was filtered. The filter cake was washed with 10 mL*2 of dichloromethane and rotary-evaporated to dryness to give compound **007-2.** ¹H NMR (400 MHz, DMSO-*d₆*) δ = 7.69 - 7.57 (d, *J* = 0.8Hz, 1H); LCMS:(ESI)m/z: 267.9[M+H]⁺.

### Step 2: Synthesis of compound 007-3

Compound **007-2** (3.5 g, 13.04 mmol, 1 eq) and urea (7.83 g, 130.37 mmol, 6.99 mL, 10 eq) were added to a flask. The mixture was reacted at 200 °C for 1 h. After the reaction was completed, the reaction solid was slurried for 1 h using 30 mL of ethyl acetate. The filter cake was filtered and rotary-evaporated to dryness, and then slurried using 30 mL of water for 1 h and filtered. The filter cake was rotary-evaporated to dryness to give compound **007-3.** ¹H NMR (400 MHz, DMSO- *d₆*) δ = 11.38 - 10.97 (m, 2H), 7.79 - 7.68 (d, *J* = 0.8Hz, 1H).

### Step 3: Synthesis of compound 007-4

Compound **007-3** (2 g, 6.81 mmol, 1 *eq)* was added to phosphorus oxychloride (20 mL), and diisopropylethylenediamine (2.64 g, 20.44 mmol, 3.56 mL, 3 *eq)* was added. The mixture was reacted at 100 °C for 3 h. After the reaction was completed, the reaction solution was directly concentrated, and the concentrated product was slowly added to 20 mL of ice water. The mixture was extracted using 10 mL*2 of ethyl acetate. The organic phases were combined, washed using 10 mL*2 of saturated ammonium chloride, then washed with 20 mL of saturated brine, dried with anhydrous sodium sulfate and filtered. The filtrate was concentrated to give compound **007-4.** LCMS:(ESI)m/z: 328.8[M+H]⁺.

### Step 4: Synthesis of compound 007-5

Compound **007-4** (1.7 g, 5.15 mmol, 1 *eq)* was added to anhydrous dichloromethane (20 mL), and triethylamine (1.56 g, 15.44 mmol, 2.15 mL, 3 *eq)* and compound **001-1A** (1.31 g, 6.17 mmol, 1.2 *eq)* were added. The mixture was reacted at 20 °C for 2 h. The reaction solution was rotary-evaporated to dryness directly and the crude product was purified using a column (petroleum ether:ethyl acetate = 100:0-1:1, petroleum ether:ethyl acetate = 3:1) to give compound **007-5.** LCMS:(ESI)m/z: 505.0[M+H]⁺.

### Step 5: Synthesis of compound 007-6

Compound **007-5** (1.2 g, 2.37 mmol, 1 *eq)* was added to N,N-dimethylformamide (12 mL) and anhydrous tetrahydrofuran (12 mL), and compound **001-2A** (566.11 mg, 3.56 mmol, 1.5 *eq)* and cesium carbonate (2.32 g, 7.11 mmol, 3 *eq)* and triethylenediamine (26.59 mg, 237.06 µmol, 26.07 µL, 0.1 *eq)* were added. The mixture was reacted at 20 °C for 20 h. After the reaction was completed, 20 mL of water was added to the reaction solution, and the mixture was extracted using 10 mL*2 of ethyl acetate. The organic phases were combined, washed using 20 mL of saturated brine, dried with anhydrous sodium sulfate and filtered. The filtrate was concentrated, and the crude product was purified using a column (petroleum ether:ethyl acetate = 3:1 - 0:1, dichloromethane : methanol = 100:0 - 10:1, dichloromethane : methanol = 10:1) to give compound **007-6.** LCMS:(ESI)m/z: 628.2[M+H]⁺.

### Step 6: Synthesis of compound 007-7

Compound **007-6** (0.1 g, 159.00 µmol, 1 *eq*) and compound **004-1A** (59.55 mg, 190.80 µmol, 1.2 *eq)* and sodium carbonate (50.56 mg, 477.00 µmol, 3 *eq)* were added to 1,4-dioxane (3 mL) and water (0.6 mL). The atmosphere was replaced with nitrogen three times. [1,1-bis(diphenylphosphino)ferrocene]palladium dichloride (23.27 mg, 31.80 µmol, 0.2 *eq)* was added under nitrogen. The mixture was reacted at 100 °C for 2 h. To the reaction solution was added 3 mL of water, and the mixture was extracted using 3 mL*2 of ethyl acetate. The organic phases were combined, washed using 5 mL of saturated brine, dried with anhydrous sodium sulfate and filtered. The filtrate was concentrated, and the crude product was purified using a column (petroleum ether : ethyl acetate = 10:1-0:1, dichloromethane : methanol = 100:0-10:1, dichloromethane : methanol = 10:1) to give compound **007-7.** LCMS:(ESI)m/z: 816.3[M+H]⁺.

### Step 7: Synthesis of compound 007 hydrochloride

Compound **007-7** (90 mg, 112.73 µmol, 1 *eq)* was added to trifluoroacetic acid (0.5 mL) and anhydrous dichloromethane (3 mL). The mixture was reacted at 20 °C for 3.5 h. The reaction solution was directly concentrated and purified by preparative chromatography (column: Phenomenex Luna 80*30 mm*3 µm; mobile phase: [water (HCl)-acetonitrile]; acetonitrile %: 5%-35%, 8 min) to give compound **007** hydrochloride. ¹H NMR (400 MHz, CD₃OD) δ = 7.83 - 7.77 (m, 1H), 7.32 - 7.20 (m, 1H), 7.12 - 6.96 (m, 1H), 5.67 - 5.46 (m, 1H), 4.81 - 4.60 (m, 4H), 4.30 - 4.20 (m, 2H), 3.99 - 3.85 (m, 4H), 3.55 - 3.42 (m, 2H), 2.79 - 2.57 (m, 2H), 2.52 - 2.44 (m, 1H), 2.41 - 2.29 (m, 2H), 2.22 - 2.02 (m, 5H); LCMS:(ESI)m/z: 616.2[M+H]⁺.

### Example 8

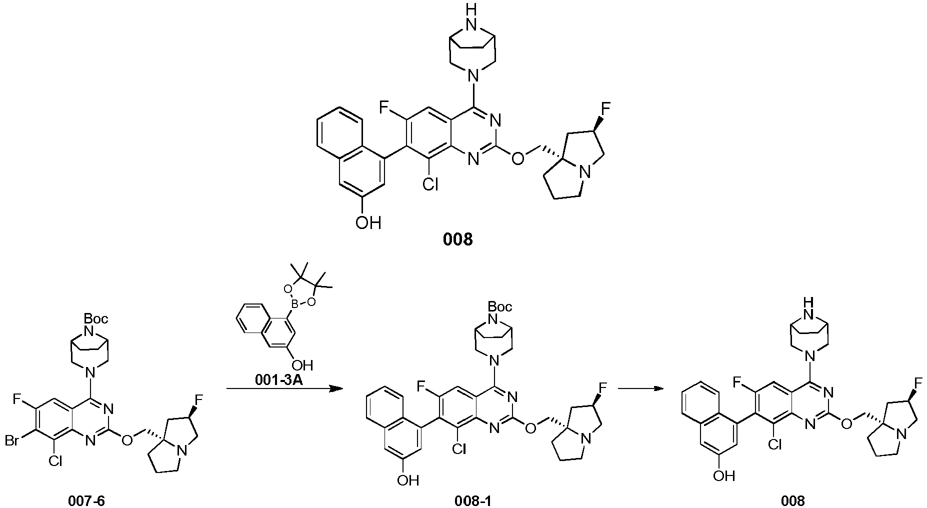

### Step 1: Synthesis of compound 008-1

Compound **007-6** (0.1 g, 159.00 µmol, 1 *eq)* and compound **001-3A** (64.43 mg, 238.50 µmol, 1.5 *eq)* and sodium carbonate (33.70 mg, 318.00 µmol, 2 *eq)* were added to 1,4-dioxane (2 mL) and water (0.4 mL). The atmosphere was replaced with nitrogen three times. [1,1-bis(diphenylphosphino)ferrocene]palladium dichloride dichloromethane (12.98 mg, 15.90 µmol, 0.1 *eq)* was added. The mixture was reacted at 100 °C for 1 h. The reaction solution was diluted using 3 mL of ethyl acetate, washed using 2 mL of water and 5 mL of saturated brine, dried with anhydrous sodium sulfate and filtered. The filtrate was concentrated to give compound **008-1** as a black liquid. LCMS:(ESI)m/z: 692.2[M+H]⁺.

### Step 2: Synthesis of compound 008 hydrochloride

Compound **008-1** (70.00 mg, 101.13 µmol, 1 *eq)* was added to anhydrous dichloromethane (1 mL), and trifluoroacetic acid (0.2 mL) was added. The mixture was reacted for 2 h at 20 °C. The reaction solution was concentrated directly and purified by pre-HPLC (column: Phenomenex Luna 80*30mm*3µm; mobile phase: [water (HCl) - acetonitrile]; acetonitrile %: 5%-35%, 8 min) to give compound **008** hydrochloride. ¹H NMR (400 MHz, DMSO-*d₆*) δ = 11.48 - 11.32 (m, 1H), 9.94 (d, *J* = 0.8Hz, 1H), 9.76 - 9.60 (m, 1H), 7.92 (d, *J* = 0.8Hz, 1H), 7.81 d, *J* = 0.8Hz, 1H), 7.44 (t, *J* = 0.8Hz, 1H), 7.30 (d, *J* = 0.4Hz, 1H), 7.25 - 7.14 (m, 2H), 7.09 (d, *J* = 0.4Hz, 1H ), 5.68 - 5.43 (m, 1H), 4.71 - 4.57 (m, 2H), 4.48 (d, *J* = 1.2 Hz, 2H), 4.15 (s, 2H), 3.98-3.75 (m, 6H), 3.35 - 3.22 (m, 1H), 2.70 - 2.57 (m, 1H), 2.38 - 2.29 (m, 1H), 2.25 - 2.11 (m, 2H), 2.08 - 1.97 (m, 5H); LCMS:(ESI)m/z: 592.2[M+H]⁺.

### Example 9

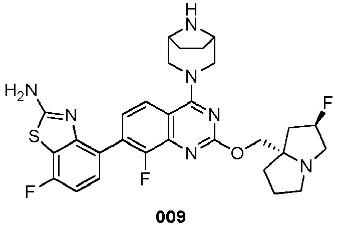

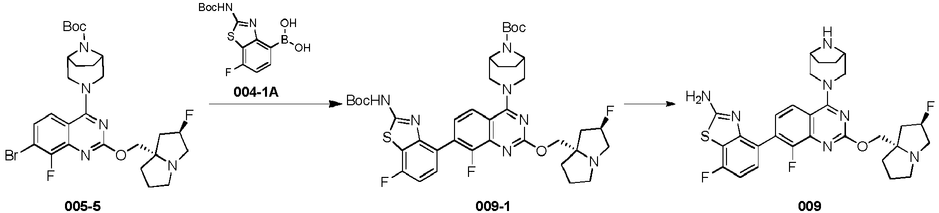

### Step 1: Synthesis of compound 009-1

Compound **005-5** (0.05 g, 84.11 µmol, 1 *eq)* and compound **004-1A** (31.50 mg, 100.93 µmol, 1.2 *eq)* and sodium carbonate (26.74 mg, 252.32 µmol, 3 *eq)* were added to 1,4-dioxane (1 mL) and water (0.2 mL). The atmosphere was replaced with nitrogen three times. [1,1-bis(diphenylphosphino)ferrocene]palladium dichloride (12.31 mg, 16.82 µmol, 0.2 *eq)* was added under nitrogen. The mixture was reacted at 100 °C for 2 h. 3 mL of water was added to the reaction solution, and the mixture was extracted with 3 mL*2 of ethyl acetate. The organic phases were combined, washed with 5 mL of saturated brine, dried with anhydrous sodium sulfate and filtered. The filtrate was concentrated to give compound **009-1.** LCMS:(ESI)m/z: 782.3[M+H]⁺.

### Step 2: Synthesis of compound 009 hydrochloride

Compound **009-1** (60 mg, 76.74 µmol, 1 *eq)* was added to anhydrous dichloromethane (3 mL), and trifluoroacetic acid (0.6 mL) was added. The mixture was reacted at 20 °C for 2.5 h. After the reaction was completed, the reaction solution was directly concentrated and purified by a preparative column: Phenomenex luna C18 80*40 mm*3 µm; mobile phase: [water (HCl)-acetonitrile]; Acetonitrile %: 12%-28%, 7 min, to give compound **009** hydrochloride. ¹H NMR (400 MHz, CD₃OD) δ = 8.13 (d, *J* = 1.2 Hz, 1H), 7.73 (t, *J* = 0.8 Hz, 1H), 7.66 - 7.57 (m, 1H), 7.28 (t, *J* = 1.2 Hz, 1H), 5.76 - 5.50 (m, 1H), 5.13 - 4.97 (m, 4H), 4.40 - 4.27 (m, 2H), 4.25 - 4.14 (m, 2H), 4.13 - 3.83 (m, 3H), 3.55 - 3.43 (m, 1H), 2.89 - 2.60 (m, 2H), 2.59-2.45 (m, 1H), 2.44 - 2.25 (m, 3H), 2.23 - 2.05 (m, 4H); LCMS:(ESI)m/z: 582.2 [M+H]⁺.

### Example 10

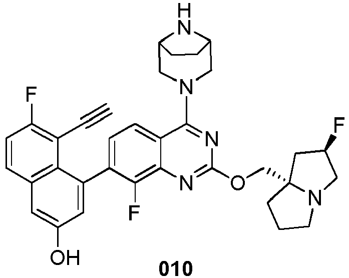

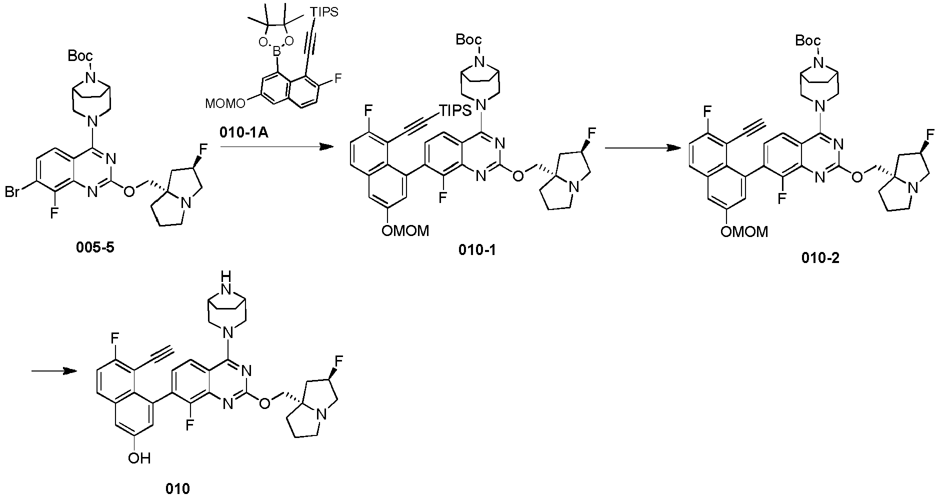

### Step 1: Synthesis of compound 010-1

Compound **005-5** (0.2 g, 336.42 µmol, 1 *eq*) and compound **010-1A** (206.91 mg, 403.71 µmol, 1.2 *eq*) and sodium carbonate (106.97 mg, 1.01 mmol, 3 *eq*) were added to 1,4-dioxane (1.5 mL) and water (0.3 mL). The atmosphere was replaced with nitrogen three times. [1,1-bis(diphenylphosphino)ferrocene]palladium dichloride (49.23 mg, 67.28 µmol, 0.2 *eq*) was added under nitrogen. The mixture was reacted at 100 °C for 2 h. After the reaction was completed, 5 mL of ethyl acetate was added to the reaction solution. The mixture was washed using 5 mL of water and saturated brine, dried with anhydrous sodium sulfate and filtered. The filtrate was concentrated, and the crude product was purified using a column (petroleum ether:ethyl acetate = 10:1-0:1, dichloromethane:methanol = 100:0-10:1) to give compound **010-1.** LCMS:(ESI)m/z: 900.4[M+H]⁺.

### Step 2: Synthesis of compound 010-2

Compound **010-1** (0.25 g, 277.73 µmol, 1 *eq*) was added to anhydrous tetrahydrofuran (5 mL), and tetramethylammonium fluoride tetrahydrate (129.34 mg, 1.39 mmol, 5 *eq*) was added. The mixture was reacted at 50 °C for 19 h. After the reaction was completed, 5 mL of ethyl acetate was added to the reaction solution. The mixture was washed respectively using 5 mL of water and 5 mL of saturated brine, dried with anhydrous sodium sulfate and filtered. The filtrate was concentrated, and the crude product was purified by preparative chromatography (column: Waters Xbridge BEH C18 100*30mm*10µm; mobile phase: [water (ammonia)-acetonitrile]; acetonitrile %: 70%-90%, 8 min) to give compound **010-2.** LCMS:(ESI)m/z:744.3[M+H]⁺.

### Step 3: Synthesis of compound 010 hydrochloride

Compound **010-2** (0.03 g, 40.33 µmol, 1 *eq*) was added to acetonitrile (2 mL) and hydrochloric acid/dioxane (4 M, 201.66 µL, 20 *eq*) was added. The mixture was reacted at 25 °C for 1 hr. After the reaction was completed, the reaction solution was filtered under nitrogen. The filter cake was washed with acetonitrile (2 mL*2) and rotary-evaporated to dryness to give compound **010** hydrochloride. LCMS:(ESI)m/z:600.3[M+H]⁺; ¹H NMR (400 MHz, CD₃OD) δ = 7.92 - 7.70 (m, 2H), 7.38 - 7.20 (m, 3H), 7.08 (d, *J* = 2.4 Hz, 1H), 5.41-5.3(m, 1H), 4.65 - 4.44 (m, 4H), 4.38 - 4.19 (m, 2H), 3.75 - 3.56 (m, 5H), 3.24 - 3.15 (m, 1H), 3.11 - 2.97 (m, 1H), 2.46 - 2.10 (m, 3H), 2.08 - 1.98 (m, 2H), 1.96 - 1.76 (m, 5H).

### Step 4: Synthesis of compound 010

The **010** hydrochloride was added to 20 mL of saturated sodium bicarbonate solution, pH 8, and the mixture was extracted with ethyl acetate (10 mL*2). The organic phases were combined, washed with 20 mL of saturated brine, dried with anhydrous sodium sulfate and filtered. The filtrate was concentrated to give compound **010.**

### Example 11

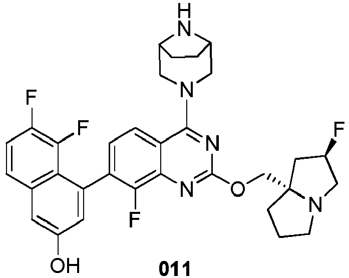

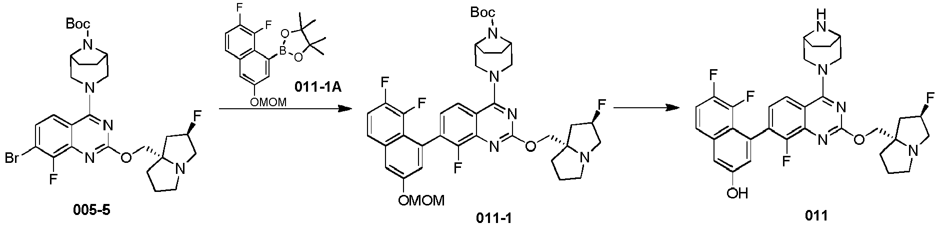

### Step 1: Synthesis of compound 011-1

Compound **005-5** (0.1 g, 168.21 µmol, 1 *eq*) and compound **011-1A** (70.68 mg, 201.85 µmol, 1.2 *eq*) and sodium carbonate (53.49 mg, 504.63 µmol, 3 *eq*) were added to 1,4-dioxane (1 mL) and water (0.2 mL). The atmosphere was replaced with nitrogen three times. [1,1-bis(diphenylphosphino)ferrocene]palladium dichloride (12.31 mg, 16.82 µmol, 0.1 *eq*) was added under nitrogen. The mixture was reacted at 100 °C for 1.5 h. After the reaction was completed, 5 mL of ethyl acetate was added to the reaction solution. The mixture was washed using 10 mL of water, 10 mL of saturated brine, dried with anhydrous sodium sulfate and filtered. The filtrate was concentrated to give compound **011-1.** LCMS:(ESI)m/z: 738.2[M+H]⁺.

### Step 2: Synthesis of compound 011 hydrochloride

Compound **011-1** (0.15 g, 203.31 µmol, 1 *eq*) was added to acetonitrile (5 mL) and hydrochloric acid/dioxane (4 M, 508.28 µL, 10 *eq*) was added. The mixture was reacted at 25 °C for 3 h. Solid was precipitated. The reaction solution was filtered under nitrogen and the filter cake was washed with 3 mL*2 of acetonitrile. The crude product was purified by preparative chromatography (column: Phenomenex Luna 80*30 mm*3 µm; mobile phase: [water (hydrochloric acid)-acetonitrile]; acetonitrile %: 10%-30%, 8 min) to give compound **011** hydrochloride. LCMS:(ESI)m/z: 594.2[M+H]⁺; ¹H NMR (400 MHz, CD₃OD) δ = 8.12 (d, *J* = 8.8 Hz, 1H), 7.70 - 7.58 (m, 2H), 7.46 - 7.33 (m, 2H), 7.15 (s, 1H), 5.66-5.54 (m, 1H), 5.19 - 4.89 (m, 5H), 4.36 - 4.02 (m, 5H), 4.00 - 3.84 (m, 2H), 3.53 - 3.32 (m, 1H), 2.76 - 2.58 (m, 1H), 2.55-2.47 (m, 1H), 2.42 - 2.30 (m, 2H), 2.29-2.26 (m, 1H), 2.25 - 2.12 (m, 4H).

### Example 12

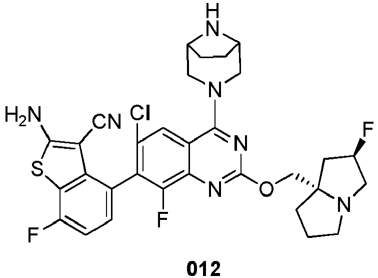

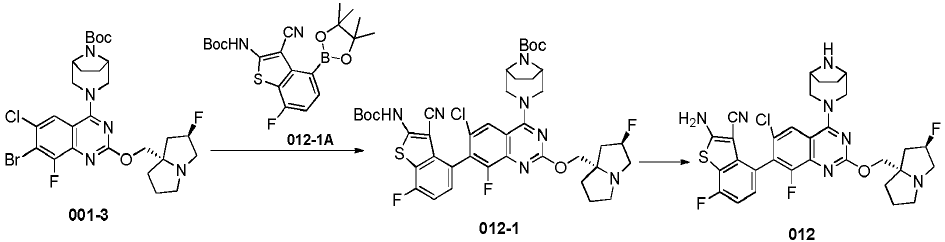

### Step 1: Synthesis of compound 012-1

Compound **001-3** (100 mg, 159.00 µmol, 1 eq) and compound **012-1A** (79.81 mg, 190.80 µmol, 1.2 eq) were added to 1,4-dioxane (3 mL) and water (0.6 mL), followed by 4-(di-tert-butylphosphino)-N,N-dimethylaniline (8.44 mg, 31.80 µmol, 0.2 eq), tris(dibenzylideneacetone)dipalladium (14.56 mg, 15.90 µmol, 0.1 eq) and potassium carbonate (65.93 mg, 477.00 µmol, 3 eq). The atmosphere was replaced with nitrogen three times. The mixture was stirred to react for 15 h in an oil bath at 95°C. After the reaction was completed, the crude product was separated by a preparative column: Welch Xtimate C18 100*40mm*3µm; mobile phase: [water (trifluoroacetic acid)-acetonitrile]; acetonitrile %: 50%-70%, 5.5 min, to give compound **012-1.** LCMS:(ESI)m/z: 840.5[M+H]⁺.

### Step 2: Synthesis of compound 012 formate

Compound **012-1** (20 mg, 23.80 µmol, 1 eq) was dissolved in trifluoroacetic acid (3 mL) and the mixture was stirred to react at 25 °C for 1 h. After the reaction was completed, the mixture was rotary-evaporated to dryness and the crude product was separated by a preparative column: Phenomenex C18 150*40 mm*5 µm; mobile phase: [water (formic acid)-acetonitrile]; acetonitrile %: 6%-36%, 10 min, to give compound **012** formate. LCMS:(ESI)m/z: 640.5[M+H]⁺.

### Example 13

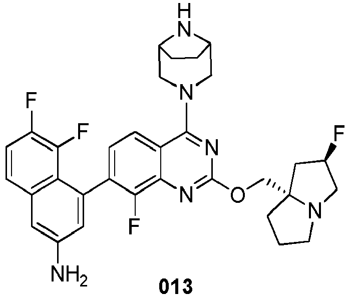

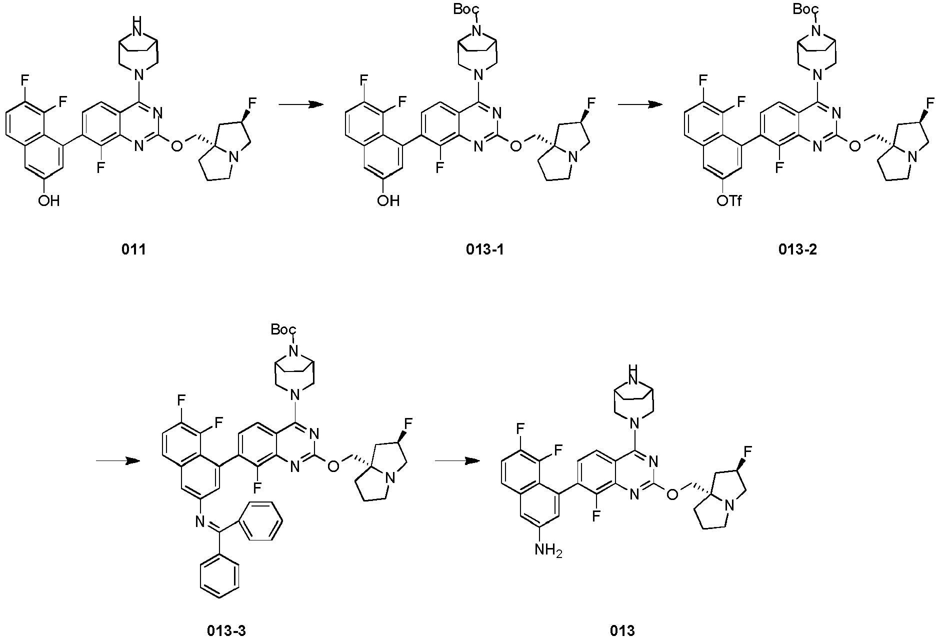

### Step 1: Synthesis of compound 013-1

Compound **011** hydrochloride (0.4 g, 600.12 µmol, 1 *eq*) was added to anhydrous dichloromethane (4 mL), and diisopropylethylenediamine (387.80 mg, 3.00 mmol, 522.64 µL, *5 eq*) and di-tert-butyl dicarbonate (157.17 mg, 720.14 µmol, 165.44 µL, 1.2 eq) were added. The mixture was reacted for 1 h at 25 °C. After the reaction was completed, 10 mL of water and 20 mL of dichloromethane were added, and then 1 M hydrochloric acid was added to adjust the pH of the aqueous phase to about 2. The aqueous phase was extracted once with 20 mL of dichloromethane. The organic phases were combined, washed once with 20 mL of saturated brine, dried over anhydrous sodium sulfate, and filtered. The filtrate was concentrated to give compound **013-1.** LCMS:(ESI)m/z: 694.6[M+H]⁺.

### Step 2: Synthesis of compound 013-2

Compound **013-1** (0.4 g, 547.80 µmol, 1 eq) was added to anhydrous dichloromethane (20 mL), and diisopropylethylenediamine (24.80 mg, 3.29 mmol, 572.50 µL, 6 eq) was added. Trifluoromethanesulfonic anhydride (463.67 mg, 1.64 mmol, 271.15 µL, 3 eq) was added at 0 °C, and the mixture was reacted at 0 °C for 1 h. After the reaction was completed, 10 mL of water was added to the reaction solution and the mixture was stirred for 10 min. Then the mixture was left to stand and the layers were separated. The aqueous phase was extracted once with 20 mL of dichloromethane. The organic phases were combined, dried over anhydrous sodium sulfate, and filtered. The filtrate was concentrated to give a crude product, which was purified using a column (ethyl acetate:petroleum ether = 0-60%) to give compound **013-2.** LCMS:(ESI)m/z: 826.4[M+H]⁺.

### Step 3: Synthesis of compound 013-3

Compound **013-2** (0.2 g, 231.95 µmol, 1 eq) was added to anhydrous toluene (6 mL), and benzophenone imine (84.07 mg, 463.90 µmol, 77.85 µL, 2 eq), cesium carbonate (226.72 mg, 695.85 µmol, 3 eq), and 4,5-bis(diphenylphosphino)-9,9-dimethylxanthene (26.84 mg, 46.39 µmol, 0.2 eq) were added. The atmosphere was replaced with nitrogen three times, and tris(dibenzylideneacetone)dipalladium (21.24 mg, 23.20 µmol, 0.1 eq) was added. The atmosphere was replaced with nitrogen three times. The mixture was stirred at 100 °C for 12 h. After the reaction was completed, 20 mL of water and 20 mL of ethyl acetate were added. The mixture was stirred for 5 min, and then left to stand. The layers were separated. The aqueous phase was extracted once with 10 mL of ethyl acetate. The organic phases were combined, washed with saturated brine (20 mL*3), dried over anhydrous sodium sulfate, and filtered. The filtrate was concentrated to give a crude product, which was purified using a column (ethyl acetate:petroleum ether = 0-80%) to give compound **013-3.** LCMS:(ESI)m/z: 857.6[M+H]⁺.

### Step 4: Synthesis of compound 013 hydrochloride

Compound **013-3** (0.14 g, 163.37 µmol, 1 *eq*) was added to acetonitrile (3 mL) and hydrochloric acid/dioxane (4 M, 408.43 µL, 10 *eq*) was added. The mixture was reacted at 25 °C for 2 h. Solid was precipitated. After the reaction was completed, the reaction solution was filtered and the filter cake was washed with 5 mL of acetonitrile. The crude product was purified by preparative chromatography (column: Phenomenex Luna 80*30 mm*3 µm; mobile phase: [water (hydrochloric acid)-acetonitrile]; acetonitrile %: 10%-40%, 8 min) to give compound **013** hydrochloride. LCMS:(ESI)m/z: 593.2[M+H]⁺. ¹H NMR (400 MHz, CD₃OD) δ ppm 8.19-8.11 (m, 2H), 8.00 (dd, J = 8.57, 4.20 Hz, 1H), 7.76-7.66 (m, 2H), 7.59 (t, J = 2.00 Hz, 1H), 5.68-5.55 (m, 1H), 5.17-5.00 (m, 2H), 5.00-4.91 (m, 2H), 4.34 (s, 2H), 4.28- 4.10 (m, 2H), 4.09-3.84 (m, 3H), 3.58-3.39 (m, 1H), 2.59-2.86 (m, 2H), 2.56-2.47 (m, 1H), 2.43-2.33 (m, 2H),2.33-2.12 (m, 5H).

### Example 14

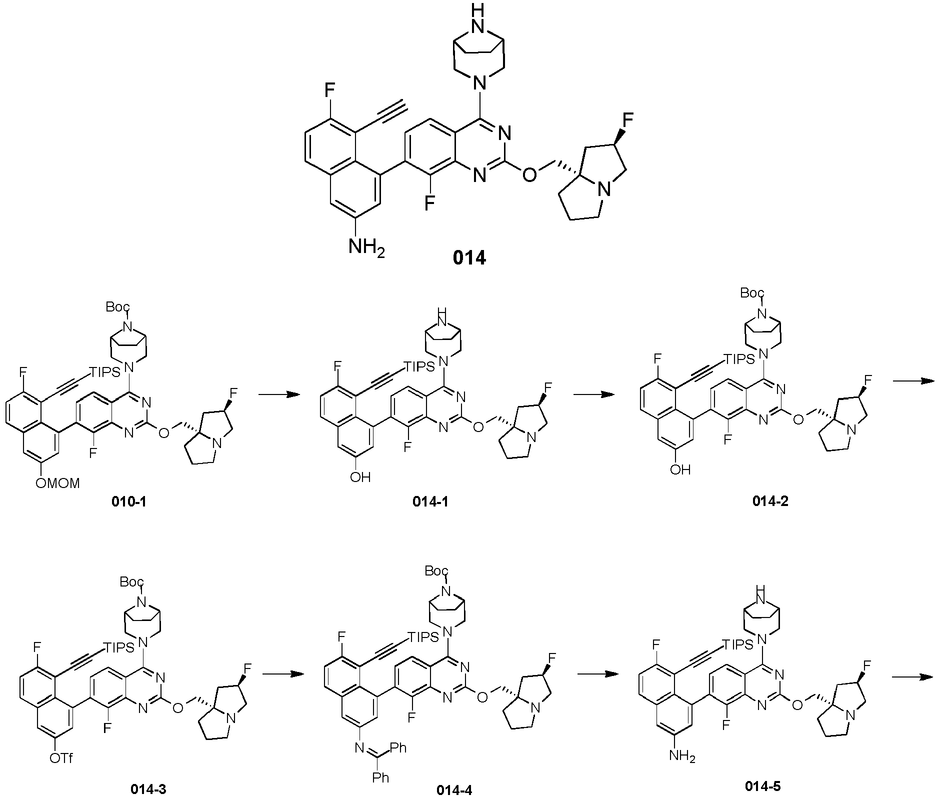

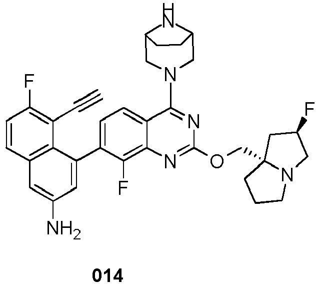

### Step 1: Synthesis of compound 014-1 hydrochloride

Compound **010-1** (250 mg, 277.73 µmol, 1 eq) was added to acetonitrile (8 mL) and hydrochloric acid/dioxane (4 M, 2.08 mL, 30 *eq*) was added. The mixture was reacted at 25 °C for 1 h. Solid was precipitated. After the reaction was completed, the reaction solution was filtered and the filtrate was washed with 5 mL of acetonitrile, and then 10 mL of methanol was used to dissolve the filter cake. The solution was concentrated under reduced pressure to give compound **014-1** hydrochloride. LCMS:(ESI)m/z:756.7[M+H]⁺. ¹H NMR (400 MHz, DMSO-*d₆*) δ ppm 11.22 (br d, J = 13.64 Hz, 1H), 9.93 (br d, J = 9.12 Hz, 1H), 9.56 (br s, 1H), 7.98 (dd, J = 9.00, 6.00 Hz, 1H), 7.87 (br d, J = 8.64 Hz, 1H), 7.53-7.35 (m, 2H), 7.10-7.04 (m, 1H), 5.68-5.46 (m, 1H), 4.74-4.44 (m, 3H), 4.22-4.11 (m, 3H), 4.01-3.84 (m, 2H), 3.80-3.79 (m, 1H), 3.74-3.62 (m, 1H), 3.31 (br s, 1H), 3.16 (s, 3H), 2.46-1.88 (m, 8H), 0.80 (dd, J = 16.64, 7.38 Hz, 18H), 0.51-0.45 (m, 3H).

### Step 2: Synthesis of compound 014-2

Compound **014-1** hydrochloride (200 mg, 264.56 µmol, 1 eq) was added to anhydrous dichloromethane (5 mL) and diisopropylethylenediamine (170.96 mg, 1.32 mmol, 230.40 µL, 5 eq), and di-tert-butyl dicarbonate (57.74 mg, 264.56 µmol, 60.78 µL, 1 eq) were added. The mixture was reacted at 25 °C for 1 h. After the reaction was completed, 5 mL of water and 10 mL of dichloromethane were added, and then 1 M hydrochloric acid was added to adjust the pH of the aqueous phase to about 2. The aqueous phase was extracted once with 5 mL of dichloromethane. The organic phases were combined, dried over anhydrous sodium sulfate, and filtered. The filtrate was concentrated to give compound **014-2.** LCMS:(ESI)m/z:856.4 [M+H]⁺.

### Step 3: Synthesis of compound 014-3

Compound **014-2** (220 mg, 256.98 µmol, 1 eq) was added to anhydrous dichloromethane (10 mL), and diisopropylethylenediamine (199.28 mg, 1.54 mmol, 268.57 µL, 6 eq) was added. Trifluoromethanesulfonic anhydride (217.51 mg, 770.94 µmol, 127.20 µL, 3 eq) was added at 0 °C. The mixture was reacted at 0 °C for 1 h. After the reaction was completed, 10 mL of water was added. The mixture was stirred for 10 min, and then left to stand. The layers were separated. The aqueous phase was extracted once with 10 mL of dichloromethane. The organic phases were combined, dried over anhydrous sodium sulfate, and filtered. The filtrate was concentrated to give a crude product, which was purified using a column (ethyl acetate:petroleum ether = 0-60%) to give compound **014-3.** LCMS:(ESI)m/z:988.3 [M+H]⁺.

### Step 4: Synthesis of compound 014-4

Compound **014-3** (140 mg, 141.68 µmol, 1 eq) was added to anhydrous toluene (2.8 mL), and benzophenone imine (51.35 mg, 283.35 µmol, 47.55 µL, 2 eq), cesium carbonate (138.48 mg, 425.03 µmol, 3 eq), and 4,5-bis(diphenylphosphino)-9,9-dimethylxanthene (16.40 mg, 28.34 µmol, 0.2 eq) were added. The atmosphere was replaced with nitrogen three times. Tris(dibenzylideneacetone)dipalladium (12.97 mg, 14.17 µmol, 0.1 eq) was added. The atmosphere was replaced with nitrogen three times. The mixture was stirred at 100°C for 12hr. After the reaction was completed, 10 mL of water and 10 mL of ethyl acetate were added and the mixture was stirred for 5 min, and then left to stand. The layers were separated. The aqueous phase was extracted once with 10 mL ethyl acetate. The organic phases were combined, washed with saturated brine (20 mL*3), dried over anhydrous sodium sulfate, and filtered. The filtrate was concentrated to give a crude product, which was purified using a column (ethyl acetate:petroleum ether = 0-60%) to give compound **014-4.** LCMS:(ESI)m/z: 1019.5 [M+H]⁺.

### Step 5: Synthesis of compound 014-5 hydrochloride

Compound **014-4** (100 mg, 98.10 µmol, 1 eq) was added to acetonitrile (2.5 mL) and hydrochloric acid/dioxane (8 M, 245.26 µL, 20 *eq*) was added. The mixture was reacted at 25 °C for 2 h. After the reaction was completed, the reaction solution was filtered and the filter cake was washed with 3 mL of acetonitrile. Then 5 mL of methanol was used to dissolve the filter cake. The solution was concentrated under reduced pressure to give compound **014-5** hydrochloride. LCMS:(ESI)m/z:755.4 [M+H]⁺.

### Step 6: Synthesis of compound 014

The compound **014-5** hydrochloride (65 mg, 78.51 µmol, 1 eq) was added to N,N-dimethylformamide (1 mL), and anhydrous potassium carbonate (70 mg, 506.49 µmol, 6.45 eq), and cesium fluoride (40 mg, 263.33 µmol, 3.35 eq) were added. The mixture was reacted at 60°C for 3 h. After the reaction was completed, the reaction solution was diluted by adding 10 mL of ethyl acetate, and filtered. The mother liquor was washed with saturated brine (10 mL*3), dried with anhydrous sodium sulfate, and filtered. The filtrate was concentrated to give a crude product. The crude product was purified by preparative chromatography (column: Phenomenex Luna 80*30mm*3µm; mobile phase: [water (hydrochloric acid)-acetonitrile]; acetonitrile %: 1%-40%, 8min), and the fraction was extracted by petroleum ether (10 mL*3). The aqueous phase was adjusted to a pH of about 9 by adding ammonia dropwise, and extracted with ethyl acetate (10 mL*2). The organic phase was collected and concentrated under reduced pressure to give compound **014.** LCMS:(ESI)m/z: 599.3 [M+H]⁺. ¹H NMR (400 MHz,CD₃OD) δ ppm 7.79-7.66 (m, 2H), 7.26-7.17 (m, 2H), 7.14 (d, J = 2.38 Hz, 1H), 7.02 (d, J = 2.26 Hz, 1H), 5.38-5.23(m, 1H), 4.50 (br t, J = 11.56 Hz, 2H), 4.33-4.16 (m, 2H), 3.68-3.55 (m, 4H), 3.27-3.17 (m, 3H), 3.14 (d, J = 9.00 Hz, 1H), 3.07-2.97 (m, 1H), 2.41-2.12 (m, 3H), 2.05-1.94 (m, 2H), 1.86 (br s, 5H).

### Example 15

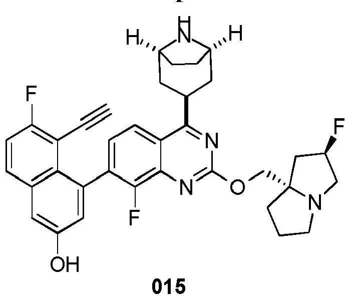

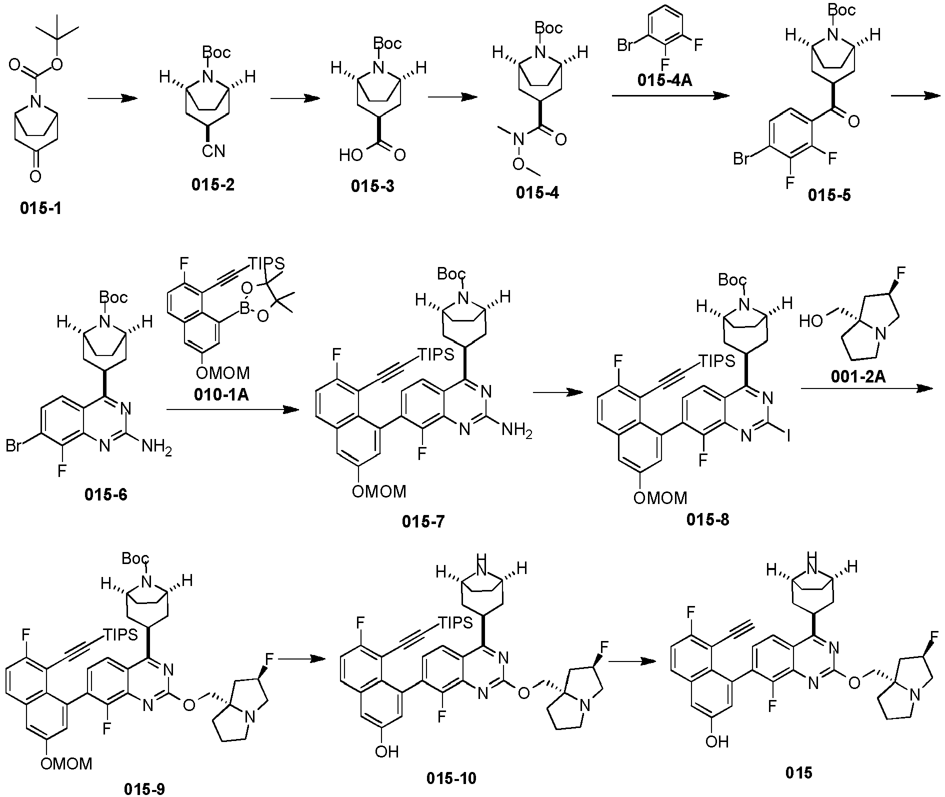

### Step 1: Synthesis of compound 015-2

To the reaction flask were added compound **015-1** (30 g, 133.17 mmol, 1 eq), ethylene glycol dimethyl ether (900 mL) and ethanol (19.5 mL). The mixture was cooled down to 0 °C under nitrogen and potassium tert-butoxide (60.00 g, 534.71 mmol, 4.02 eq) and p-toluenesulfonyl isonitrile (51.99 g, 266.29 mmol, 2.00 eq) were added. The mixture was heated to 60 °C and stirred for 12 hours. After the reaction was completed, saturated brine (500 mL) was added, and the mixture was extracted with ethyl acetate (500 mL*3). The combined organic phase was washed with saturated brine (400 mL), dried with anhydrous sodium sulfate, and filtered. The filtrate was concentrated under reduced pressure. The crude product was purified by column chromatography (ethyl acetate:petroleum ether = 0-50%) to give compound **015-2.** LCMS:(ESI)m/z:137.0[M+H-Boc]⁺. ¹H NMR (400 MHz, CDCl₃) δ = 4.28-4.26 (m, 2H), 3.01-2.94 (m, 1H), 2.05-2.02 (m, 4H), 1.88-1.86 (m, 2H), 1.62-1.59 (m, 2H), 1.48-1.46 (m, 9H).

### Step 2: Synthesis of compound 015-3

To the reaction flask were added compound **015-2** (16 g, 67.71 mmol, 1 *eq*)*,* ethanol (240 mL), and water (240 mL). Potassium hydroxide (22.79 g, 406.25 mmol, 6 *eq*) was added and the mixture was heated to 80 °C and stirred for 12 hours. After the reaction was completed, the reaction solution was cooled to 0 °C and the pH was adjusted to about 3 with hydrochloric acid (1 N). The reaction was extracted with ethyl acetate (500 mL*3). The combined organic phase was washed with saturated brine (200 mL), dried over anhydrous sodium sulfate, and filtered. The filtrate was concentrated under reduced pressure to give compound **015-3.** ¹H NMR (400 MHz, CDCl₃) δ = 8.90-8.12 (m, 1H), 4.30-4.22 (m, 2H), 2.86-2.82 (m, 1H), 2.00-1.98 (m, 2H), 1.78-1.77 (m, 2H), 1.75-1.74 (m, 2H), 1.66-1.64 (m, 2H), 1.46 (s, 9H).

### Step 3: Synthesis of compound 015-4

To the reaction flask were added compound **015-3** (17 g, 66.59 mmol, 1 *eq*)*,* triethylamine (13.48 g, 133.17 mmol, 18.54 mL, 2 *eq*) and dichloromethane (170 mL). Carbonyl diimidazole (12.96 g, 79.90 mmol, 1.2 *eq*) and N-methyl-N-methoxyamine hydrochloride (9.74 g, 99.88 mmol, 1.5 *eq,* HCl) were added. The mixture was stirred at 20 °C for 1 hr. After the reaction was completed, water (200 mL) was added. The layers were separated, and the aqueous layer was extracted with DCM (200 mL*2). The combined organic phase was washed with saturated brine (200 mL), dried over anhydrous sodium sulfate, and filtered. The filtrate was concentrated under reduced pressure. The crude product was purified by column chromatography (ethyl acetate:petroleum ether = 0-50%) to give compound **015-4.** LCMS:(ESI)m/z: 321.0 [M+Na]⁺. ¹H NMR (400 MHz, CDCl₃) δ = 4.31-4.21 (m, 2H), 3.74-3.71 (m, 3H), 3.26-3.21 (m, 1H), 3.16 (s, 3H), 2.01-1.96 (m, 4H), 1.93-1.84 (m, 2H), 1.68-1.66 (m, 2H), 1.46 (s, 9H).

### Step 4: Synthesis of compound 015-5

The reaction flask was charged with compound **015-4A** (12.08 g, 62.60 mmol, 7.02 mL, 1.33 *eq*) and tetrahydrofuran (420 mL). The mixture was cooled down to -70 °C under nitrogen and lithium diisopropylamine (2 M, 31.20 mL, 1.33 *eq*) was added. The mixture was stirred for 1 h at -70 °C. A solution of compound **015-4** (14 g, 46.92 mmol, 1 *eq*) in tetrahydrofuran (14 mL) was added at -60 ~ -70 °C. The mixture was stirred for another 0.5 h. After the reaction was completed, the reaction solution was quenched by pouring into saturated aqueous ammonium chloride solution (400 mL). The mixture was extracted with ethyl acetate (400 mL*2). The combined organic phase was dried over anhydrous sodium sulfate, and filtered. The filtrate was concentrated under reduced pressure. The crude product was purified by column chromatography (ethyl acetate:petroleum ether = 0-15%) to give compound **015-5.** LCMS:(ESI)m/z: 451.9 [M+Na]⁺. ¹H NMR (400 MHz, CDCl₃) δ = 7.45-7.39 (m, 2H), 4.37-4.27 (m, 2H), 3.62-3.56 (m, 1H), 2.07-2.04 (m, 2H), 1.88-1.85 (m, 2H), 1.77-1.75 (m, 4H), 1.48-1.47 (m, 9H).

### Step 5: Synthesis of compound 015-6

To the reaction flask were added compound **015-5** (4 g, 9.30 mmol, 1 *eq*)*,* guanidine hydrochloride (1.78 g, 18.59 mmol, 3.26 µL, 2 *eq,* HCl), cesium carbonate (6.06 g, 18.59 mmol, 2 *eq*) and N-methylpyrrolidone (40 mL). The mixture was stirred at 120 °C for 12 h. After the reaction was completed, the reaction was cooled down to room temperature. Water (100 mL) was added and a yellow solid was not dissolved. The mixture was stirred with ethyl acetate (20 mL) and filtered. The filtrate was concentrated under reduced pressure to give a yellow solid of 2.5 g. The filtrate was extracted with EA (50 mL*2) and the combined organic phase was washed with saturated brine (50 mL × 2), dried over anhydrous sodium sulfate, and filtered. The filtrate was concentrated under reduced pressure and the crude product was purified by column chromatography (ethyl acetate:petroleum ether = 0-15%). The product was combined with the filter cake to give compound **015-6.** LCMS:(ESI)m/z: 451.0[M+H]⁺. ¹H NMR (400 MHz, CDCl₃) δ = 7.58-7.56 (m, 1H), 7.36-7.32 (m, 1H), 5.47 (s, 2H), 4.43-4.33 (m, 2H), 3.96-3.89 (m, 1H), 2.29-2.13 (m, 4H), 1.91-1.87 (m, 2H), 1.71.-1.67 (m, 2H), 1.51 (s, 9H).

### Step 6: Synthesis of compound 015-7

Compound **016-6** (1.2 g, 2.66 mmol, 1 *eq*)*,* compound **010-1A** (1.50 g, 2.92 mmol, 1.1 *eq*), and anhydrous potassium phosphate (1.13 g, 5.32 mmol, 2 *eq*) were dissolved in 1,4-dioxane (12 mL) and water (2.5 mL). The atmosphere was replaced with nitrogen three times. (2-dicyclohexylpho sphino-2',6'-dimethoxy-1,1'-biphenyl) [2-(2'-amino-1,1'-biphenyl)]palladium (II) chloride (191.60 mg, 265.88 µmol, 0.1 *eq*) was added. The atmosphere was replaced with nitrogen three times. The mixture was reacted at 100° C for 3 h. After the reaction was completed, the mixture was poured into water (10 mL). The mixture was extracted three times with ethyl acetate (10 mL*3). The organic layer was washed with saturated brine (10 mL), dried over anhydrous sodium sulfate, and filtered. The filtrate was concentrated to give a crude product. The crude product was purified by column chromatography (petroleum ether/ethyl acetate = 50/1-0/1) to give the product. The product was then slurried with petroleum ether/methyl tert-butyl ether = 5/1 (15 mL) for 30 min, and filtered. The solid was collected to give compound **015-7.** LCMS:(ESI)m/z: 757.4[M+H]⁺.

### Step 7: Synthesis of compound 015-8

The reaction flask was charged with compound **015-7** (600 mg, 792.61 µmol, 1 *eq*) and tetrahydrofuran (12 mL). The atmosphere was replaced with nitrogen three times. Cuprous iodide (150.95 mg, 792.61 µmol, 1 *eq*)*,* isoamyl nitrite (278.56 mg, 2.38 mmol, 320.19 µL, 3 *eq*)*,* and diiodomethane (1.06 g, 3.96 mmol, 319.71 µL, 5 *eq*) were added. The reaction was heated to 80°C and stirred for 3 hours. After the reaction was completed, the reaction solution was concentrated. The crude product was purified by column chromatography (ethyl acetate:petroleum ether = 0-30%) to give compound **015-8.** LCMS:(ESI)m/z: 868.2[M+H]⁺.

### Step 8: Synthesis of compound 015-9

To the reaction flask were added compound **015-8** (377 mg, 434.39 µmol, 1 *eq*), compound **001-2A** (207.47 mg, 1.30 mmol, 3 *eq*)*,* 4A molecular sieve (300 mg, 434.39 µmol, 1 *eq*) and dioxane (8 mL). Cesium carbonate (424.60 mg, 1.30 mmol, 3 *eq*) and (2-dicyclohexylphosphino-2',6'-dimethoxy-1,1'-biphenyl)[2-(2'-amino-1,1'-biphenyl)]palladium (II) chloride (31.30 mg, 43.44 µmol, 0.1 *eq*) were added. The atmosphere was replaced with nitrogen three times. The reaction was heated to 90 °C and stirred for 4 h. After the reaction was completed, the mixture was concentrated under reduced pressure. The crude product was purified by column chromatography (ethyl acetate:petroleum ether = 0-100%) to give compound **015-9.** LCMS:(ESI)m/z:899.4[M+H]⁺.

### Step 9: Synthesis of compound 015-10

To the reaction flask were added compound **015-9** (247 mg, 274.70 µmol, 1 *eq*) and acetonitrile (2.5 mL). Hydrochloric acid/dioxane (4 M, 2.06 mL, 30 *eq*) was added. The mixture was stirred at 20 °C for 1 hr. After the reaction was completed, the pH was adjusted to about 10 with ammonia. The mixture was extracted with dichloromethane (10 mL*3). The combined organic phase was washed with saturated brine (5 mL), dried over anhydrous sodium sulfate, and filtered. The filtrate was concentrated under reduced pressure to give the crude compound **015-10.** LCMS:(ESI)m/z:755.4[M+H]⁺.

### Step 10: Synthesis of compound 015 hydrochloride

To the reaction flask were added compound **015-10** (50 mg, 66.23 µmol, 1 *eq*)*,* and N,N-dimethylformamide (1 mL). Potassium carbonate (91.53 mg, 662.26 µmol, 10 *eq*) and cesium fluoride (50.30 mg, 331.13 µmol, 12.21 µL, 5 *eq*) were added. The mixture was heated to 65 °C for 3 hr. After the reaction was completed, the mixture was cooled to room temperature, and water (5 mL) was added. The mixture was extracted with ethyl acetate (5 mL*3). The combined organic phase was washed with saturated brine (5 mL), dried over anhydrous sodium sulfate, and filtered. The filtrate was concentrated under reduced pressure. The crude product was purified by high performance liquid chromatography (column: Phenomenex Luna 80*30mm*3µm; mobile phase: [water (hydrochloric acid)-acetonitrile]; acetonitrile %: 10%-35%, 8min) to give compound **015** hydrochloride. LCMS:(ESI)m/z: 599.3[M+H]⁺. ¹H NMR (400 MHz, CD₃OD) δ = 8.14-8.11 (m, 1H), 7.87-7.84 (m, 1H), 7.49-7.48 (m, 1H), 7.34-7.29 (m, 2H), 7.09-7.08 (m, 1H), 5.38-5.24 (d, *J* = 54 Hz, 1H), 4.38-4.29 (m, 3H), 4.13 (s, 2H), 3.25-3.20 (m, 2H), 3.14-3.13 (m, 1H), 3.06-3.00 (m, 1H), 2.51-2.43(m, 1H), 2.40-2.31 (m, 3H), 2.30-2.22 (m, 3H), 2.18-2.14(m, 1H), 2.12-2.05(m, 2H), 2.03-1.97 (m, 2H), 1.92 (s, 2H).

### Example 16

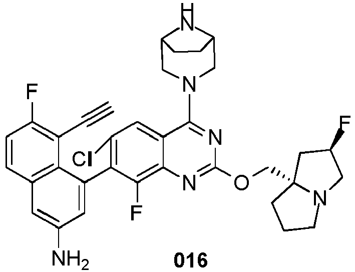

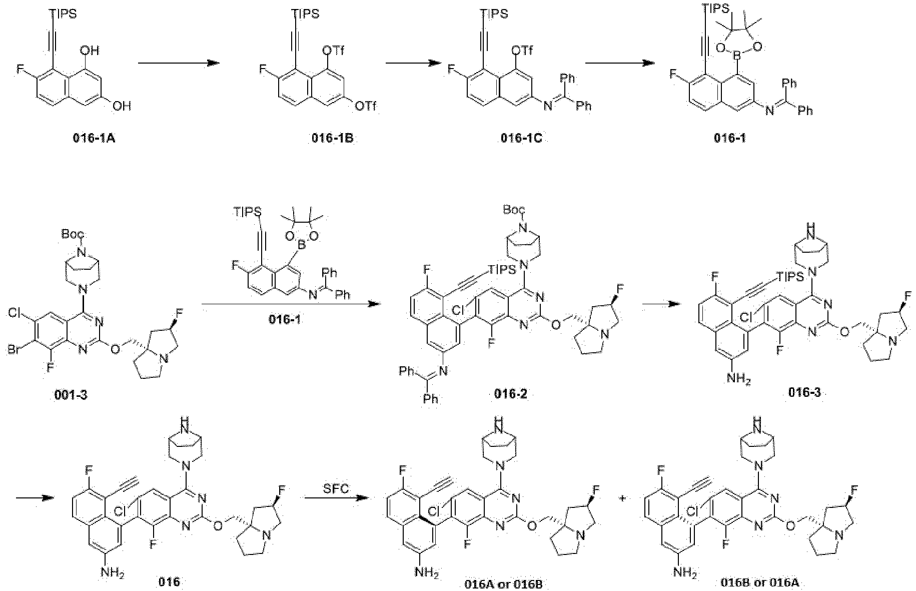

### Step 1: Synthesis of compound 016-1B

To a pre-dried reaction flask were added compound **016-1A** (2 g, 5.58 mmol, 1 eq), dichloromethane (40 mL), and N,N-diisopropylethylamine (4.33 g, 33.47 mmol, 5.83 mL, 6 eq). Trifluoromethanesulfonic anhydride (6.30 g, 22.31 mmol, 3.68 mL, 4 eq) was added at 0 °C. The mixture was stirred at 0°C for 1 hr. After the reaction was completed, water (5 mL) was added. The mixture was extracted with dichloromethane (5 mL × 4). The combined organic phase was washed with saturated brine (5 mL), dried over anhydrous sodium sulfate, and filtered. The filtrate was concentrated under reduced pressure, and the crude product was purified by silica gel column chromatography (gradient elution: petroleum ether : ethyl acetate = 10:1) to give compound **016-1B.**

### Step 2: Synthesis of compound 016-1C

To the reaction flask were added compound **016-1B** (3.2 g, 5.14 mmol, 1 eq), diphenylimine (1.86 g, 10.28 mmol, 1.72 mL, 2 eq), and anhydrous toluene (64 mL), followed by cesium carbonate (5.02 g, 15.42 mmol, 3 eq), and 4,5-bis(diphenylphosphino)-9,9-dimethylxanthene (594.75 mg, 1.03 mmol, 0.2 eq) under nitrogen. Tris(dibenzylideneacetone)dipalladium (470.62 mg, 513.94 umol, 0.1 q) was added and the mixture was stirred at 100 °C for 2 hr. After the reaction was completed, the reaction solution was cooled to room temperature and concentrated under reduced pressure. The crude product was purified by column chromatography (gradient elution: petroleum ether : ethyl acetate = 10:1) to give compound **016-1C.** LCMS:(ESI)m/z = 654.2 [M+H]⁺. ¹H NMR (400 MHz,CDCl₃) δ ppm 7.83-7.78 (m, 5 H), 7.60-7.49(m, 9 H), 1.20-1.16(m, 21 H).

### Step 3: Synthesis of compound 016-1

Compound **016-1C** (2.4 g, 3.67 mmol, 1 eq), bis(pinacolato)diboron (1.86 g, 7.34 mmol, 2 eq), and potassium acetate (1.08 g, 11.01 mmol, 3 eq) were dissolved in anhydrous toluene (48 mL) and 1,1'-bis(diphenylphosphino)ferrocene palladium dichloride (537.20 mg, 734.17 umol, 0.2 eq) was added. Under nitrogen, the mixture was stirred at 130 °C for 12 h. After the reaction was completed, the mixture was concentrated under reduced pressure and the crude product was purified by column chromatography (gradient elution: petroleum ether : ethyl acetate = 10:1) to give compound **016-1.** LCMS:(ESI)m/z = 632.3[M+H]⁺. ¹H NMR (400 MHz, DMSO) δ ppm 7.80 - 7.87 (m, 1 H), 7.68 - 7.74 (m, 2 H), 7.55 - 7.62 (m, 1 H), 7.47 - 7.53 (m, 2 H), 7.39 - 7.47 (m, 1 H), 7.27 - 7.35 (m, 4 H), 7.18 - 7.23 (m, 2 H), 7.10 - 7.15 (m, 1 H), 1.26 (s, 12 H), 1.07 - 1.15 (m, 21 H).

### Step 4: Synthesis of compound 016-2

Compound **001-3** (0.15 g, 238.50 µmol, 1 *eq*) and compound **016-1** (225.99 mg, 357.75 µmol, 1.5 *eq*) and potassium phosphate (151.88 mg, 715.50 µmol, 3 *eq*) were added to toluene (4 mL) and water (1 mL). The atmosphere was replaced with nitrogen three times. [(bis(1-adamantyl)-N-butylphosphine)-2-(2-aminobiphenyl)palladium(II) chloride (15.95 mg, 23.85 µmol, 0.1 *eq*) was added under nitrogen. The atmosphere was replaced with nitrogen three times. The mixture was reacted at 100 °C for 1.5 h. After the reaction was completed, the reaction solution was cooled down to room temperature and 20 mL of ethyl acetate and 10 mL of water were added. The mixture was left to stand, and the layers were separated. The aqueous phase was extracted once with 10 mL of ethyl acetate. The organic phases were combined, dried with anhydrous sodium sulfate and filtered. The filtrate was concentrated and purified by silica gel column chromatography (gradient elution: petroleum ether : ethyl acetate = 100:0-60:40, 5 parts per thousand of triethylamine was added to ethyl acetate) to give compound **016-2.** LCMS:(ESI)m/z = 1053.6[M+H]⁺.

¹H NMR (400 MHz, CD₃OD) δ ppm 7.83 (dd, J = 9.2, 5.6 Hz, 1H), 7.77-7.71 (m, 3H), 7.55-7.48 (m, 1H), 7.47-7.40 (m, 2H), 7.38-7.24 (m, 5H),7.17-7.12 (m, 2H), 6.81 (d, J = 2.0 Hz, 1 H), 5.30-5.23 (m, 1H), 4.73 (br t, J = 14.0 Hz, 1H), 4.44-4.33 (m, 2H), 4.30-4.22(m, 1H), 4.19-4.04 (m, 3H), 3.76 (s, 1H), 3.37-3.16 (m, 4H), 3.06-2.97 (m, 1H), 2.34-1.82 (m, 9H), 1.52 (s, 9H), 0.95-0.81 (m, 18 H), 0.61 - 0.43 (m, 3 H).

### Step 5: Synthesis of compound 016-3

Compound **016-2** (0.15 g, 142.35 µmol, 1 *eq*) was added to hydrogen chloride/methanol (4 M, 4 mL, 112.40 *eq*)*.* The mixture was reacted at 15 °C for 2 h. After the reaction was completed, the reaction solution was concentrated to dryness under reduced pressure, and then the residue was purified by slurring with 4 mL of ethyl acetate overnight, and filtered. The filter cake was collected and dried under vacuum to give compound **016-3.** LCMS:(ESI)m/z = 789.5[M+H]⁺.

### Step 6: Synthesis of compound 016

Compound **016-3** (0.1 g, 115.96 µmol, 1 eq) was added to N,N-dimethylformamide (1 mL), and potassium carbonate (256.42 mg, 1.86 mmol, 16 eq) and cesium fluoride (2.84 mg, 347.88 µmol, 3 eq) were added. The mixture was stirred at 65 °C for 3 h. After the reaction was completed, the mixture was cooled down to room temperature, then filtered. The filter cake was rinsed with 10 mL of ethyl acetate. Then the mother liquor was washed with saturated brine (20mL*3), dried over anhydrous sodium sulfate, and filtered. The filtrate was concentrated to give a crude product. The crude product was purified by preparative high performance liquid chromatography (HPLC) with the following method: column: Phenomenex Luna 80*30mm*3µm; mobile phase: [water (hydrochloric acid)-acetonitrile]; acetonitrile %: 1%-40%, 8 min. The obtained fraction was adjusted to a pH of about 8 by dropwise adding ammonia, and then concentrated under reduced pressure to remove acetonitrile. The residue was extracted twice with 20 mL of ethyl acetate, dried with anhydrous sodium sulfate, and filtered. The filtrate was concentrated to give the product **016.** The product was purified by SFC chromatographic column: DAICEL CHIRALPAK IC(250mm*30mm,10µm); mobile phase: [0.1% ammonia-isopropanol]; isopropanol %: 60%-60%, 16 min, and then concentrated under reduced pressure to remove the solvent to give compound **016A** and compound **016B.**

Compound **016A** was analyzed and characterized as follows:
SFC analysis method:

Column: Chiralpak IC-3, 3 µm, 50×4.6mm I.D; mobile phase: A (CO₂) and B (IPA with 0.1% isopropylamine); gradient: B% = 5-50% for 5 min; flow rate: 4.0 mL/min; wavelength: 220 nm; pressure: 124.14 bar, Rt = 1.13 min, chiral isomer excess 100%.

LCMS:(ESI)m/z = 633.3[M+H]⁺.

¹H NMR (400 MHz, CD₃OD ) δ ppm 7.81 (s, 1H), 7.73 (dd, J = 8.8, 5.6 Hz, 1H), 7.23 (t, J = 8.8 Hz, 1H), 7.17 (d, J = 2.2 Hz, 1H), 6.97 (d, J = 2.2 Hz, 1H), 5.24-5.42(m, 1H), 4.54 (d, J = 12.8 Hz, 1H), 4.43 (d, J = 12.8 Hz, 1H), 4.33 (d, J = 10.4 Hz, 1H), 4.23 (d, J = 10.8 Hz, 1H), 3.73-3.66 (m, 3H), 3.60 (d, J = 12.4 Hz, 1H), 3.35-3.23 (m, 3H), 3.21-3.19 (m, 1H), 3.10-3.02 (m, 1H), 2.44-2.13 (m, 3H), 2.06-1.98 (m, 2H), 1.94-1.81 (m, 5 H).

Compound **016B** was analyzed and characterized as follows:
SFC analysis method:

Column: Chiralpak IC-3, 3 µm, 50×4.6mm I.D; mobile phase: A (CO₂) and B (IPA with 0.1% isopropylamine); gradient: B% = 5-50% for 5 min; flow rate: 4.0 mL/min; wavelength: 220 nm; pressure: 124.14 bar, Rt = 2.20 min, chiral isomer excess 97.76%.

LCMS:(ESI)m/z = 633.3[M+H]⁺.

¹H NMR (400 MHz, CD₃OD ) δ ppm 7.80 (s, 1H), 7.73 (dd, J = 8.8, 6.0 Hz, 1H), 7.23 (t, J = 9.0 Hz, 1H), 7.17 (d, J = 2.2 Hz, 1H), 6.97 (d, J = 2.4 Hz, 1H), 5.39-5.25 (m, 1H), 4.54-4.40 (m, 2H), 4.26 (dd,J = 23.2 Hz, 10.4 Hz, 2H), 3.70-3.59 (m, 4H), 3.29-3.19 (m, 4H), 3.08-3.01 (m, 1H), 2.40-2.13 (m, 3H), 2.06 - 1.96 (m, 2 H), 1.92-1.82 (m, 5 H).

### Example 17

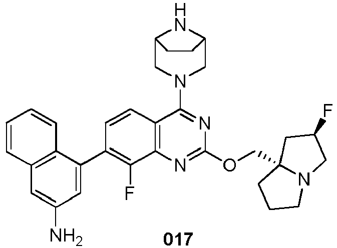

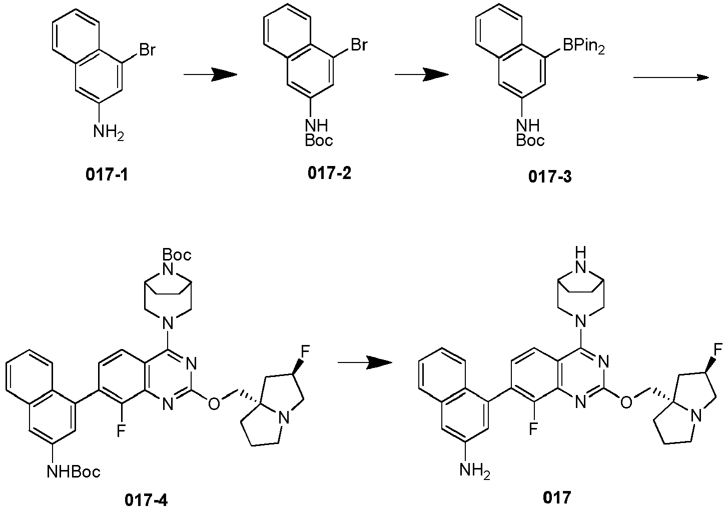

### Step 1: Synthesis of compound 017-2

Compound **017-1** (0.27 g, 1.22 mmol, 1 eq) was dissolved in THF (10 mL), and di-tert-butyl dicarbonate (318.41 mg, 1.46 mmol, 335.16 µL, 1.2 eq) was slowly added. The mixture was reacted at 80 °C for 12 h. After the reaction was completed, the mixture was directly rotary-evaporated to dryness and the crude product was purified on silica gel column (gradient elution: petroleum ether:ethyl acetate = 10:1 -5:1) to give compound **017-2.**

### Step 2: Synthesis of compound 017-3

Compound **017-2** (0.35 g, 1.09 mmol, 1 eq), bis(pinacolato)diboron (413.78 mg, 1.63 mmol, 1.5 eq), 1,1'-bis(diphenylphosphino)ferrocene palladium(II) dichloride (79.48 mg, 108.63 µmol, 0.1 eq), and potassium acetate (159.92 mg, 1.63 mmol, 1.5 eq) were dissolved in 1,4-dioxane (10 mL) under nitrogen. The mixture was reacted at 80 °C for 16 h. After the reaction was completed, the mixture was directly rotary-evaporated to dryness. The crude product was purified on silica gel column (gradient elution: petroleum ether : ethyl acetate = 50:1 -20:1) to give compound **017-3.**

### Step 3: Synthesis of compound 017-4

Compound **017-3** (100 mg, 270.81 µmol, 1 eq), 1,1'-bis(diphenylphosphino)ferrocene palladium(II) dichloride (19.82 mg, 27.08 µmol, 0.1 eq), potassium carbonate (74.86 mg, 541.62 µmol, 2 eq), and compound 005-5 (160.99 mg. 270.81 µmol, 1 eq) were dissolved in 1,4-dioxane (2.5 mL) and water (0.5 mL) under nitrogen. The mixture was reacted at 95 °C for 16 h. After the reaction was completed, the mixture was directly rotary-evaporated to dryness and the crude product was purified on silica gel column (gradient elution: petroleum ether : ethyl acetate = 50:1 -20:1) to give compound **017-4.**

### Step 4: Synthesis of compound 017

Compound **017-4** (49.93 mg, 65.97 µmol, 1 eq) was dissolved in 1,4-dioxane (2 mL), and hydrochloric acid (48.11 mg, 1.32 mmol, 47.17 µL, 20 eq) was added. The mixture was reacted at 25 °C for 4hr. After the reaction was completed, the mixture was directly rotary-evaporated to dryness to give compound **017** hydrochloride, LCMS:(ESI)m/z = 557.3[M+H]⁺.

¹H NMR (400 MHz, CD₃OD ) δ = 8.11 (br d, J = 7.3 Hz, 1H), 8.05 - 8.00 (m, 1H), 7.68 - 7.49 (m, 5H), 5.58 - 5.46 (m, 1H), 4.43 - 3.71 (m, 7H), 3.62 - 3.56 (m, 2H), 3.44 - 3.32 (m, 1H), 2.78 - 2.50 (m, 2H), 2.48 - 2.38 (m, 1H), 2.34 - 1.96 (m, 7H).

### Example 18

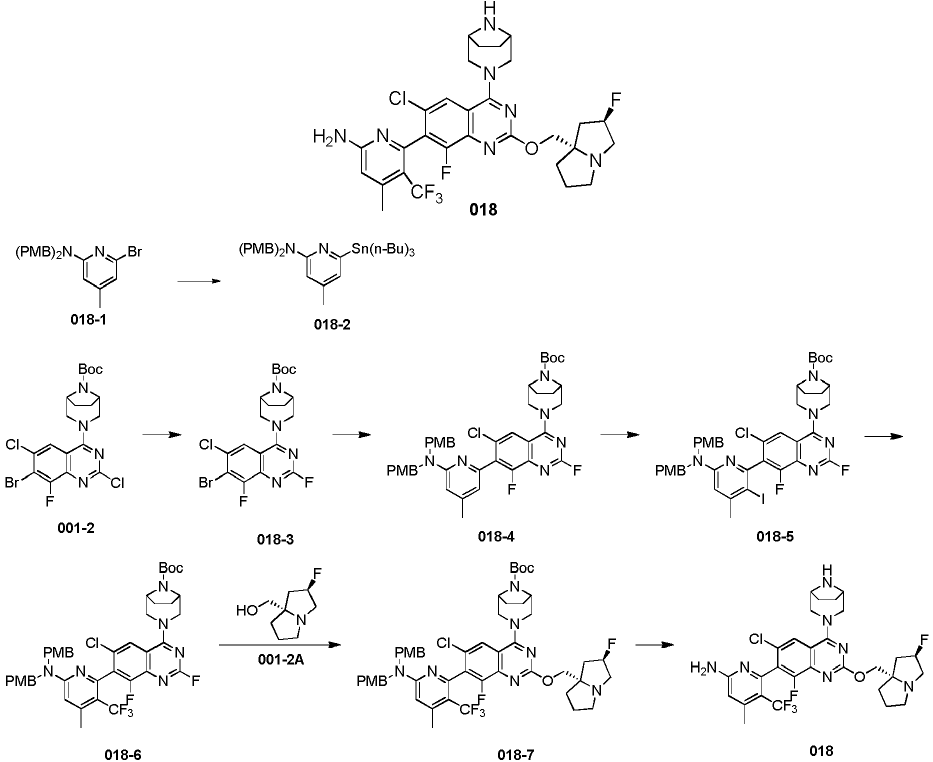

### Step 1: Synthesis of compound 018-2

Under nitrogen, compound **018-1** (5 g, 11.70 mmol, 1 eq), hexa-n-butyltin (20.36 g, 35.10 mmol, 17.55 mL, 3 eq), tris(dibenzylideneindenylacetone)dipalladium (1.07 g, 1.17 mmol, 0.1 eq), tricyclohexylphosphine (656.23 mg, 2.34 mmol, 758.64 µL, 0.2 eq), lithium chloride (2.48 g, 58.50 mmol, 1.20 mL, 5 eq) and 1,4-dioxane (50 mL) were stirred at 110 °C for 2 h. After the reaction was completed, the mixture was rotary-evaporated to dryness and the crude product was purified on silica gel column (gradient elution: petroleum ether : ethyl acetate = 100:1) to give compound **018-2.** LCMS:(ESI)m/z = 639.3[M+H]⁺.

### Step 2: Synthesis of compound 018-3

Compound **001-2** (2.5 g, 4.94 mmol, 1 eq) was dissolved in N,N-dimethylacetamide (30 mL) and potassium fluoride (5.74 g, 98.78 mmol, 2.31 mL, 20 eq) was added. The mixture was reacted at 120 °C for 18 h. After the reaction was completed, water was added and the mixture was extracted with 100 mL of ethyl acetate. The organic phase was dried with anhydrous sodium sulfate, and rotary-evaporated to dryness. The crude product was purified on silica gel column (gradient elution: petroleum ether : ethyl acetate = 20:1 -10:1) to give compound **018-3.** LCMS:(ESI)m/z = 489.1[M+H]⁺.

### Step 3: Synthesis of compound 018-4

Compound **018-3** (384.12 mg, 784.34 µmol, 1 eq), compound **018-2** (0.5 g, 784.34 µmol, 1 eq), tetrakis(triphenylphosphine)palladium (90.63 mg, 78.43 µmol, 0.1 eq), cuprous iodide (22.41 mg, 117.65 µmol, 0.15 eq) and lithium chloride (39.90 mg, 941.20 µmol, 19.28 µL, 1.2 eq) were dissolved in 1,4-dioxane (10 mL). The mixture was reacted at 120 °C for 18 h. After the reaction was completed, the mixture was directly rotary-evaporated to dryness. The crude product was purified on silica gel column (gradient elution: petroleum ether : ethyl acetate = 20:1 -2:1) to give compound **018-4,** LCMS:(ESI)m/z = 757.3[M+H]⁺.

### Step 4: Synthesis of compound 018-5

Compound **018-4** (0.1 g, 132.05 µmol, 1 eq) was dissolved in N,N-dimethylformamide (5 mL) and N-iodosuccinimide (44.57 mg, 198.08 µmol, 1.5 eq) was added. The mixture was reacted at 25 °C for 1 h. After the reaction was completed, water was added. The mixture was extracted with 100 mL of ethyl acetate. The organic phase was dried with anhydrous sodium sulfate and rotary-evaporated to dryness. The crude product was purified on silica gel column (gradient elution: petroleum ether : ethyl acetate = 20:1 -5:1) to give compound **018-5.** LCMS:(ESI)m/z = 883.2[M+H]⁺.

### Step 5: Synthesis of compound 018-6

Compound **018-5** (0.1 g, 113.23 µmol, 1 eq) was dissolved in N,N-dimethylformamide (2 mL), then cuprous iodide (64.69 mg, 339.69 µmol, 3 eq), and hexamethylphosphoramide (202.91 mg, 1.13 mmol, 198.93 µL, 10 eq) were added. Under nitrogen, methyl fluorosulfonyldifluoroacetate (217.53 mg, 1.13 mmol, 144.06 µL, 10 eq) was then added and the mixture was reacted at 90 °C for 2 h. After the reaction was completed, water was added. The mixture was extracted with 100 mL of ethyl acetate. The organic phase was dried with anhydrous sodium sulfate and rotary-evaporated to dryness. The crude product was purified on silica gel column (gradient elution: petroleum ether : ethyl acetate = 20:1 -5:1) to give compound **018-6.**

### Step 6: Synthesis of compound 018-7

**001-2A** (28.94 mg, 181.76 µmol, 3 eq) was dissolved in THF (2 mL) and sodium hydride (14.54 mg, 363.52 µmol, 60% content, 6 eq) was added at 0 °C. After the addition was completed, the mixture was heated to 25 °C and stirred for 1 h. Then **018-6** (0.05 g, 60.59 µmol, 1 eq) was added and the mixture was stirred for 1 h. After the reaction was completed, methanol (0.5 mL) was added to quench the reaction. The mixture was concentrated to dryness to remove the solvent, and the crude product was purified on silica gel column (gradient elution: petroleum ether : ethyl acetate = 50:1 -20:1) to give compound **018-7.**

### Step 7: Synthesis of compound 018-8

Compound **018-7** (0.05 g, 51.84 µmol, 1 eq) was dissolved in trifluoroacetic acid (2 mL) and the mixture was stirred at 50 °C for 5 h. The mixture was rotary-evaporated to dryness and purified by preparative HPLC column: Welch Xtimate C18 100*40 mm*3 µm; mobile phase: [water (trifluoroacetic acid)-acetonitrile]; acetonitrile %: 7%-37%, 8 min, to give compound **018** trifluoroacetate, LCMS:(ESI)m/z = 624.3[M+H]⁺. ¹H NMR (400 MHz, CD₃OD ) δ ppm 2.01 - 2.27 (m, 5 H) 2.30 - 2.41 (m, 2 H) 2.45 (br d, J = 8.78 Hz, 1 H) 2.52 (br s, 3H) 2.55 - 2.85 (m, 2 H) 3.41 - 3.58 (m, 1 H) 3.80 - 4.12 (m, 5 H) 4.26 (br s, 2 H) 4.60 - 4.80 (m, 4 H) 5.53 (br s, 1 H) 5.67 (br d, J = 3.51 Hz, 1 H) 6.64 - 6.89 (m, 1 H) 6.80 (s, 1 H) 7.97 (s, 1 H).

### Example 19

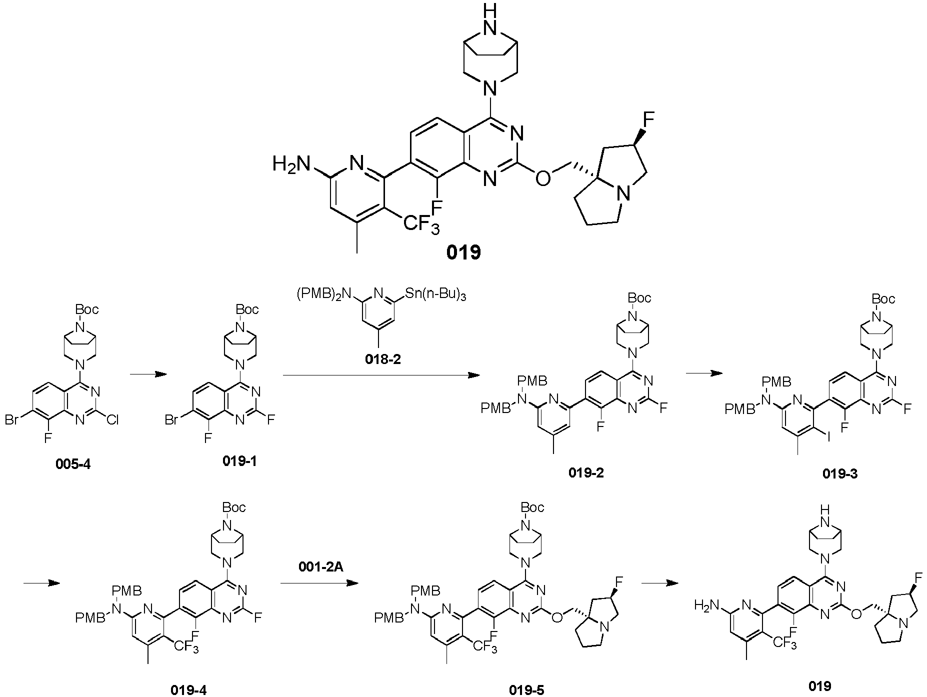

### Step 1: Synthesis of compound 019-1

Compound **005-4** (2 g, 4.24 mmol, 1 eq) was dissolved in N,N-dimethylacetamide (30 mL) and potassium fluoride (1.23 g, 21.20 mmol, 496.58 µL, 5 eq) was added. The mixture was reacted at 120 °C for 24 h. After the reaction was completed, water was added and the mixture was extracted with 100 mL of ethyl acetate. The organic phase was dried with anhydrous sodium sulfate and rotary-evaporated to dryness. The crude product was purified on silica gel column (gradient elution: petroleum ether : ethyl acetate = 20:1 -10:1) to give compound **019-1,** LCMS:(ESI)m/z = 455.0[M+H]⁺.

### Step 2: Synthesis of compound 019-2

Compound **019-1** (220 mg, 483.20 µmol, 1 eq), compound **018-2** (308.03 mg, 483.20 µmol, 1 eq), tetrakis(triphenylphosphine)palladium (55.84 mg, 48.32 µmol, 0.1 eq), cuprous iodide (13.80 mg, 72.48 µmol, 0.15 eq) and lithium chloride (20.48 mg, 483.20 µmol, 9.90 µL, 1 eq) were dissolved in 1,4-dioxane (10 mL). The mixture was reacted at 120 °C for 18 h. After the reaction was completed, the mixture was directly rotary-evaporated to dryness. The crude product was purified on silica gel column (gradient elution: petroleum ether : ethyl acetate = 20:1 -2:1) to give compound **019-2,** LCMS:(ESI)m/z = 723.3[M+H]⁺.

### Step 3: Synthesis of compound 019-3

Compound **019-2** (200 mg, 276.69 µmol, 1 eq) was dissolved in N,N-dimethylformamide (5 mL), and then N-iodosuccinimide (186.75 mg, 830.08 µmol, 3 eq) was added. The mixture was reacted at 25°C for 5 h. After the reaction was completed, water was added and the mixture was extracted with 100 mL of ethyl acetate. The organic phase was dried with anhydrous sodium sulfate and filtered. The filtrate was concentrated and the crude product was purified on silica gel column (gradient elution: petroleum ether : ethyl acetate = 20:1 -5:1) to give compound **019-3.** LCMS:(ESI)m/z = 849.2[M+H]⁺.

### Step 4: Synthesis of compound 019-4

Compound **019-3** (120 mg, 141.39 µmol, 1 eq) was dissolved in N,N-dimethylformamide (2 mL), and cuprous iodide (53.86 mg, 282.78 µmol, 2 eq), and hexamethylphosphoramide (126.69 mg, 706.95 µmol, 124.20 µL, 5 eq). Under nitrogen, methyl fluorosulfonyldifluoroacetate (135.81 mg, 706.95 µmol, 89.94 µL, 5 eq) was then added and mixture was reacted at 80 °C for 18 h. After the reaction was completed, water was added. The mixture was extracted with 100 mL of ethyl acetate. The organic phase was dried over anhydrous sodium sulfate, and filtered. The filtrate was concentrated to give a crude product. The crude product was purified on silica gel column (gradient elution: petroleum ether : ethyl acetate = 20:1 -5:1) to give compound **019-4.** LCMS:(ESI)m/z = 791.3[M+H]⁺.

### Step 5: Synthesis of compound 019-5

Compound **019-4** (200 mg, 252.90 µmol, 1 eq) was dissolved in tetrahydrofuran (2 mL) and N,N-dimethylformamide (2 mL), and then cesium carbonate (164.80 mg, 505.80 µmol, 2 eq) and triethylenediamine (14.18 mg, 126.45 µmol, 13.91 µL, 0.5 eq) were added. Finally, **001-2A** (80.52 mg, 505.80 µmol, 2 eq) was added and the mixture was reacted at 25°C for 28 h. After the reaction was completed, water was added and the mixture was extracted with 100 mL of ethyl acetate. The organic phase was dried over anhydrous sodium sulfate, and filtered. The filtrate was concentrated to give a crude product. The crude product was purified on silica gel column (gradient elution: petroleum ether : ethyl acetate = 50:1 -20:1) to give compound **019-5.** LCMS:(ESI)m/z = 930.7[M+H]⁺.

### Step 6: Synthesis of compound 019

Compound **019-5** (100 mg, 107.53 µmol, 1 eq) was dissolved in trifluoroacetic acid (1.54 g, 13.51 mmol, 1 mL, 125.61 eq). The reaction solution was reacted at 55°C for 4hr, and separated by preparative HPLC (column: Phenomenex C18 150*40mm*5µm; mobile phase: [water (hydrochloric acid)-acetonitrile]; acetonitrile %:5%-35%, 10 min) to give compound **019** hydrochloride. LCMS:(ESI)m/z = 590.5[M+H]⁺. ¹H NMR (400 MHz, CD₃OD ) δ = 8.09 (d, J = 8.8 Hz, 1H), 7.57 (t, J = 7.5 Hz, 1H), 7.05 (s, 1H), 5.69 - 5.56 (m, 1H), 4.86 - 4.75 (m, 4H), 4.31 (br s, 2H), 4.16 - 3.84 (m, 5H), 3.55 - 3.44 (m, 1H), 2.86 - 2.56 (m, 5H), 2.51 (br s, 1H), 2.43 - 2.32 (m, 2H), 2.31 - 2.13 (m, 5H).

### Example 20

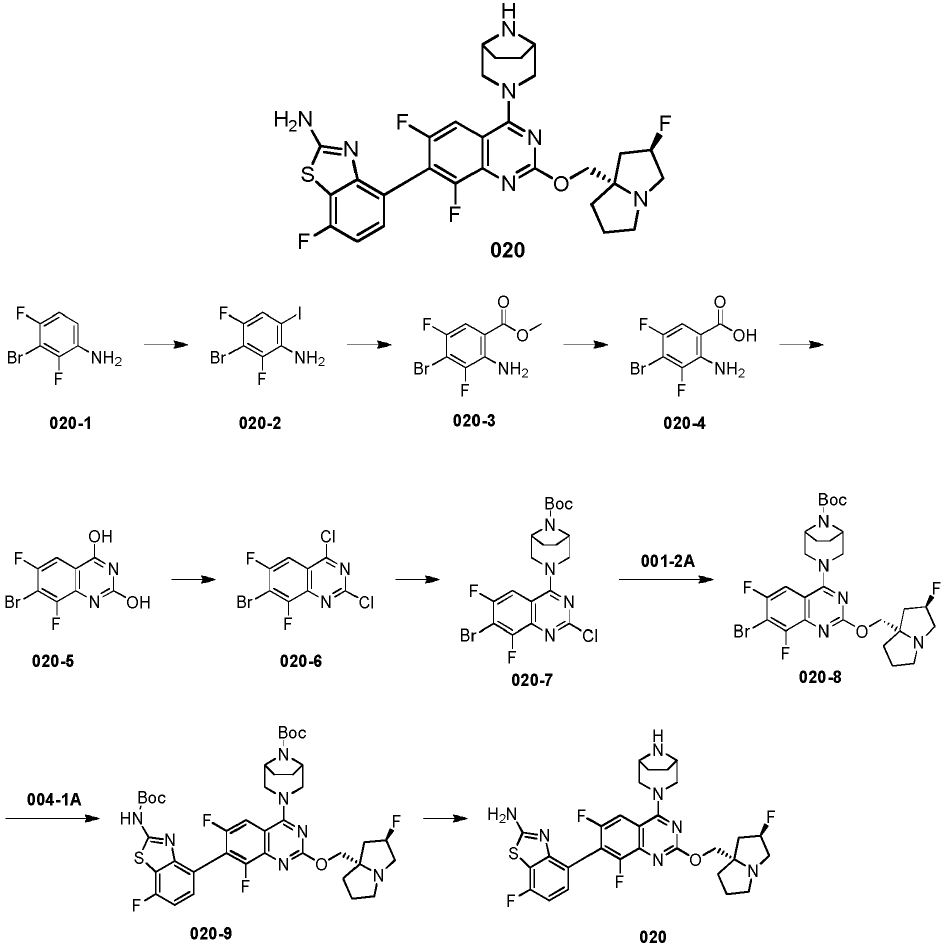

### Step 1: Synthesis of compound 020-2

Compound **020-1** (30 g, 144.23 mmol, 1 eq) and silver sulfate (44.97 g, 144.23 mmol, 24.44 mL, 1 *eq*) were dissolved in ethanol (300 mL), and then iodine (40.27 g, 158.65 mmol, 31.96 mL, 1.1 *eq*) was added. The mixture was stirred at 25 °C for 2 h. After the reaction was completed, the reaction solution was filtered, and the filtrate was evaporated to dryness. The crude product was purified on silica gel column (gradient elution: petroleum ether : ethyl acetate = 100:1 -20:1) to give compound **020-2.** ¹H NMR (400MHz, CDCl₃) δ: 5.08 (d, *J* = 7.8 Hz, 1H), 4.30 - 4.24 (m, 1H), 1.63 - 1.53 (m, 2H), 1.32 (s, 9H), 0.70 - 0.58 (m, 1H), 0.42 - 0.32 (m, 2H), 0.05 - -0.05 (m, 2H).

### Step 2: Synthesis of compound 020-3

Compound **020-2** (22 g, 65.89 mmol, 1 eq) and 1,1'-bis(diphenylphosphino)ferrocene]palladium dichloride dichloromethane (5.38 g, 6.59 mmol, 0.1 *eq*) were dissolved in methanol (100 mL) under a carbon monoxide environment at a pressure of 50 Psi and a temperature of 30 °C. The mixture was stirred for 5 min, and then triethylamine ( 46.67 g, 461.22 mmol, 64.20 mL, 7 *eq*) was added. The mixture was stirried for another 24 h. After the reaction was completed, the mixture was filtered and the filtrate was evaporated. The crude product was purified on silica gel column (gradient elution: petroleum ether : ethyl acetate = 20:1 -5:1) to give compound **020-3.** LCMS:(ESI)m/z = 265.8 [M+H]⁺.

### Step 3: Synthesis of compound 020-4

Compound **020-3** (15 g, 56.38 mmol, 1 eq) was dissolved in methanol (50 mL) and a solution of sodium hydroxide (9.02 g, 225.53 mmol, 4 eq) in water (50 mL) was added. The mixture was stirred at 25 °C for 2 h. After the reaction was completed, the reaction solution was concentrated and adjusted to pH = 5 using 2 mol/L hydrochloric acid. A white solid was precipitated and filtered by suction to give compound **020-4.** LCMS:(ESI)m/z = 251.8 [M+H]⁺.

### Step 4: Synthesis of compound 020-5

Compound **020-4** (12 g, 47.62 mmol, 1 *eq*) and urea (85.79 g, 1.43 mol, 76.60 mL, 30 *eq*) were added to a reaction flask. The mixture was reacted at 200 °C for 4 h. The reaction solution was cooled to room temperature and slurried using 200 mL of water. The mixture was filtered by suction to give compound **020-5.** LCMS:(ESI)m/z = 276.9 [M+H]⁺.

### Step 5: Synthesis of compound 020-6

N,N-diisopropylethylamine (11.66 g, 90.25 mmol, 15.72 mL, 5 eq) was added dropwise to phosphorus oxychloride (82.50 g, 538.05 mmol, 50 mL, 29.81 eq) at 0 °C, and then compound **020-5** (5 g, 18.05 mmol, 1 eq) was added in batches. The mixture was refluxed at 80 °C for 20 h. The phosphorus oxychloride was evaporated. The crude product was purified on silica gel column (gradient elution: petroleum ether : ethyl acetate = 20:1 -10:1) to give compound **020-6.** LCMS:(ESI)m/z = 312.9 [M+H]⁺.

### Step 6: Synthesis of compound 020-7

Compound **001-1A** (1.2 g, 3.82 mmol, 1 *eq*) was dissolved in N,N-dimethylformamide (2 mL), and compound **020-6** (811.51 mg, 3.82 mmol, 1 *eq*) and N,N-diisopropylethylamine (1.48 g, 11.47 mmol, 2.00 mL, 3 *eq*) were added. The mixture was stirred to react at 25°C for 2h. The reaction solution was poured into 50 mL of water, and a solid was precipitated. The solid was washed with water (3*20 mL) to give compound **020-7.** LCMS:(ESI)m/z = 489.0[M+H]⁺.

### Step 7: Synthesis of compound 020-8

Compound **020-7** (1.6 g, 3.27 mmol, 1 eq) was dissolved in N,N-dimethylformamide (10 mL) and THF (10 mL), and then cesium carbonate (3.19 g, 9.80 mmol, 3 eq), compound **001-2A** (780.17 mg, 4.90 mmol, 1.5 eq), and triethylenediamine ( 36.65 mg, 326.70 µmol, 35.93 µL, 0.1 eq) were added. The mixture was stirred to react at 25 °C for 18 h. After the reaction was completed, the mixture was extracted with 40 mL of ethyl acetate, and washed with water and saturated brine. The organic phase was dried with anhydrous sodium sulfate, and filtered. The filtrate was concentrated to give a crude product. The crude product was purified on silica gel column (gradient elution: petroleum ether : ethyl acetate = 20:1 -10:1) to give compound **020-8.** LCMS:(ESI)m/z = 612.2 [M+H]⁺.

### Step 8: Synthesis of compound 020-9

Compound **020-8** (0.2 g, 408.38 µmol, 1 *eq*) and compound **004-1A** (127.47 mg, 408.38 µmol, 1 *eq*) were dissolved in 1,4-dioxane (2 mL) and water (2 mL), and 1,1'-bis(di-tert-butylphosphino)ferrocene palladium dichloride (26.62 mg, 40.84 µ mol, 0.1 *eq*)*,* and potassium phosphate (130.03 mg, 612.57 µmol, 1.5 *eq*) were added. The mixture was reacted under nitrogen at 90 °C for 18 h. After the reaction was completed, the reaction solution was rotary-evaporated to dryness and the crude product was purified on silica gel column (gradient elution: petroleum ether : ethyl acetate = 20:1 -10:1) to give compound **020-9.** LCMS:(ESI)m/z = 800.3 [M+H]⁺.

### Step 9: Synthesis of compound 020

Compound **020-9** (25 mg, 31.25 µmol, 1 *eq*) was dissolved in acetonitrile (1 mL) and hydrochloric acid/1,4-dioxane (1 mL). The mixture was reacted at 25°C for 12 h. After the reaction was completed, the mixture was rotary-evaporated to dryness to remove the solvent and separated by preparative column: Phenomenex C18 150*40mm*5µm; mobile phase: [water (formic acid)-acetonitrile]; acetonitrile %. 1%-30%, 10 min, to give compound **020** formate. LCMS:(ESI)m/z = 600.1 [M+H]⁺.

### Example 21

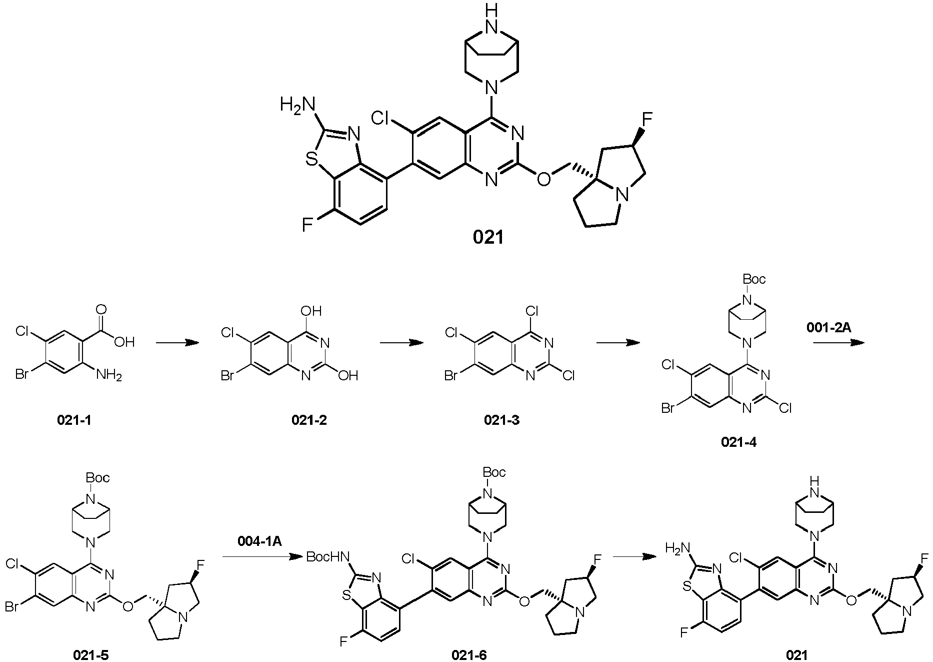

### Step 1: Synthesis of compound 021-2

Compound **021-1** (15 g, 59.89 mmol, 1 *eq)* was added to a reaction vial and then urea (60 g, 999.08 mmol, 53.57 mL, 16.68 *eq*) was added. The mixture was heated to 200 °C to reflux for 4 h. The mixture was cooled to room temperature and slurried with hot water to give compound **021-2.** LCMS:(ESI)m/z = 274.9[M+H]⁺.

### Step 2: Synthesis of compound 021-3

N,N-diisopropylethylamine (11.73 g, 90.75 mmol, 15.81 mL, 5 eq) was added to phosphorus oxychloride (82.50 g, 538.05 mmol, 50 mL, 29.64 eq) at 0 °C. **021-2** (5 g, 18.15 mmol, 1 eq) was added in batches, and then the mixture was reacted at 100 °C. After the reaction was completed, the reaction solution was evaporated to dryness, and phosphorus trichloride was removed. The crude product was purified on silica gel column (gradient elution: petroleum ether : ethyl acetate = 20:1 -10:1 ) to give compound **021-3.** ¹H NMR (400 MHz, DMSO-*d₆*) δ ppm 8.50 (s, 1 H) 8.58 (s, 1 H).

### Step 3: Synthesis of compound 021-4

**021-3** (3 g, 9.60 mmol, 1 *eq*) and **001-1A** (2.04 g, 9.60 mmol, 1 *eq*) were dissolved in N,N-dimethylformamide (10 mL), and N,N-diisopropylethylamine (3.72 g, 28.81 mmol, 5.02 mL, 3 *eq*) was added. The reaction solution was stirred at 25 °C for 4 h. The reaction solution was poured into 200 mL of water and filtered to give a solid. The solid was washed three times with 20 mL of water to give compound **021-4.** LCMS:(ESI)m/z = 487.0 [M+H]⁺.

### Step 4: Synthesis of compound 021-5

**021-4** (1.5 g, 3.07 mmol, 1 *eq*) was dissolved in N,N-dimethylformamide (10 mL) and THF (10 mL), and then cesium carbonate (3.00 g, 9.22 mmol, 3 *eq*)*,* **001-2A** (733.71 mg, 4.61 mmol, 1.5 *eq*)*,* and triethylenediamine (34.46 mg. 307.25 µmol, 33.79 µL, 0.1 *eq*) were added. The mixture was stirred at 25 °C for 18 h. After the reaction was completed, the mixture was extracted with 50 mL of ethyl acetate, washed three times with water, and washed with saturated brine. The organic phases were combined, dried over anhydrous sodium sulfate, and filtered. The filtrate was concentrated, and the crude product was purified on silica gel column (gradient elution: petroleum ether : ethyl acetate = 100:1 -20:1) to give compound **021-5.** LCMS:(ESI)m/z = 610.2 [M+H]⁺.

### Step 5: Synthesis of compound 021-6

**021-5** (0.4 g, 654.72 µmol, 1 *eq*) and **004-1A** (204.36 mg, 654.72 µmol, 1 eq) were dissolved in 1,4-dioxane (6 mL) and water (6 mL), and 1,1'-bis(di-tert-butylphosphino)ferrocene palladium dichloride (42.67 mg, 65.47 µmol, 0.1 *eq*)*,* and potassium phosphate (208.47 mg, 982.09 µmol, 1.5 *eq*) were added. The mixture was reacted at 90 °C under nitrogen for 18 h. After the reaction was completed, the reaction solution was rotary-evaporated to dryness and the crude product was purified on silica gel column (gradient elution: dichloromethane : methanol = 100:1 -20:1) to give compound **021-6.** LCMS:(ESI)m/z = 798.3 [M+H]⁺.

### Step 6: Synthesis of compound 021

**021-6** (0.1 g, 125.26 µmol, 1 *eq*) was dissolved in acetonitrile (2 mL), and hydrogen chloride/1,4-dioxane (2 mL) was added. The mixture was reacted at 25°C for 15 h. After the reaction was completed, the reaction solution was evaporated to dryness and separated by preparative HPLC: column: Phenomenex C18 150*40mm*5µm; mobile phase: [water (formic acid) - acetonitrile acetonitrile]; acetonitrile %: 7%-37%, 10 min, to give compound **021** formate. ¹H NMR (400 MHz, DMSO-*d₆*) δ ppm 1.67 - 1.95 (m, 8 H) 1.96 - 2.06 (m, 2 H) 2.10 - 2.19 (m, 1 H) 2.79 - 2.87 (m, 1 H) 2.99 - 3.15 (m, 3 H) 3.63 (br d, J = 13.30 Hz, 1 H) 3.87 (br s, 2 H) 3.96 - 4.12 (m, 2 H) 4.32 (br d, J = 12.80 Hz, 2 H) 5.17 - 5.38 (m, 1 H) 7.03 (t, J = 8.78 Hz, 1 H) 7.22 (dd, J = 8.28, 5.77 Hz, 1 H) 7.54 (s, 1 H) 7.87 (s, 2 H) 7.98 (s, 1 H) 8.22 (s, 1 H).

### Example 22

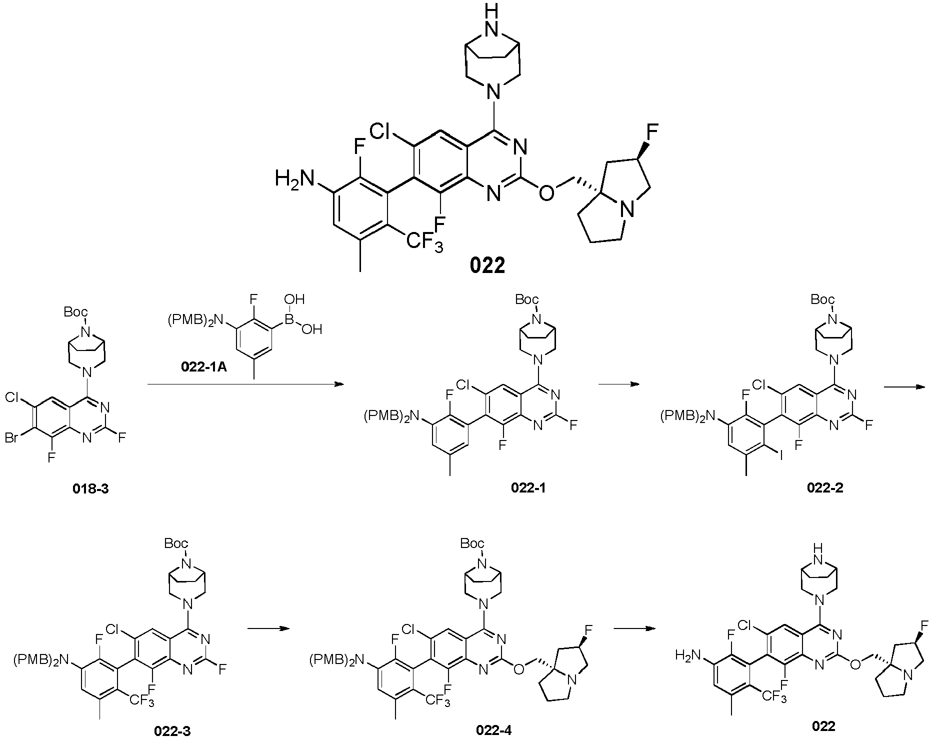

### Step 1: Synthesis of compound 022-1

**018-3** (1.8 g, 3.68 mmol, 1 *eq*) and **022-1A** (1.50 g, 3.68 mmol, 1 *eq*) were dissolved in 1,4-dioxane (2 mL) and water (2 mL), and 1,1'-bis(di-tert-butylphosphino)ferrocene palladium dichloride (479.09 mg, 735.08 µmol, 0.2 *eq*) and potassium phosphate (1.17 g, 5.51 mmol, 1.5 *eq*) were added. The mixture was reacted at 90 °C under nitrogen for 18 h. After the reaction was completed, the mixture was extracted twice with 30 mL of ethyl acetate. The organic phases were combined, dried over anhydrous sodium sulfate, and filtered. The filtrate was concentrated and the crude product was purified by silica gel column (gradient elution: dichloromethane : methanol = 100:1 -20:1) to give compound **022-1.** LCMS:(ESI)m/z = 774.3[M+H]⁺.

### Step 2: Synthesis of compound 022-2

**022-1** (1.3 g, 1.68 mmol, 1 eq) was dissolved in N,N-dimethylformamide (2 mL) and N-iodosuccinimide (1.13 g, 5.04 mmol, 3 eq) was added. The mixture was reacted at 25°C for 2 h. After the reaction was completed, the mixture was extracted twice with 30 mL of ethyl acetate. The organic phases were combined, dried over anhydrous sodium sulfate, and filtered. The filtrate was concentrated and the crude product was purified on silica gel column (gradient elution: petroleum ether : ethyl acetate = 100:1 -10:1) to give compound **022-2.** LCMS:(ESI)m/z = 900.2[M+H]⁺.

### Step 3: Synthesis of compound 022-3

**022-2** (100 mg, 111.09 µmol, 1 *eq*) was dissolved in N-methylpyrrolidone (2 mL), and then methyl fluorosulfonyldifluoroacetate (3.02 g, 15.72 mmol, 2 mL, 141.50 *eq*)*,* and cuprous iodide (105.79 mg, 555.45 µmol, 5 *eq*) were added. Under nitrogen, hexamethylphosphoramide (99.54 mg, 555.45 µmol, 97.59 µL, 5 *eq*) was added. The mixture was refluxed at 80°C for 18 hours. After the reaction was completed, the mixture was extracted twice with 20 mL of ethyl acetate. The organic phases were combined, dried over anhydrous sodium sulfate, and filtered. The filtrate was concentrated and the crude product was purified on silica gel column (gradient elution: petroleum ether : ethyl acetate = 100:1 -10:1) to give the compound **022-3.** LCMS:(ESI)m/z = 842.3[M+H]⁺.

### Step 4: Synthesis of compound 022-4

**022-3** (70 mg, 83.11 µmol, 1 *eq*) was dissolved in tetrahydrofuran (1 mL) and N,N-dimethylformamide (1 mL), and then cesium carbonate (27.08 mg, 83.11 µmol, 1 *eq*)*,* **001-2A** (15.88 mg, 99.73 µmol, 1.2 *eq*)*,* and triethylenediamine (932.23 µ g, 8.31 µmol, 9.14e-1 µL, 0.1 *eq*) were added. The mixture was reacted at 25 °C for 18 h. After the reaction was completed, water was added and the mixture was extracted with 100 mL of ethyl acetate. The organic phase was dried over anhydrous sodium sulfate, and filtered. The filtrate was concentrated, and the crude product was purified on silica gel column (gradient elution: dichloromethane : methanol = 100:1 -20:1) to give compound **022-4.** LCMS:(ESI)m/z = 981.4[M+H]⁺.

### Step 5: Synthesis of compound 022

**022-4** (50 mg, 50.94 µmol, 1 eq) was dissolved in trifluoroacetic acid (5.81 mg, 50.94 µmol, 3.77 µL, 1 eq) and the mixture was stirred at 25°C for 2 h. After the reaction was completed, the mixture was rotary-evaporated to dryness to remove the solvent and separated by preparative column: Phenomenex C18 150*40 mm*5 µm; mobile phase: [water (formic acid)-acetonitrile]; % acetonitrile: 5%-35%, 10 min, to give compound 022 formate. LCMS:(ESI)m/z = 641.2[M+H]⁺.

### Example 23

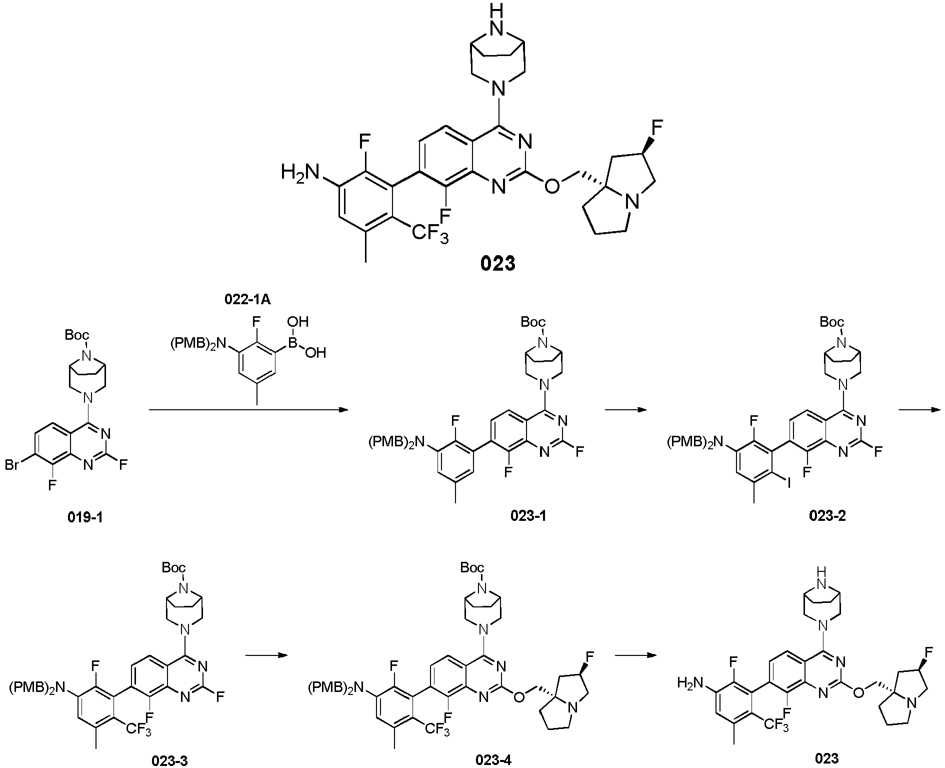

### Step 1: Synthesis of compound 023-1

**019-1** (1.1 g, 2.42 mmol, 1 eq) and **022-1A** (988.77 mg, 2.42 mmol, 1 eq) were dissolved in 1,4-dioxane (2 mL) and water (2 mL), and 1,1'-bis(di-tert-butylphosphino)ferrocene palladium dichloride (314.93 mg, 483.20 µmol, 0.2 eq) was added. The mixture was reacted at 90°C for 18 hours under nitrogen. After the reaction was completed, the mixture was extracted twice with 30 mL of ethyl acetate. The organic phases were combined, dried over anhydrous sodium sulfate, and filtered. The filtrate was concentrated and the crude product was purified by silica gel column (gradient elution: petroleum ether/ethyl acetate = 10:1 -5:1) to give compound **023-1.** LCMS:(ESI)m/z = 740.3 [M+H]⁺

### Step 2: Synthesis of compound 023-2

**023-1** (1.1 g, 1.49 mmol, 1 eq) was dissolved in N,N-dimethylformamide (2 mL) and N-iodosuccinimide (501.77 mg, 2.23 mmol, 1.5 eq) was added. The mixture was reacted at 25 °C for 2 h. After the reaction was completed, the mixture was extracted twice with 30 mL of ethyl acetate. The organic phases were combined, dried over anhydrous sodium sulfate, and filtered. The filtrate was concentrated to give a crude product. The crude product was purified on silica gel column (gradient elution: petroleum ether : ethyl acetate = 10:1 -5:1) to give compound **023-2.** LCMS:(ESI)m/z = 866.2 [M+H]⁺.

### Step 3: Synthesis of compound 023-3

**023-2** (1.1 g, 1.27 mmol, 1 *eq*) was dissolved in N,N-dimethylformamide (2 mL), and methyl fluorosulfonyldifluoroacetate (1.22 g, 6.35 mmol, 808.29 µL, 5 *eq*)*,* hexamethylphosphoramide (1.14 g, 6.35 mmol, 1.12 mL, 5 *eq*)*,* and cuprous iodide ( 483.98 mg, 2.54 mmol, 2 *eq*) were added. The mixture was refluxed at 80 °C under nitrogen for 18 h. After the reaction was completed, the mixture was extracted twice with 20 mL of ethyl acetate. The organic phases were combined, dried over anhydrous sodium sulfate, and filtered. The filtrate was concentrated, and the crude product was purified on silica gel column (gradient elution: petroleum ether : ethyl acetate = 10:1 -5:1) to give compound **023-3.** LCMS:(ESI)m/z = 866.2 [M+H]⁺.

### Step 4: Synthesis of compound 023-4

**023-3** (118.24 mg, 742.74 µmol, 1 *eq*) was dissolved in tetrahydrofuran (2 mL) and N,N-dimethylformamide (2 mL), and cesium carbonate (242.00 mg, 742.74 µmol, 1 *eq*)*,* triethylenediamine (8.33 mg, 74.27 µmol, 8.17 µL, 0.1 *eq*) and **001-2A** (0.6 g, 742.74 µmol, 1 *eq*) were added. The mixture was reacted at 25 °C for 18 h. After the reaction was completed, water was added and the mixture was extracted with 100 mL of ethyl acetate. The organic phase was dried over anhydrous sodium sulfate, and filtered. The filtrate was concentrated and the crude product was purified on silica gel column (gradient elution: dichloromethane : methanol = 100:1 -20:1) to give compound **023-4.** LCMS:(ESI)m/z = 947.4 [M+H]⁺.

### Step 5: Synthesis of compound 023

**023-4** (60 mg, 63.36 µmol, 1 *eq*) was dissolved in trifluoroacetic acid (7.22 mg, 63.36 µmol, 4.69 µL, 1 *eq*) and the solution was stirred at 25°C for 1 h. After the reaction was completed, the mixture was rotary-evaporated to dryness to remove the solvent and separated by preparative column: Phenomenex C18 150*40mm*5µm; mobile phase: [water (formic acid)-acetonitrile]; acetonitrile %: 5%-35%, 10min, to give compound **023** formate. LCMS:(ESI)m/z = 607.2[M+H]⁺; ¹H NMR (400 MHz, DMSO-*d₆*) δ ppm 1.68 - 1.80 (m, 6 H) 2.00 - 2.17 (m, 3 H) 2.36 (br s, 3 H) 2.83 (br d, J = 5.25 Hz, 2 H) 2.95 - 3.14 (m, 3 H) 3.66 (br s, 2 H) 3.98 - 4.12 (m, 2 H) 4.27 (br d, J = 11.76 Hz, 2 H) 5.18 - 5.39 (m, 1 H) 6.00 (br s, 2 H) 6.80 (br d, J = 8.75 Hz, 1 H) 7.10 (br t, J = 7.63 Hz, 1 H) 7.80 (br d, J = 8.76 Hz, 1 H) 8.24 (s, 1 H).

### Example 24

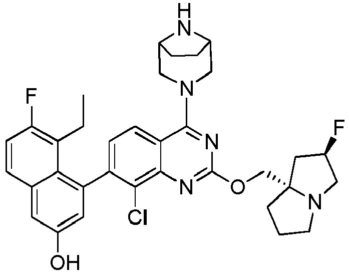

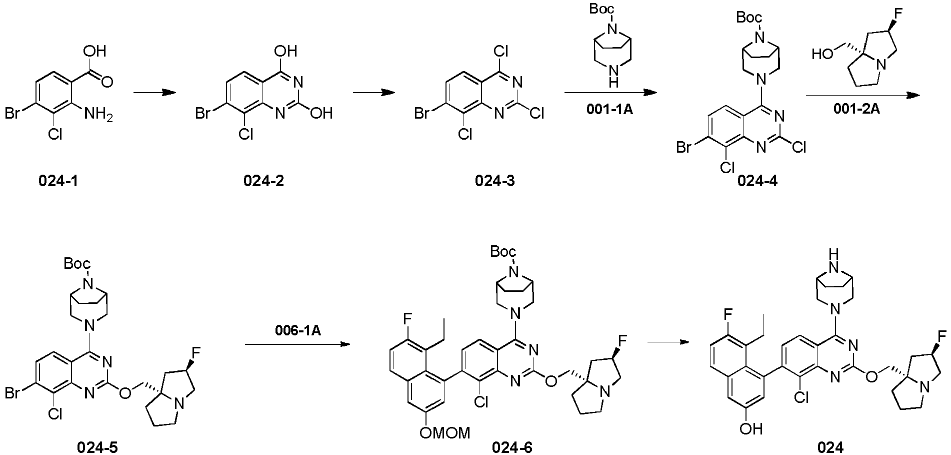

### Step 1: Synthesis of compound 024-2

**024-1** (4.8 g, 19.16 mmol, 1 *eq*) and urea (69.06 g, 1.15 mol, 61.66 mL, 60 *eq*) were added to a reaction flask and the mixture was stirred to react at 200 °C for 2 h. After the reaction was completed, the mixture was cooled down to room temperature, and 200 mL of water was added. The mixture was filtered by suction to give compound **024-2.** LCMS:(ESI)m/z = 274.9 [M+H]⁺.

### Step 2: Synthesis of compound 024-3

N,N-diisopropylethylamine (11.13 g, 86.12 mmol, 15.00 mL, 11.86 *eq*) was added dropwise to phosphorus oxychloride (50 mL) at 0 °C. **024-2** (2 g, 7.26 mmol, 1 *eq*) was added in batches and the mixture was refluxed at 80 °C for 20 h. After the reaction was completed, the reaction solution was evaporated to dryness to remove phosphorus trichloride. The crude product was purified on silica gel column (gradient elution: petroleum ether : ethyl acetate = 10:1 -3:1) to give compound **024-3.** LCMS:(ESI)m/z = 310.8 [M+H]⁺.

### Step 3: Synthesis of compound 024-4

**024-3** (1 g, 3.20 mmol, 1 *eq*) was dissolved in N,N-dimethylformamide (2 mL), and **001-1A** (679.59 mg, 3.20 mmol, 1 *eq*) and N,N-diisopropylethylamine (1.24 g, 9.60 mmol, 1.67 mL, 3 *eq*) were added. The mixture was stirred to react at 25°C for 2 h. After the reaction was completed, the reaction solution was poured into 200 mL of water and a solid was precipitated. The solid was washed with 60 mL of water to give compound **024-4.** LCMS:(ESI)m/z = 487.0 [M+H]⁺.

### Step 4: Synthesis of compound 024-5

**024-4** (1.3 g, 2.66 mmol, 1 *eq*) was dissolved in N,N-dimethylformamide (10 mL) and tetrahydrofuran (10 mL), and then cesium carbonate (867.60 mg, 2.66 mmol, 1 *eq*)*,* **001-2A** (635.88 mg, 3.99 mmol, 1.5 *eq*)*,* and triethylenediamine (29.87 mg, 266.28 µmol, 29.28 µL, 0.1 eq) were added. The mixture was stirred to react at 25 °C for 18 h. After the reaction was completed, the mixture was extracted with 50 mL of ethyl acetate, and washed with saturated brine. The organic phases were combined, dried over anhydrous sodium sulfate, and filtered. The filtrate was concentrated, and the crude product was purified on silica gel column (gradient elution: petroleum ether : ethyl acetate = 100:1 -20:1) to give compound **024-5.** LCMS:(ESI)m/z = 610.2 [M+H]⁺.

### Step 5: Synthesis of compound 024-6

**024-5** (0.2 g, 327.36 µmol, 1 *eq*) and compound **006-1A** (91.03 mg, 327.36 µmol, 1 *eq*) were dissolved in 1,4-dioxane (1 mL) and water (0.2 mL). [1,1-bis(diphenylphosphino)ferrocene]palladium dichloride (21.34 mg, 32.74 µmol, 0.1 *eq*)*,* and potassium phosphate (104.23 mg, 491.04 µmol, 1.5 *eq*) were added. The mixture was reacted at 90 °C under nitrogen for 18 h. After the reaction was completed, the reaction solution was evaporated to dryness and the crude product was purified on silica gel column (gradient elution: petroleum ether : ethyl acetate = 100:1 -20:1) to give compound **024-6.** LCMS:(ESI)m/z = 764.3 [M+H]⁺.

### Step 6: Synthesis of compound 024

**024-6** (100 mg, 130.84 µmol, 1 *eq*) was dissolved in hydrochloric acid/1,4-dioxane (2 mL), and acetonitrile (2 mL) was added. The mixture was stirred to react at 25°C for 15 h. The reaction solution was rotary-evaporated to dryness and separated by preparative HPLC: column: Phenomenex C18 150*40mm*5µm; mobile phase: [water (formic acid)-acetonitrile] ; Acetonitrile %: 1%-30%, 10 min, to give compound **024** formate. ¹H NMR (400 MHz, DMSO-*d₆*) δ ppm 0.71 (br s, 3 H) 1.69 - 1.91 (m, 8 H) 2.04 (br d, J = 18.89 Hz, 2 H) 2.23 (br s, 1 H) 2.83 (br s, 2 H) 3.01 (br s, 2 H) 3.09 (br s, 3 H) 3.92 - 4.20 (m, 4 H) 4.23 - 4.35 (m, 2 H) 5.20 - 5.38 (m, 1 H) 6.83 (br s, 1 H) 7.25 - 7.39 (m, 3 H) 7.74 (br s, 1 H) 7.95 (br d, J = 7.25 Hz, 1 H) 8.28 (br s, 1 H).

### Example 25

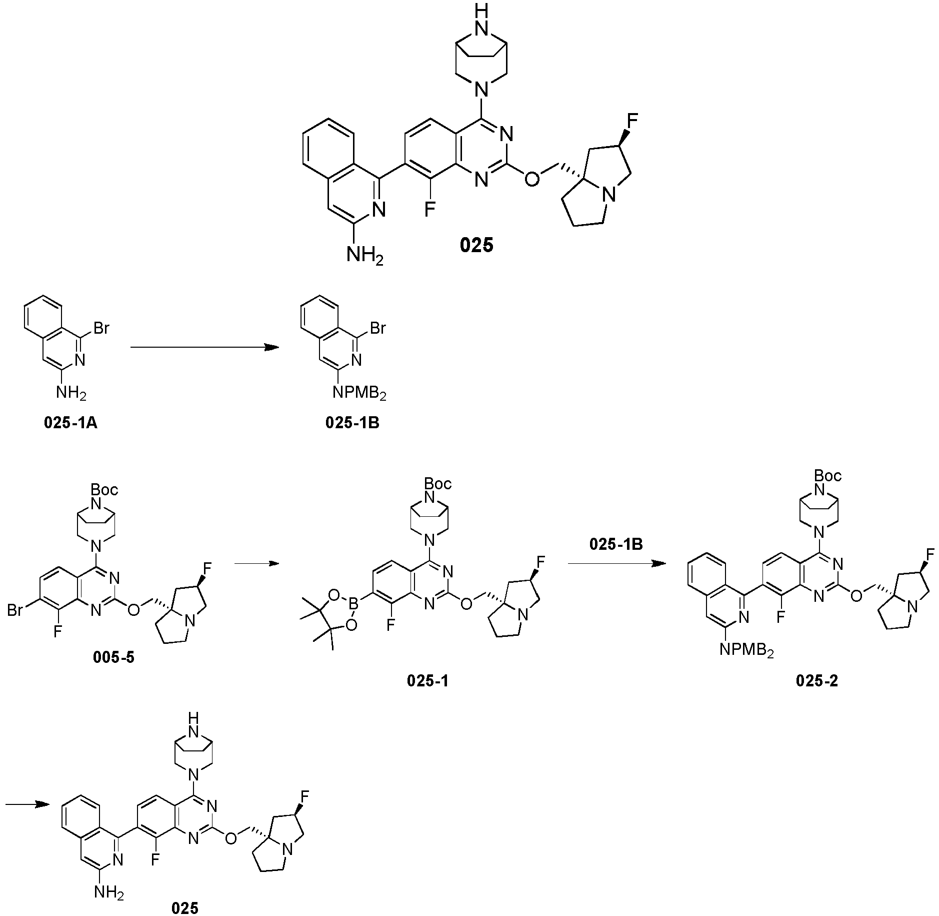

### Step 1: Synthesis of compound 025-1B

**025-1A** (0.9 g, 4.03 mmol, 1 *eq)* was dissolved in N-methylpyrrolidone (5 mL), and potassium carbonate (1.39 g, 10.09 mmol, 2.5 *eq),* potassium iodide (669.76 mg, 4.03 mmol, 1 *eq)* and p-methoxybenzyl chloride (1.58 g, 10.09 mmol, 1.37 mL, 2.5 *eq)* were added. The mixture was reacted at 25 °C for 15 h. After the reaction was completed, the mixture was extracted twice with 20 mL of ethyl acetate. The organic phases were combined, dried over anhydrous sodium sulfate, and filtered. The filtrate was concentrated and the crude product was purified on silica gel column (gradient elution: petroleum ether : ethyl acetate = 100:1 -10:1) to give compound **025-1B.** LCMS:(ESI)m/z = 463.1[M+H]⁺.

### Step 2: Synthesis of compound 025-1

**005-5** (0.6 g, 1.01 mmol, 1 *eq*) was dissolved in 1,4-dioxane (10 mL), and bis(pinacolato)diboron (384.44 mg, 1.51 mmol, 1.5 *eq*)*,* bis(diphenylphosphino)ferrocene palladium dichloride (73.85 mg, 100.93 µmol, 0.1 *eq*)*,* and potassium acetate (148.58 mg, 1.51 mmol, 1.5 *eq*) were added. The mixture was refluxed at 105 °C for 15 h. After the reaction was completed, the reaction solution was rotary-evaporated to dryness and separated by preparative HPLC: column: Phenomenex C18 150*40 mm*5 µm; mobile phase: [water (formic acid)-acetonitrile]; acetonitrile %: 10%-40%, 10 min, to give compound **025-1** (110 mg, 171.46 µmol, 16.99% yield). LCMS:(ESI)m/z = 560.3[M-82+H]⁺.

### Step 3: Synthesis of compound 025-2

**025-1** (82.83 mg, 178.76 µmol, 1 *eq*) and **025-1B** (100 mg, 178.76 µmol, 1 *eq*) were dissolved in 1,4-dioxane (2 mL) and water (0.4 mL), and potassium phosphate (56.92 mg, 268.14 µmol, 1.5 *eq*), and 1,1-bis(tert-butylphosphine)ferrocene palladium chloride (11.65 mg, 17.88 µmol, 0.1 *eq*) were added. The mixture was reacted at 80 °C under nitrogen for 15 h. After the reaction was completed, the reaction solution was directly evaporated to dryness and the crude product was purified on silica gel column (gradient elution: petroleum ether : ethyl acetate = 100:1 -30:1) to give compound **025-2.** LCMS:(ESI)m/z = 898.4[M+H]⁺.

### Step 4: Synthesis of compound 025

**025-2** (100 mg, 111.35 µmol, 1 *eq*) was dissolved in trifluoroacetic acid (2 mL) and the solution was stirried at 25 °C for 2 h. After the reaction was completed, the reaction mixture was rotary-evaporated to dryness to remove the solvent and separated by preparative HPLC: column: Phenomenex C18 150*40 mm*5 µm; mobile phase: [water (formic acid)-acetonitrile]; acetonitrile %: 55%-85%, 10min, to give compound **025** formate. LCMS:(ESI)m/z = 558.3[M+H]⁺.

### Example 26

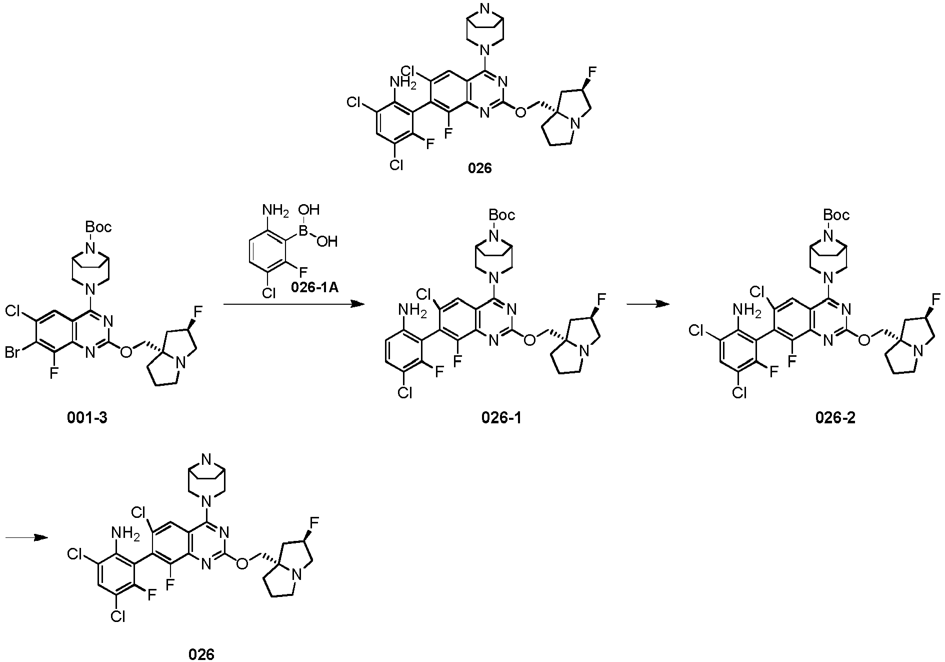

### Step 1: Synthesis of compound 026-1

Compound **001-3** (0.30 g, 477.00 µmol, 1 eq) and compound **026-1A** (180.67 mg, 953.99 µmol, 2 eq) and anhydrous potassium phosphate (506.25 mg, 2.38 mmol, 5 eq ) were added to 1,4-dioxane (15 mL). The atmosphere was replaced with nitrogen three times and (2-dicyclohexylphosphino-2,4,6-triisopropyl-1,1-biphenyl)[2-(2-amino-1,1-biphenyl)]palladium(II) chloride (37.53 mg, 47.70 µmol, 0.1 eq) was added under nitrogen. The atmosphere was replaced with nitrogen three times. The mixture was reacted at 100 °C for 16 h. After the reaction was completed, the reaction solution was cooled down to room temperature, and 20 mL of water and 20 mL of ethyl acetate were added to the reaction solution. The reaction solution was stirred for 5 min and then left to stand. The layers were separated. The aqueous phase was extracted once with 20 mL ethyl acetate. The organic phases were combined, washed three times with 20 mL of saturated brine, then dried with anhydrous sodium sulfate, and filtered. The filtrate was concentrated to give a crude product, which was purified using a column (petroleum ether:ethyl acetate = 100:0-0:100) to give compound **026-1.** LCMS:(ESI)m/z = 693.2[M+H]⁺; ¹H NMR (400 MHz, CDCl₃) δ ppm 7.7 (s, 1 H) 7.30-7.23 (m, 1H), 6.67-6.48 (m, 1H), 5.37 -5.24 (m, 1 H), 4.43-4.21 (m, 6H), 3.79-3.48 (m, 3H), 3.39 (s, 2H), 3.33-3.15 (m, 2H), 3.11-2.93 (m, 1H), 2.43-2.16 (m, 3H), 2.01-1.94 (m, 5H), 1.85-1.76 (m, 2H), 1.53 (s, 9H).

### Step 2: Synthesis of compound 026-2

Compound **026-1** (110 mg, 158.60 µmol, 1 eq) was added to N,N-dimethylformamide (2 mL), and N-chlorosuccinimide (23.30 mg, 174.46 µmol, 1.1 eq) was added. The mixture was reacted at 70°C for 3 h. After the reaction was completed, 10 mL of ethyl acetate and 5 mL of water were added to the reaction solution and the mixture was stirred for 5 min. The mixture was left to stand and the layers were separated. The aqueous phase was extracted once with 10 mL ethyl acetate. The organic phases were combined, washed twice with 20 mL saturated brine, dried over anhydrous sodium sulfate, and filtered. The filtrate was concentrated to give a crude product, which was purified by silica gel column (dichloromethane : methanol (with 1 ‰ ammonia added dropwise) = 20:1) to give compound **026-2.** LCMS:(ESI)m/z = 727.1[M+H]⁺.¹HNMR (400 MHz, CDCl₃) δ ppm 7.77 (s, 1H), 7.42 (d, J = 7.25 Hz, 1H), 5.34-5.20 (m, 1H),4.40-4.27 (m, 5 H), 3.7-3.5 (s, 3H), 3.32-3.21 (m, 2H), 3.15-3.19 (m, 1H) 2.93-3.04 (m, 2H), 2.32-2.10 (m, 4H), 2.00-1.76 (m, 7H), 1.52 (s, 9H).

### Step 3: Synthesis of compound 026

Compound **026-2** (40 mg, 54.94 µmol, 1 eq) was added to anhydrous dichloromethane (2 mL) and trifluoroacetic acid (308.00 mg, 2.70 mmol, 200.00 µL, 49.16 eq) was added. The mixture was reacted at 25°C for 2 h. Solid was precipitated. After the reaction was completed, the reaction solution was concentrated to dryness under reduced pressure and the crude product was purified by preparative chromatography (column: Phenomenex Luna 80*30mm*3µm; mobile phase: [water (hydrochloric acid)-acetonitrile]; acetonitrile %: 15%-35%, 7min) to give compound **026** hydrochloride. ¹H NMR (400 MHz, CD₃OD ) THANOL-d MHz, a 80*30mm*3.00 J = 7.25 Hz, 1H), 5.34-5.20 (m, 1H),4.40-4.27 (m, 5 H), 3.7-3.5 (s, 3H), 3.32-3.21 (m, 2H), 3.15-3.19m, 2H), 3.84-4.04 (m, 3H), 3.42-3.59 (m, 1H), 2.56-2.82 (m, 2H), 2.44-2.54 (m, 1H), 2.31-2.42 (m, 2H), 2.29-2.14 (m, 5 H).

### Example 27

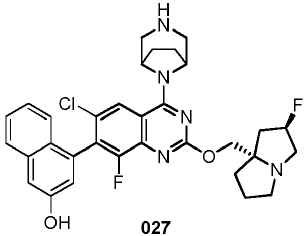

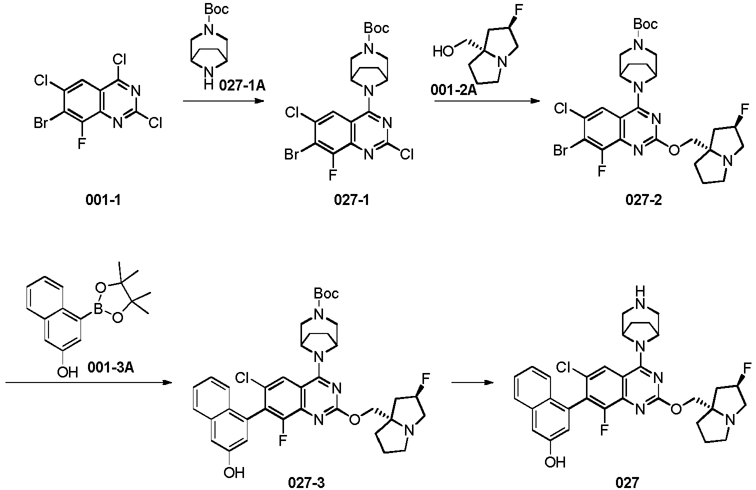

### Step 1: Synthesis of compound 027-1

Compound **001-1** (400 mg, 1.21 mmol, 1 eq) was dissolved in anhydrous N,N-dimethylformamide (10 mL) at 20 °C, and N,N-diisopropylethylamine (391.21 mg, 3.03 mmol, 527.24 µL, 2.5 eq) and **027-1A** (257.03 mg, 1.21 mmol, 1 eq) were added. The mixture was stirred for 0.5 h. After the reaction was completed, the mixture was diluted with ethyl acetate (150 mL), washed with saturated brine (30 mL), and dried with anhydrous sodium sulfate. The organic phase was separated and dried. The organic solvent was removed under reduced pressure to give a crude product **027-1.** ¹H NMR (400MHz, CDCl₃) δ = 7.79 (d, J = 2.0 Hz, 1H), 4.93 (br d, J = 12.8 Hz, 1H), 5.00 - 4.87 (m, 1H), 4.15 -3.90 (m, 2H), 3.42 - 3.20 (m, 2H), 2.06 - 1.85 (m, 4H), 1.50 (s, 9H).

### Step 2: Synthesis of compound 027-2

Compound **027-1** (600 mg, 1.19 mmol, 1 eq) was dissolved in tetrahydrofuran (3 mL) and N,N-dimethylformamide (3 mL), and **001-2A** (284.17 mg, 1.78 mmol, 1.5 eq), cesium carbonate (1.16 g, 3.57 mmol, 3 eq) and triethylenediamine (13.35 mg, 119.00 µmol, 13.09 µL, 0.1 eq) were added sequentially under nitrogen. The reaction solution was stirred at 20°C for another 16 h. After the reaction was completed, 100 mL of ethyl acetate was added to the reaction solution. The organic phase was washed with saturated brine (10 mL), dried with anhydrous sodium sulfate, filtered, and dried. The organic solvent was removed under reduced pressure, and the resulting crude product was separated by silica gel column chromatography (eluent: ethyl acetate:petroleum ether = 0-80%) to give compound **027-2.** ¹H NMR (400MHz, CDCl₃) δ = 7.73 (d, J = 2.0 Hz, 1H), 5.38 - 5.20 (m, 1H), 4.89 - 4.75 (m, 2H), 4.29 - 4.21(m, 1H), 4.19 - 4.13 (m, 1H), 4.04 (br d, J = 12.0 Hz, 1H), 3.91 (br d, J = 12.0 Hz, 1H), 3.43 - 3.16 (m, 5H), 3.03 - 2.97 (m, 1H),2.34 - 2.11 (m, 3H), 2.01 - 1.76 (m, 8H), 1.65 (br s, 1H), 1.50 (s, 9H).

### Step 3: Synthesis of compound 027-3

Under nitrogen protection, **027-2** (50 mg, 79.50 µmol, 1 eq), **001-3A** (25.77 mg, 95.40 µmol, 1.2 eq) and sodium carbonate (25.28 mg, 238.50 µmol, 3 eq) were dissolved in 1,4-dioxane (2 mL) and water (1 mL). [1,1'-bis(diphenylphosphino)ferrocene]palladium dichloride dichloromethane complex (6.49 mg, 7.95 µmol, 0.1 eq) was added. The mixture was reacted at 100 °C for 3 h. After the reaction was completed, the mixture was cooled and the organic solvent was removed under reduced pressure. The resulting crude product was purified by a preparative silica gel plate (developing agent: dichloromethane:methanol = 10:1) to give compound **027-3.** LCMS:(ESI)m/z = 692.2[M+H]⁺.

### Step 4: Synthesis of compound 027

Compound **027-3** (40 mg, 57.79 µmol, 1 eq) was dissolved in dichloromethane (2 mL) at 20 °C. Trifluoroacetic acid (770.00 mg, 6.75 mmol, 0.5 mL, 116.86 eq) was added and the reaction solution was stirred for 1 h. After the reaction was completed, the reaction solution was rotary-evaporated to dryness and the resulting crude product was purified by preparative chromatography (column: Phenomenex Synergi C18 150*30 mm*4 µm; mobile phase: [water (0.05% hydrochloric acid)-acetonitrile]; % acetonitrile: 20%-50%, 9 min) to give compound **027** hydrochloride. LCMS:(ESI)m/z = 592.1[M+H]⁺.

### Example 029

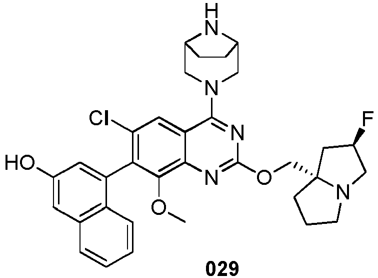

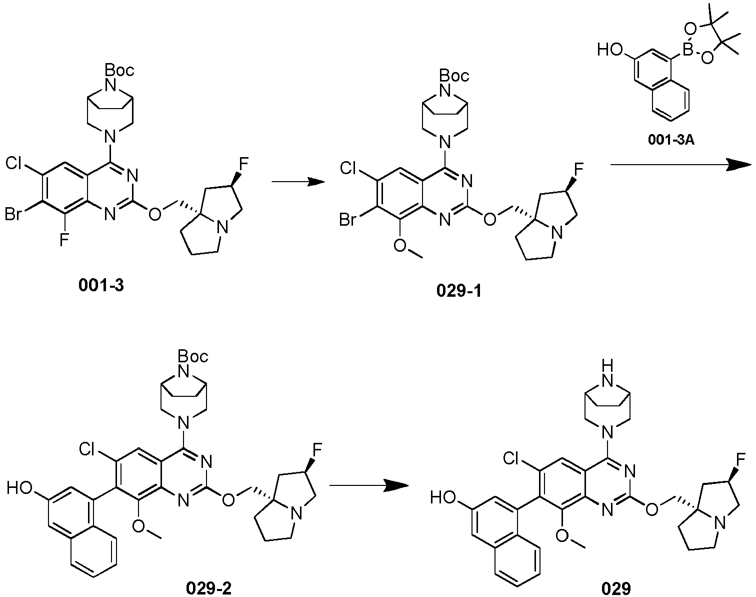

### Step 1: Synthesis of compound 029-1

Compound **001-3** (500 mg, 794.99 µmol, 1 eq) was dissolved in tetrahydrofuran (5 mL) at 20 °C, and sodium methanol (85.90 mg, 1.59 mmol, 2 eq) was added. The reaction solution was heated to 70 °C and stirred for 48 h. After the reaction was completed, the mixture was rotary-evaporated to dryness to remove the solvent and the resulting crude product was purified by a preparative silica gel plate (developing agent: ethyl acetate/petroleum ether = 0~ 100%) to give compound **029-1.** ¹H NMR (400MHz, CDCl₃) δ = 7.65 (s, 1H), 5.37 (br s, 1H), 5.24 (br s, 1H), 4.28 (br d, J = 7.3 Hz, 6H), 4.15 -4.12 (m, 3H), 3.54 (br s, 2H), 3.37 - 3.17 (m, 3H), 3.01 (br s, 1H), 2.34 - 2.10 (m, 3H), 2.01 - 1.88 (m, 5H), 1.81 (br d, J = 8.0 Hz, 2H), 1.52 (s, 9H).

### Step 2: Synthesis of compound 029-2

Compound **029-1** (50 mg, 78.01 µmol, 1 eq), **001-3A** (25.29 mg, 93.61 µmol, 1.2 eq) and cesium carbonate (76.25 mg, 234.02 µmol, 3 eq) were dissolved in 1,4-dioxane (5 mL) and water (1 mL) under nitrogen, and Pd(PPh₃)₄ (9.01 mg, 7.80 µmol, 0.1 eq) was added. The mixture was reacted at 100 °C for 16 h. After the reaction was completed, the mixture was rotary-evaporated to dryness to remove the solvent and the resulting crude product was purified by a preparative silica gel plate (developing agent: dichloromethane:methanol = 10:1) to give compound **029-2,** LCMS:(ESI)m/z = 704.3 [M+H]⁺.

### Step 3: Synthesis of compound 029

Compound **029-2** (20.00 mg, 28.40 µmol, 1 eq) was dissolved in dichloromethane (3 mL) at 20 °C and trifluoroacetic acid (1.54 g, 13.51 mmol, 1 mL, 475.58 eq) was added. The reaction solution was stirred for another 16 h. After the reaction was completed, the mixture was rotary-evaporated to dryness to remove the solvent and purified by preparative chromatography (column: Welch Xtimate C18 100*40 mm*3 µm; mobile phase: [water (trifluoroacetic acid)-acetonitrile]; acetonitrile %: 15%-45%, 8 min) to give compound **029** trifluoroacetate. LCMS:(ESI)m/z = 604.5 [M+H]⁺.

### Example 30

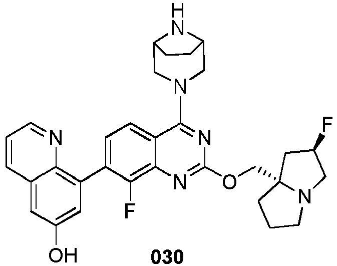

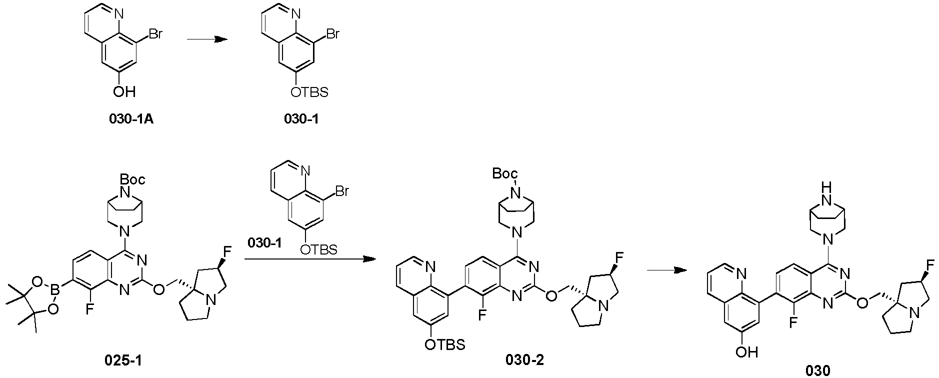

### Step 1: Synthesis of compound 030-1

Compound **030-1A** (0.22 g, 981.91 µmol, 1 *eq*) and imidazole (147.07 mg, 2.16 mmol, 2.2 *eq*) were dissolved in anhydrous dichloromethane (4 mL), and the solution was cooled down to 0°C. Tert-butyldimethylchlorosilane (162.79 mg, 1.08 mmol, 132.35 µL, 1.1 *eq*) was added dropwise. The atmosphere was replaced with nitrogen. The reaction system was slowly warmed up to 20°C and stirred for 3 hours. After the reaction was completed, the reaction system was diluted by adding 5 mL of dichloromethane and 10 mL of water. The layers were separated. The organic phase was collected and the aqueous phase was extracted with 30 mL of dichloromethane. The organic phases were combined, washed with saturated brine, dried over anhydrous sodium sulfate, and concentrated under reduced pressure to give a crude product, which was purified by column chromatography (gradient elution: petroleum ether : ethyl acetate = 10:0- 1:10) to give compound **030-1.** LCMS:(ESI)m/z = 338.0[M+H]⁺

### Step 2: Synthesis of compound 030-2

Compound **025-1** (0.2 g), compound **030-1** (43.54 mg, 128.71 µmol, 1.2 *eq*) and sodium carbonate (34.10 mg, 321.77 µmol, 3 *eq*) were added to 1,4-dioxane (4 mL) and water (0.8 mL). The atmosphere was replaced with nitrogen three times, and [1,1-bis(diphenylphosphino)ferrocene]palladium dichloride (7.85 mg, 10.73 µmol, 0.1 *eq*) was added under nitrogen. The mixture was reacted at 100 °C for 2 h. After the reaction was completed, 5 mL of ethyl acetate was added to the reaction solution. The organic phase was washed with 10 mL of saturated brine, dried with anhydrous sodium sulfate and filtered. The filtrate was concentrated, and the crude product was purified by preparative high performance liquid chromatography (column: Phenomenex luna C18 80*40mm*3 µm; mobile phase A: water (HCl), mobile phase B: acetonitrile; running gradient: acetonitrile %: 40%-70%, running time: 7 min) to give compound **030-2.** LCMS:(ESI)m/z = 773.3[M+H]⁺.

### Step 3: Synthesis of compound 030

Compound **030-2** (0.035 g, 45.28 µmol, 1 eq) was added to acetonitrile (2 mL) and hydrochloric acid/1,4-dioxane (4 M, 226.39 µL, 20 eq) was added. The mixture was reacted at 25 °C for 2 h. After the reaction was completed, the reaction solution was added to 5 mL of water. The mixture was extracted twice using 3 mL of ethyl acetate and the aqueous phase was concentrated to give compound **030.** LCMS:(ESI)m/z = 559.3[M+H]⁺. ¹H NMR (400 MHz, CD₃OD ) δ = 8.47 - 8.43 (m, 1H), 8.15 - 8.10 (m, 1H), 7.81 (d, *J* = 8.8 Hz, 1H), 7.43 - 7.27 (m, 3H), 7.14 (d, *J* = 2.4 Hz, 1H), 5.40 - 5.22 (m, 1H), 4.57-4.50 (m, 2H), 4.33 - 4.17 (m, 2H), 3.66 - 3.57 (m, 4H), 3.26 - 3.13 (m, 3H), 3.03-2.95 (m, 1H), 2.42 - 2.27 (m, 1H), 2.41 - 2.09 (m, 2H), 1.94 (s, 2H), 1.89 (s, 2H), 1.84 (s, 3H).

### Example 31

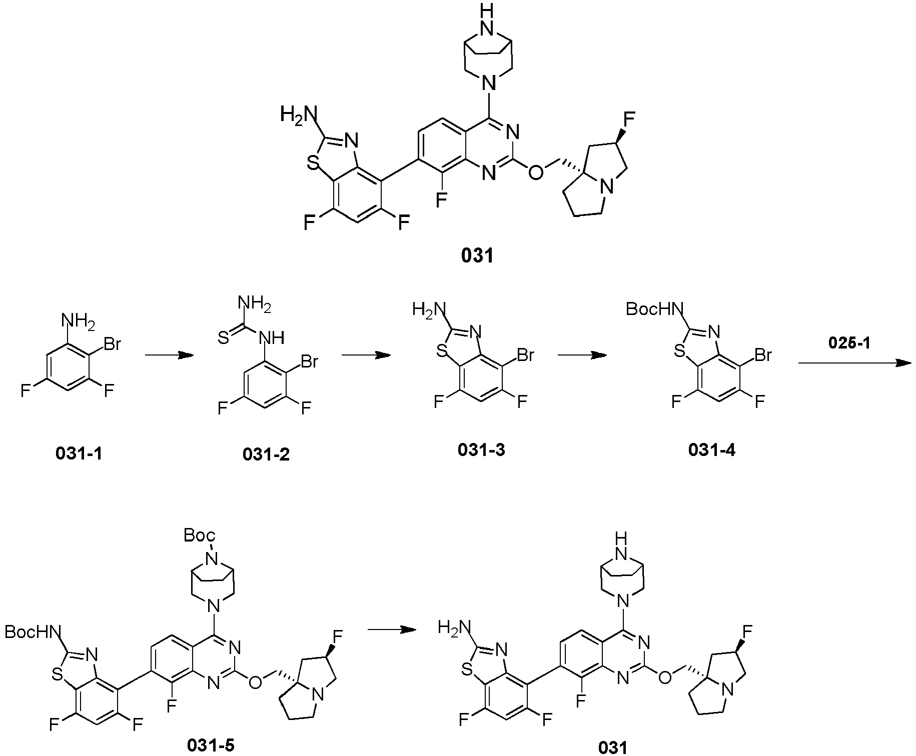

### Step 1: Synthesis of compound 031-2

Ammonium thiocyanate (2.38 g, 31.25 mmol, 2.38 mL, 1.3 *eq*) was added to acetone (50 mL), and benzoyl chloride (3.38 g, 24.04 mmol, 2.79 mL, 1 *eq*) were added. The mixture was reacted at 70°C for 0.5 h. The mixture was cooled down to 50°C, and a solution of compound **031-1** (5 g, 24.04 mmol, 1 *eq*) in acetone (7 mL) was added in batches. The mixture was reacted at 70°C for 0.5 h. Sodium hydroxide (2 M, 42.07 mL, 3.5 *eq*) was added and the mixture was refluxed for another 0.5 h. After the reaction was completed, the reaction solution was cooled down to room temperature. The reaction solution was adjusted to pH 5 with hydrochloric acid, and then adjusted to pH 8 with ammonia. The mixture was stirred for 0.5 h, and then extracted twice with 30 mL of ethyl acetate. The organic phases were combined, dried with anhydrous sodium sulfate and filtered. The filtrate was concentrated to give a crude product, and the crude product was slurried with stirring with 30 mL of dichloromethane for 1 h. The mixture was filtered to give compound **031-2.** LCMS:(ESI)m/z = 266.9[M+H]⁺.

### Step 2: Synthesis of compound 031-3

Compound **031-2** (2 g, 7.49 mmol, 1 *eq*) was added to dichloroethane (20 mL) and a solution of bromine (2.39 g, 14.98 mmol, 772.04 µL, 2 *eq*) in dichloroethane (2 mL) was added. The mixture was reacted at 95 °C for 2 h. After the reaction was completed, the reaction solution was filtered and the filter cake was rinsed with 20 mL of 1,2-dichloroethane to give compound **031-3.** LCMS:(ESI)m/z = 264.8[M+H]⁺.

### Step 3: Synthesis of compound 031-4

Compound **031-3** (1.0 g, 3.77 mmol, 1 *eq*) was added to anhydrous tetrahydrofuran (15 mL), and N,N-diethylisopropylamine (1.22 g, 9.43 mmol, 1.64 mL, 2.5 *eq*) and 4-dimethylaminopyridine (46.09 mg, 377.25 µmol, 0.1 *eq*) were added. Di-tert-butyl dicarbonate (988.01 mg, 4.53 mmol, 1.04 mL, 1.2 eq) was added under nitrogen to the reaction solution. The mixture was reacted at 20 °C for 19 h. After the reaction was completed, 20 mL of water was added to the reaction solution. The mixture was extracted twice using 10 mL of ethyl acetate. The organic phases were combined, washed with 20 mL of saturated brine, dried with anhydrous sodium sulfate and filtered. The filtrate was concentrated to give a crude product. The crude product was slurried with 10 mL of dichloromethane for 1 h, and filtered to give compound **031-4.** LCMS:(ESI)m/z = 308.8[M-55]⁺.

### Step 4: Synthesis of compound 031-5

Compound **025-1** (0.2 g), compound **031-4** (47.00 mg, 128.71 µmol, 1.2 *eq)* and sodium carbonate (34.10 mg, 321.77 µmol, 3 *eq)* were added to 1,4-dioxane (4 mL) and water (0.8 mL). The atmosphere was replaced three times with nitrogen, and [1,1-bis(diphenylphosphino)ferrocene]palladium dichloride (7.85 mg, 10.73 µmol, 0.1 eq) was added under nitrogen. The mixture was reacted at 100 °C for 2 h. After the reaction was completed, 5 mL of ethyl acetate was added to the reaction solution. The mixture was washed using 8 mL of water and saturated brine, dried with anhydrous sodium sulfate, and filtered. The filtrate was concentrated to give a crude product. The crude product was purified by preparative high performance liquid chromatography (column: Phenomenex luna C18 80*40mm*3 µm; mobile phase A: water (hydrochloric acid), mobile phase B: acetonitrile; running gradient: acetonitrile %: 40%-70%, running time: 7 min) to give compound **031-5.** LCMS:(ESI)m/z = 800.3[M+H]⁺.

### Step 5: Synthesis of compound 031

Compound **031-5** (0.025 g, 31.25 µmol, 1 *eq*) was added to acetonitrile (1 mL), and hydrochloric acid/1,4-dioxane (4 M, 156.27 µL, 20 *eq*) was added. The mixture was reacted at 25 °C for 7 h. After the reaction was completed, the reaction solution was added to 5 mL of water. The mixture was extracted twice with 3 mL of ethyl acetate, and the crude product was purified by preparative high performance liquid chromatography (column: Phenomenex luna C18 80*40mm*3 µm; mobile phase A: water (hydrochloric acid), mobile phase B: acetonitrile; running gradient: acetonitrile %: 10%-35%, running time: 7 min) to give compound **031** hydrochloride. LCMS:(ESI)m/z = 600.2[M+H]⁺; ¹H NMR (400 MHz, CD₃OD ) δ = 8.09 - 7.97 (m, 1H), 7.73 - 7.62 (m, 1H), 7.04 - 6.93 (m, 1H), 5.70 - 5.52 (m, 1H), 4.34 - 4.27 (m, 2H), 4.17 - 4.03 (m, 3H), 4.00 - 3.85 (m, 3H), 3.52 - 3.43 (m, 3H), 2.91 - 2.57 (m, 3H), 2.54 - 2.45 (m, 1H), 2.42 - 2.32 (m, 2H), 2.28 - 2.21 (m, 1H), 2.19 - 2.09 (m, 4H).

### Example 32

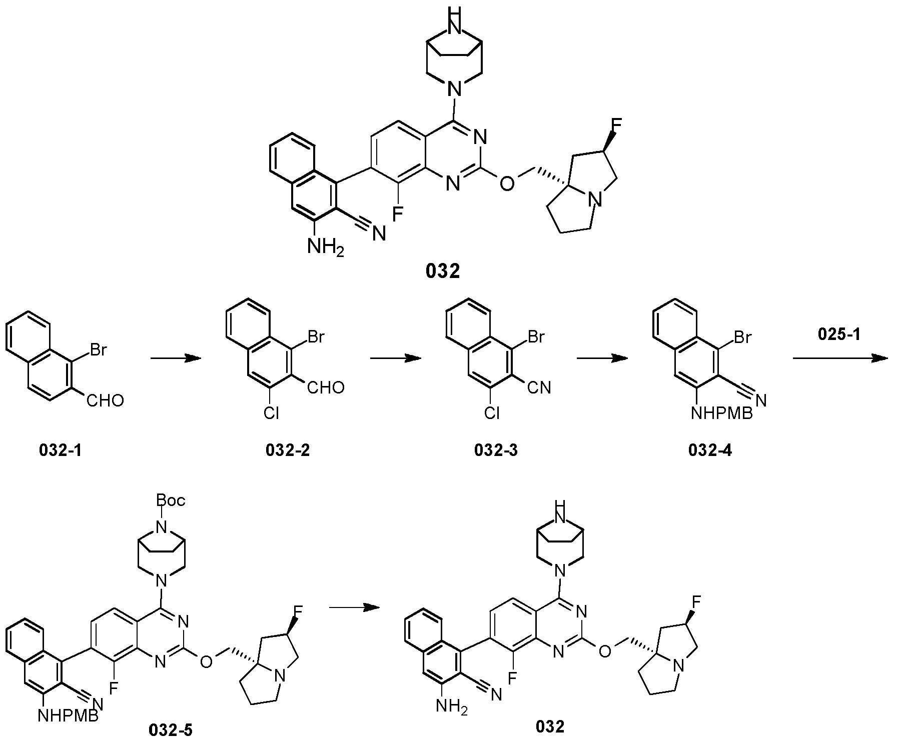

### Step 1: Synthesis of compound 032-2

Compound **032-1** (5 g, 21.27 mmol, 1 *eq*)*,* N-chlorosuccinimide (4.26 g, 31.90 mmol, 1.5 *eq*)*,* 4-nitro-2-(trifluoromethyl)phenol (1.32 g, 6.38 mmol, 0.3 *eq*) and 4-trifluoromethylaniline (342.71 mg, 2.13 mmol. 263.62 µL, 0.1 *eq*) were dissolved in dichloroethane (20 mL), and trifluoroacetic acid (24.25 g, 212.70 mmol, 15.75 mL, 10 *eq)* and palladium acetate (77.53 mg, 2.13 mmol, 0.1 *eq*) were added under nitrogen. The mixture was reacted at 80 °C for 16 h. After the reaction was completed, the reaction solution was cooled down to room temperature. 20 mL of water was added to the reaction solution and the mixture was extracted twice with 20 mL of dichloromethane. The organic phases were combined, dried with anhydrous sodium sulfate and concentrated to give a crude product, which was purified using a column (petroleum ether:ethyl acetate = 10:1) to give compound **032-2.** LCMS:(ESI)m/z = 268.9[M+H]⁺.

### Step 2: Synthesis of compound 032-3

Compound **032-2** (3 g, 11.13 mmol, 1 *eq*) was added to dimethyl sulfoxide (10 mL), and then hydroxylamine hydrochloride (1.55 g, 22.26 mmol, 2 *eq*) was added. The mixture was reacted at 95°C for 6 h. After the reaction was completed, 30 mL of water was added to the reaction solution. The mixture was extracted 3 times using 30 mL of methyl tert-butyl ether. The organic phases were combined, washed with 20 mL of saturated brine, dried over anhydrous sodium sulfate and concentrated to give compound **032-3.** ¹H NMR (400 MHz, CD₃OD ) δ = 8.31-8.26 (m, 1H), 7.97-7.91 (m, 1H), 7.83-7.81 (m, 1H), 7.78-7.50 (m, 2H).

### Step 3: Synthesis of compound 032-4

p-Methoxybenzylamine (617.64 mg, 4.50 mmol, 582.68 µL, 1.2 *eq*) and compound 032-3 (1 g, 3.75 mmol, 1 *eq*) were added to N,N-dimethylformamide (5 mL), and then potassium carbonate (1.04 g, 7.50 mmol, 2 *eq*) was added. The mixture was reacted at 80°C for 16 h. After the reaction was completed, 30 mL of water was added to the reaction solution. The mixture was extracted twice using 20 mL of methyl tert-butyl ether. The organic phases were combined, washed with 30 mL of saturated brine, dried over anhydrous sodium sulfate and concentrated. The crude product was purified by silica gel column chromatography (petroleum ether:ethyl acetate = 1:0-1:1) to give compound **032-4.** ¹H NMR (400 MHz, CD₃OD ) δ = 7.78-7.73 (m, 1H),7.61 (d, J = 8.4 Hz, 1H), 7.58-7.50(m, 1H), 7.40(d, J = 7.2 Hz, 1H), 7.30-7.24 (m,3H), 6.85-6.78 (m, 1H).

### Step 4: Synthesis of compound 032-5

**Compound 032-4** (70.90 mg, 193.06 µmol, 1.2 *eq*) and sodium bicarbonate (27.03 mg, 321.77 µmol, 12.51 µL, 2 *eq*) were added to 1,4-dioxane (5 mL) and water (3 mL). The atmosphere was replaced with nitrogen three times, and [1,1-bis(diphenylphosphino)ferrocene]palladium dichloride (11.77 mg, 16.09 µmol, 0.1 *eq*) was added under nitrogen. The mixture was heated to 100 °C, and compound **025-1** (0.09 g, 160.88 µmol, 1 *eq*) was added. The atmosphere was replaced with nitrogen three times, and the mixture was reacted under nitrogen at 100 °C for 1 h. After the reaction was completed, the reaction solution was poured into 30 mL of water, and the mixture was extracted three times with 20 mL of ethyl acetate. The organic phases were combined, washed with 20 mL of saturated brine, then dried with anhydrous sodium sulfate and concentrated. The crude product was purified by preparative high performance liquid chromatography (column: Phenomenex luna C18 80*40mm*3 µm; mobile phase A: water (hydrochloric acid), mobile phase B: acetonitrile; running gradient: acetonitrile %: 34%-54%, running time: 7 min) to give compound **032-5.** LCMS:(ESI)m/z = 802.2[M+H]⁺.

### Step 5: Synthesis of compound 032

Compound **032-5** (0.002 g, 2.49 µmol, 1 *eq*) was added to anhydrous dichloromethane (0.25 mL) and trifluoroacetic acid (0.05 mL). The mixture was reacted at 25 °C for 5 h. After the reaction was completed, the mixture was rotary-evaporated to dryness to remove the solvent and the crude product was purified by preparative high performance liquid chromatography (column: Phenomenex luna C18 80*40 mm*3 µm; Mobile phase A: water (0.04% hydrochloric acid), mobile phase B: acetonitrile; running gradient: % acetonitrile: 10%-30%, running time: 8 min) to give compound **032** hydrochloride. LCMS:(ESI)m/z = 582.3[M+H]⁺.

### Example 33

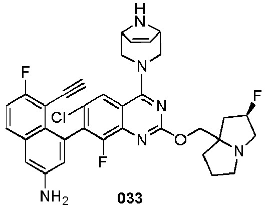

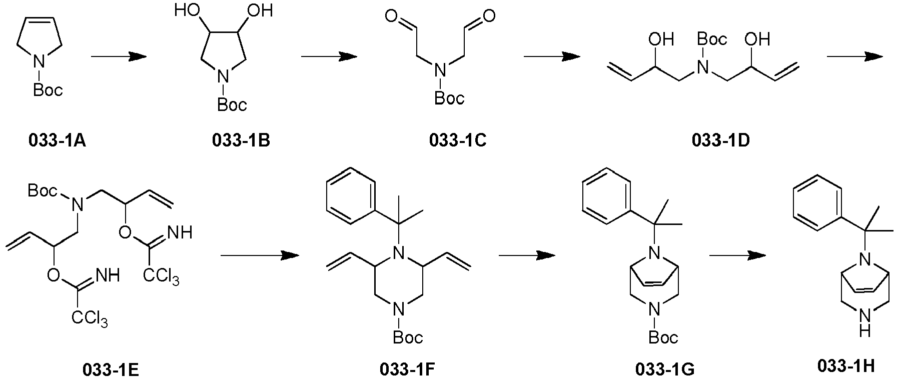

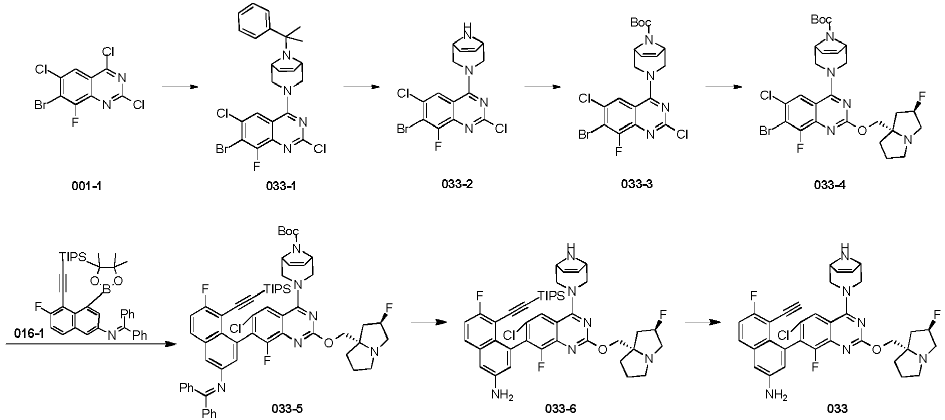

### Step 1: Synthesis of compound 033-1B

Compound **033-1A** (120 g, 709 mmol, 1.00 *eq*) was dissolved in tert-butanol (1200 mL) and water (1200 mL), and then potassium osmate (10.4 g, 28.3 mmol, 0.04 *eq*) and N-methylmorpholine oxide (249 g, 2.13 mol, 224 mL, 3.00 *eq*) were added. The mixture was stirred at 45 °C for 16 h. After the reaction was completed, the reaction solution was concentrated under reduced pressure, and extracted with ethyl acetate and aqueous sodium sulfite solution (1000 mL). The organic phase was washed three times with 500 mLof saturated brine, dried over anhydrous sodium sulfate, and filtered. The organic phase was concentrated, and the crude product was purified by column chromatography (eluent: petroleum ether/ethyl acetate = 1/0 to 0/1) to give compound **033-1B.** ¹H NMR: (400 MHz, CDCl₃) δ = 4.23 (t, *J* = 3.6 Hz, 2H), 3.55-3.58 (m, 2H), 3.33 (d, *J* = 10.4 Hz, 2H), 2.85 (s, 2H), 1.45 (s, 9H).

### Step 2: Synthesis of compound 033-1C

Compound **033-1B** (107 g, 526 mmol, 1.00 *eq*) was dissolved in dichloromethane and iodobenzene diacetate (254 g, 789 mmol, 1.50 *eq*) was added at 0 °C. The mixture was stirred at 25 °C for 3 h. After the reaction was completed, 500 mL of aqueous sodium bicarbonate and 100 mL of dichloromethane were added to the reaction solution, and the mixture was stirred for 0.5 h. The organic phases were combined, dried with anhydrous sodium sulfate and filtered. The filtrate was concentrated to give compound **033-1C.** ¹H NMR: (400 MHz, CDCl₃) δ = 9.73 (d, J = 6.8 Hz, 2H), 4.28 (s, 2H), 4.06 (s, 2H), 1.29 (s, 9H).

### Step 3: Synthesis of compound 033-1D

Compound **033-1C** (200 g) was dissolved in tetrahydrofuran (600 mL) and vinylmagnesium bromide (1 M, 1.79 L, 6.00 *eq*) was added dropwise at -78 °C. The mixture was stirred at 25 °C for 16 h. After the reaction was completed, the mixture was quenched with 1000 mL of saturated ammonium chloride at 10 °C, and extracted with 500 mL ethyl acetate. The organic phase was then extracted with saturated brine (500 mL * 3), dried over anhydrous sodium sulfate, and filtered. The filtrate was concentrated, and the crude product was purified by column chromatography (eluent: petroleum ether/ethyl acetate = 1/0 to 0/1) to give compound **033-1D.** ¹H NMR: (400 MHz, CDCl₃) δ = 5.82-5.89 (m, 2H), 5.32 (t, J = 11.6 Hz, 2H), 5.16-5.19 (m, 2H), 4.45 (s, 2H), 3.60-3.70 (m, 1H), 3.37 (s, 2H), 3.25 (s, 1H), 2.95 (d, J = 8.8 Hz, 1H), 1.48 (s, 9H).

### Step 4: Synthesis of compound 033-1E

Compound **033-1D** (80.0 g, 310 mmol, 1.00 *eq*) was dissolved in dichloromethane (1000 mL), and diazabicycle (23.6 g, 155 mmol, 23.4 mL, 0.50 *eq*) and trichloroacetonitrile (269 g, 1.87 mol, 187 mL, 6.00 *eq*) were added at 0 °C. The mixture was stirred at 25 °C for 16 h. After the reaction was completed, the mixture was filtered and the filtrate was concentrated to give a crude product. The crude product was purified by column chromatography (eluent: petroleum ether/ethyl acetate = 5/1) to give compound **033-1E.** ¹H NMR: (400 MHz, CDCl₃) δ = 8.37 (s, 2H), 5.81-5.87 (m, 2H), 5.45 (s, 2H), 5.39-5.43 (m, 2H), 5.25-5.30 (m, 2H), 3.61-3.81 (m, 4H), 1.48 (s, 9H).

### Step 5: Synthesis of compound 033-1F

Compound α,α-dimethylbenzylamine (32.1 g, 238 mmol, 1.30 *eq*) was dissolved, and then chloro(1,5-cyclooctadiene)iridium(I) dimer (12.3 g, 18.3 mmol, 0.10 *eq*) was added. Compound **033-1E** was dissolved in 1,2-dichloroethane (1.00 L), and then added dropwise to the reaction solution at 0 °C. The mixture was stirred at 25 °C for 16 h. After the reaction was completed, the mixture was filtered, and the filtrate was concentrated to give a crude product. The crude product was purified by column chromatography (eluent: petroleum ether/ethyl acetate = 10:1) to give compound **033-1F.** ¹H NMR: (400 MHz, CDCl₃) δ = 7.52-7.55 (m, 2H), 7.30 (t, J = 7.2 Hz, 2H), 7.22 (t, J = 7.2 Hz, 1H), 5.94-6.03 (m, 2H), 5.10 (t, J = 19.2 Hz, 2H), 4.99 (d, J = 10.4 Hz, 2H), 3.51-3.61 (m, 4H), 3.33 (t, J = 13.6 Hz, 2H), 1.48 (s, 15H).

### Step 6: Synthesis of compound 033-1G

Compound **033-1F** (36.0 g, 50.4 mmol, 1.00 *eq*) was dissolved in toluene (900 mL) and 1,3-bis(2,4,6-trimethylphenyl)-2-(imidazolidinylidene)(dichlorophenylmethylene)(tricyclohexylphosphine)ruthenium (2.14 g, 2.52 mmol, 0.05 *eq*) was added. The mixture was stirred at 125 °C for 16 h. After the reaction was completed, the mixture was filtered, and the filtrate was concentrated to give a crude product. The crude product was purified by column chromatography (eluent: petroleum ether/ethyl acetate = 10:1) to give compound **033-1G.** LCMS:(ESI)m/z = 329.2 [M+H]⁺. ¹H NMR: (400 MHz, CDCl₃) δ = 7.60 (t, *J* = 1.2 Hz, 2H), 7.31 (t, *J* = 7.2 Hz, 2H), 7.22 (s, 1H), 5.96 (t, *J* = 9.2 Hz, 2H), 3.60-3.65 (m, 2H), 3.46-3.53 (m, 2H), 3.09-3.14 (m, 2H), 1.42 (s, 9H), 1.25 (d, *J* = 6.0 Hz, 6H).

### Step 7: Synthesis of compound 033-1H

Compound **033-1G** (26.8 g, 81.6 mmol, 1.00 *eq*) was dissolved in methanol (201 mL) and hydrochloric acid/methanol (4 M, 67.3 mL, 3.30 *eq*) was added. The mixture was reacted at 35 °C for 16 h. After the reaction was completed, the mixture was adjusted to pH 12, and extracted with 30 mL of ethyl acetate. The organic phase was dried over anhydrous sodium sulfate, and filtered. The filtrate was concentrated to give compound **033-1H.** LCMS:(ESI)m/z = 229.2[M+H]⁺; ¹H NMR: (400 MHz, CDCl₃) δ = 7.62 (t, *J=* 7.2 Hz, 2H), 7.31 (t, *J=* 7.6 Hz, 2H), 7.21 (s, 1H), 6.01 (s, 2H), 3.42 (s, 2H), 2.89-2.93 (m, 2H), 2.30-2.34 (m, 2H), 1.23 (s, 6H).

### Step 8: Synthesis of compound 033-1

Compound **001-1** (138.23 mg, 605.38 µmol, 1 *eq*) and **033-1H** (0.2 g, 605.38 µmol, 1 *eq*) was dissolved in N,N-dimethylformamide (2 mL), and N,N-diisopropylethylamine (234.72 mg, 1.82 mmol, 316.34 µL, 3 *eq*) was added. The mixture was stirred to react at 25°C for 2 h. After the reaction was completed, 10 mL of water was added, and a solid was precipitated. The solid was filtered to give compound **033-1.** LCMS:(ESI)m/z = 521.02 [M+H]⁺; ¹H NMR (400 MHz, CDCl₃) δ ppm 1.23 (s, 6 H) 3.58 - 3.74 (m, 4 H) 4.22 (br d, J = 11.04 Hz, 2 H) 5.91 (s, 2 H) 7.16 - 7.23 (m, 2 H) 7.26 - 7.34 (m, 2 H) 7.54 (s, 1 H) 7.69 (d, J = 2.01 Hz, 1 H).

### Step 9: Synthesis of compound 033-2 trifluoroacetate

Compound **033-1** (300 mg, 574.45 µmol, 1 *eq*) was dissolved in trifluoroacetic acid (2 mL) and the mixture was stirred at 75°C for 1 h. After the reaction was completed, the mixture was concentrated to give compound **033-2** trifluoroacetate. LCMS:(ESI)m/z = 402.92 [M+H]⁺.

### Step 10: Synthesis of compound 033-3

Compound **033-2** trifluoroacetate (220 mg, 544.47 µmol, 1 eq) was dissolved in tetrahydrofuran (2 mL), and then di-tert-butyl dicarbonate (142.60 mg, 653.36 µmol, 150.10 µL, 1.2 eq) and triethylamine (165.28 mg, 1.63 mmol, 227.35 µL, 3 eq) were added. The mixture was reacted at 25°C for 2 h. After the reaction was completed, the mixture was concentrated directly. The crude product was purified by column chromatography (eluent: petroleum ether/ethyl acetate = 10:1-3:1) to give compound **033-3.** LCMS:(ESI)m/z = 502.92 [M+H]⁺; ¹H NMR (400 MHz, CDCl₃) δ = 1.55 (s, 9 H) 3.62 - 3.90 (m, 2 H) 4.27 - 4.52 (m, 2 H) 4.60 - 4.82 (m, 2 H) 6.19 (s, 2 H) 7.75 (d, J = 2.01 Hz, 1 H).

### Step 11: Synthesis of compound 033-4

Compound **033-3** (250 mg, 495.86 µmol, 1 *eq*) was dissolved in N,N-dimethylformamide (5 mL) and tetrahydrofuran THF (5 mL), and then triethylenediamine (5.56 mg, 49.59 µmol, 5.45 µL, 0.1 *eq*)*,* **001-2A** (94.73 mg, 595.03 µmol. 1.2 *eq*)*,* and cesium carbonate (242.34 mg, 743.78 µmol, 1.5 *eq*) were added. The mixture was stirred at 25°C for 2 h. After the reaction was completed, the mixture was concentrated and the crude product was purified by column chromatography (eluent: dichloromethane/methanol = 10:1-5:1) to give compound **033-4.** LCMS:(ESI)m/z = 626.12 [M+H]⁺; ¹H NMR (400 MHz, DMSO-*d₆*) δ ppm 1.47 (s, 9 H) 1.77 - 1.92 (m, 2 H) 1.96 - 2.16 (m, 3 H) 2.74 (s, 1 H) 2.79 - 2.90 (m, 2 H) 3.04 - 3.19 (m, 2 H) 3.66 (br d, J = 10.54 Hz, 2 H) 3.90 - 4.11 (m, 2 H) 4.30 (br s, 2 H) 4.60 (br s, 2 H) 5.15 - 5.38 (m, 1 H) 6.06 - 6.22 (m, 1 H) 6.16 (br s, 1 H) 7.92 - 8.05 (m, 1 H).

### Step 12: Synthesis of compound 033-5

Compound **033-4** (0.25, 398.78 µmol, 1 *eq*)*,* compound **016-1** (327.48 mg, 518.41 µmol, 1.3 *eq*) and potassium phosphate (253.94 mg, 1.20 mmol, 3 *eq*) were added to 1,4-dioxane and water (0.4 mL). The atmosphere was replaced with nitrogen three times and [(bis(1-adamantyl)-N-butylphosphine)-2-(2-aminobiphenyl)palladium(II) chloride (26.66 mg, 39.88 µmol, 0.1 *eq*) was added under nitrogen. The mixture was reacted at 80°C for 12 h. After the reaction was completed, 3 mL of water was added to the reaction solution, and the mixture was extracted with ethyl acetate (2 mL*2). The organic phases were combined, washed with 5 mL of saturated brine, dried over anhydrous sodium sulfate, and filtered. The filtrate was concentrated to give a crude product. The crude product was purified by column chromatography (petroleum ether:ethyl acetate = 100:0-1:2, dichloromethane:methanol= 10:1) to give compound **033-5.** LCMS:(ESI)m/z = 1051.4[M+H]⁺.

### Step 13: Synthesis of compound 033-6 hydrochloride

Compound **033-5** (0.13 g, 123.60 µmol, 1 *eq*) was added to hydrochloric acid/methanol (2 mL). The mixture was reacted at 18°C for 2 h. After the reaction was completed, the reaction solution was concentrated. The crude product was slurried with 5 mL of ethyl acetate for 1 h, and then filtered. The filter cake was rinsed with 2 mL of ethyl acetate and rotary-evaporated to dryness to give compound **033-6** hydrochloride. LCMS:(ESI)m/z = 787.3[M+H]⁺.

### Step 14: Synthesis of compound 033

Compound **033-6** (0.02 g, 24.28 µmol, 1 *eq,* HCl) was added to N,N-dimethylformamide (1 mL), and potassium carbonate (33.55 mg, 242.75 µmol, 10 *eq*) and cesium fluoride (7.37 mg, 48.55 µmol, 1.79 µL, 2 *eq*) were added. The mixture was reacted at 60°C for 15 h. The raw material disappeared and converted to product by LCMS monitoring. To the reaction solution was added 5 mL of water. The mixture was extracted with ethyl acetate (3 mL*2). The organic phases were combined, washed with saturated brine (10 mL*3), dried over anhydrous sodium sulfate, and filtered. The filtrate was concentrated to give the crude compound **033.** LCMS:(ESI)m/z = 631.1[M+H]⁺.

### Example 34

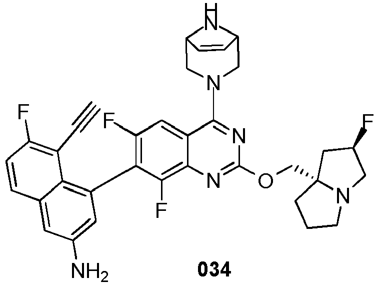

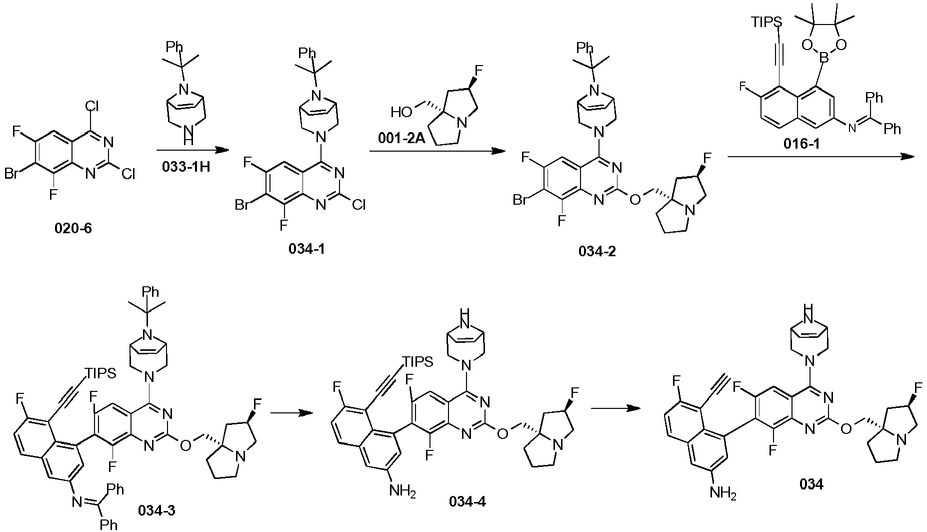

### Step 1: Synthesis of compound 034-1

Compound **020-6** (0.5 g, 1.59 mmol, 1 *eq*) was added to anhydrous dichloromethane (10 mL), and compound **033-1H** (400.05 mg, 1.75 mmol, 1.1 *eq*) and triethylamine (322.35 mg, 3.19 mmol, 443.40 µL, 2 *eq*) were added. The mixture was reacted at 18°C for 1 h. After the reaction was completed, the mixture was washed twice with 10 mL of saturated ammonium chloride and washed with 10 mL of saturated brine, dried with anhydrous sodium sulfate and filtered. The filtrate was concentrated, and the crude product was slurried with stirring for 1 h using 10 mL of solvent (petroleum ether:methyl tert-butyl ether = 3:1) and filtered. The filter cake was rinsed with 10 mL of this ratio of solvent to give compound **034-1.** LCMS:(ESI)m/z = 505.1 [M+H]⁺; ¹H NMR (400 MHz, CDCl₃) δ = 7.63 (d, *J=* 7.6 Hz, 2H), 7.44 - 7.34 (m, 3H), 7.30 - 7.25 (m, 1H), 5.99 (s, 2H), 4.29 (d, *J* = 11.2 Hz, 2H), 3.78 (s, 2H), 3.70 (d, *J=* 11.6 Hz, 2H), 1.31 (s, 6H).

### Step 2: Synthesis of compound 034-2

Compound **034-1** (0.35 g, 691.99 µmol, 1 *eq*) was added to anhydrous tetrahydrofuran (2 mL) and N,N-dimethylformamide (2 mL), and cesium carbonate (676.40 mg, 2.08 mmol, 3 *eq*), compound **001-2A** (132.20 mg, 830.39 µmol, 1.2 *eq*) and triethylenediamine (23.29 mg, 207.60 µmol, 22.83 µL, 0.3 *eq*) were added. The mixture was reacted at 25°C for 17 h. After the reaction was completed, the reaction solution was added to 10 mL of methyl tert-butyl ether, washed twice using 10 mL of saturated ammonium chloride followed by 10 mL of saturated brine, dried with anhydrous sodium sulfate and filtered. The filtrate was concentrated to give compound **034-2.** LCMS:(ESI)m/z = 628.0[M+H]⁺; ¹H NMR (400 MHz, CDCl₃) δ = 7.65-7.60 (m, 2H), 7.40 - 7.32 (m, 3H), 7.30 - 7.28 (m, 1H), 5.97 - 5.93 (m, 2H), 5.40 - 5.15 (m, 1H), 4.34 - 4.22 (m, 2H), 4.16 (d, *J* = 10.0 Hz, 1H), 4.02 (d, *J* = 10.4 Hz, 1H), 3.75-3.71 (m, 2H), 3.69-3.60 (m, 2H), 3.30 - 3.17 (m, 2H), 3.15 - 3.08 (m, 1H), 3.01 - 2.92 (m, 1H), 2.33 - 2.06 (m, 3H), 1.98 - 1.80 (m, 3H), 1.30 (s, 6H).

### Step 3: Synthesis of compound 034-3

Compound **034-2** (0.3 g, 477.31 µmol, 1 *eq*)*,* compound **016-1** (452.27 mg, 715.96 µmol, 1.5 *eq*) and potassium phosphate (303.95 mg, 1.43 mmol, 3 *eq*) were added to toluene (3 mL) and water (0.6 mL). The atmosphere was replaced with nitrogen three times and [(bis(1-adamantyl)-N-butylphosphine)-2-(2-aminobiphenyl)palladium(II) chloride (31.91 mg, 47.73 µmol, 0.1 *eq*) was added under nitrogen. The mixture was reacted at 80°C for 4.5 h. A small amount of raw material remained and a product signal was present by LCMS monitoring. 20 mL of water was added to the reaction solution. The mixture was extracted with ethyl acetate (20 mL*2). The organic phases were combined, washed with 30 mL of saturated brine, dried with anhydrous sodium sulfate and filtered. The filtrate was concentrated, and the crude product was purified by column (petroleum ether : ethyl acetate = 1:0-0:1, dichloromethane : methanol = 10:1) to give compound **034-3.** LCMS:(ESI)m/z = 1053.5[M+H]⁺; ¹H NMR (400 MHz, CDCl₃) δ = 7.81 - 7.76 (m, 2H), 7.67 - 7.62 (m, 3H), 7.52 - 7.47 (m, 1H), 7.46 - 7.31 (m, 5H), 7.30-7.28 (m, 1H),7.26 - 7.22 (m, 3H), 7.21 - 7.17 (m, 2H), 7.15 - 7.10 (m, 2H), 6.96 - 6.90 (m, 1H), 6.12 - 6.06 (m, 1H), 5.98-5.94 (m, 1H), 5.36 - 5.18 (m, 1H), 4.65-4.58 (m, 1H), 4.22 - 4.08 (m, 1H), 4.06 - 3.97 (m, 1H), 3.94 - 3.87 (m, 1H), 3.85 - 3.77 (m, 2H), 3.70-3.68 (m, 1H), 3.50-3.43 (m, 1H), 3.30 - 3.13 (m, 3H), 3.02 - 2.92 (m, 1H), 2.33 - 2.13 (m, 3H), 1.94 - 1.85 (m, 3H), 1.25 (s, 6H), 0.93 - 0.81 (m, 18H), 0.59-0.51 (m, 3H).

### Step 4: Synthesis of compound 034-4

Compound **034-3** (0.23 g, 218.35 µmol, 1 *eq*) was added to trifluoroacetic acid (2 mL). The mixture was heated to 70°C and reacted for 0.5 h. After the reaction was completed, the reaction solution was concentrated and diluted with 5 mL of ethyl acetate. The mixture was washed twice with 5 mL of water, washed with 10 mL of saturated sodium bicarbonate solution, and washed with 10 mL of saturated brine. The organic phase was dried over anhydrous sodium sulfate and filtered. The filtrate was concentrated to give compound **034-4.** LCMS:(ESI)m/z = 771.3[M+H]⁺.

### Step 5: Synthesis of compound 034

Compound **034-4** (0.27 g, 350.21 µmol, 1 *eq*) was added to N,N-dimethylformamide (3 mL), and potassium carbonate (242.01 mg, 1.75 mmol, 5 *eq*) and cesium fluoride (106.39 mg, 700.41 µmol, 25.82 µL, 2 *eq*) were added. The mixture was reacted at 60°C for 5 h. After the reaction was completed, the reaction solution was diluted with 10 mL of ethyl acetate and washed with 10 mL of water. The aqueous phase was further extracted with 5 mL of ethyl acetate. The organic phases were combined, washed 3 times with 10 mL of saturated brine, dried with anhydrous sodium sulfate and filtered. The filtrate was concentrated to give a crude product. The crude product was slurried with stirring with a mixed solvent (n-heptane:ethyl acetate = 5:1) for 1hr, and filtered. The filter cake was washed three times with 3 mL of the same ratio of the mixed solvent, rotary-evaporated to dryness and purified by high performance liquid chromatography (column: Phenomenex Luna 80*30mm*3µm; mobile phase A: water (0.04% hydrochloric acid), mobile phase B: acetonitrile; running gradient: acetonitrile%: 10%-35%, 8min). The fractions were adjusted to pH 8 with 10 mL of saturated sodium bicarbonate solution, and the mixture was extracted with ethyl acetate (20 mL*2). The organic phases were combined, washed with 20 mL of saturated brine, dried with anhydrous sodium sulfate and filtered. The filtrate was concentrated to give compound **034.** LCMS:(ESI)m/z = 615.2[M+H]⁺.

### Example 35

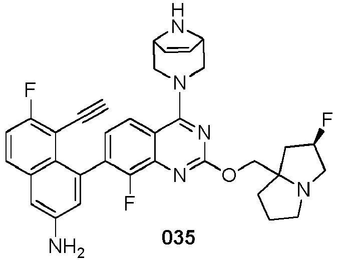

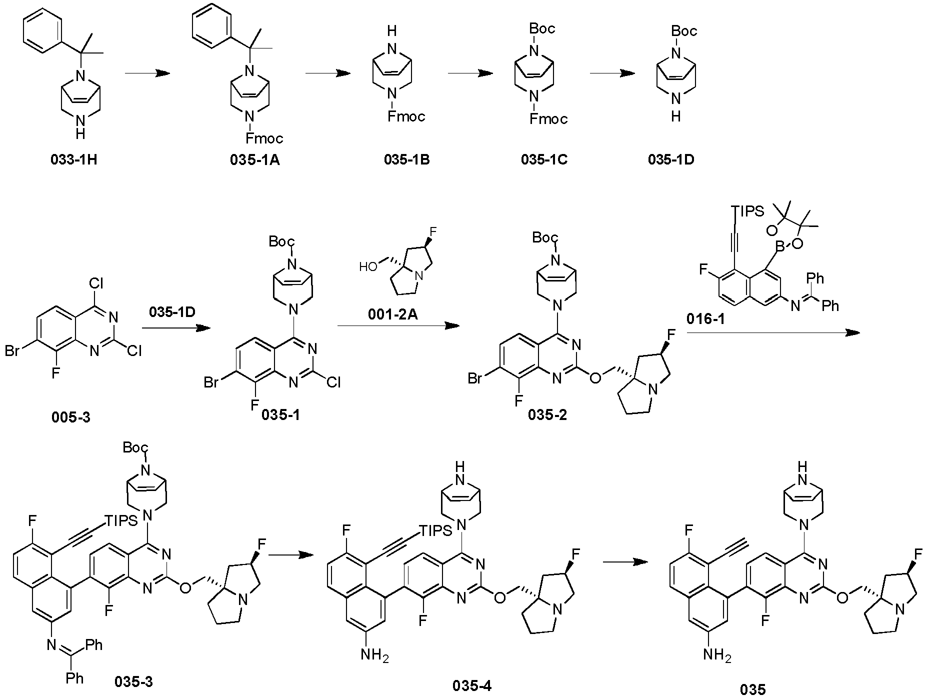

### Step 1: Synthesis of compound 035-1A

Compound **033-1H** (18.6 g) was dissolved in tetrahydrofuran (190 mL), and then 9-fluorenylmethyl chloroformate (20.5 g, 79.4 mmol, 1.00 *eq*)*,* and sodium carbonate (25.2 g, 238.2 mmol, 3.00 *eq*) were added. The mixture was stirred at 0 °C for 1 h. After the reaction was completed, 100 mL of ethyl acetate and 200 mL of water were added to the reaction solution for extraction. The organic phase was washed with saturated brine, dried over anhydrous sodium sulfate, and filtered. The filtrate was concentrated to give compound **035-1A.** LCMS:(ESI)m/z = 451.3 [M+H]⁺; ¹H NMR: (400 MHz, CDCl₃) δ = 7.76 (d, *J=* 13.6 Hz, 2H), 7.54-7.61 (m, 4H), 7.24-7.40 (m, 7H), 5.93-6.01 (m, 2H), 4.34-4.40 (m, 2H), 4.21 (s, 1H), 3.70 (t, *J=* 2 Hz, 2H), 3.55-3.59 (m, 2H), 3.15-3.23 (m, 2H), 1.27 (d, *J* = 2.4 Hz, 6H).

### Step 2: Synthesis of compound 035-1B

Compound **035-1A** (9.52 g, 21.1 mmol, 1.00 *eq*) was dissolved in trifluoroacetic acid (192 mL) and the solution was stirred at 75 °C for 16 h. After the reaction was completed, 20 mL of water was added to the reaction solution and the reaction solution was adjusted to pH 9. Then the mixture was extracted with 20 mL of dichloromethane. The organic phase was dried over anhydrous sodium sulfate, and filtered. The filtrate was concentrated to give a crude product. The crude product was slurried with 6 mL of n-heptane at 25 °C for 2 h, and filtered to give compound **035-1B.** LCMS:(ESI)m/z = 333.3[M+H]⁺; ¹H NMR: (400 MHz, CDCl₃) δ = 7.77 (d, *J=* 7.6 Hz, 2H), 7.56 (d, *J=* 7.2 Hz, 2H), 7.41 (t, *J=* 7.6 Hz, 2H), 7.33 (t, *J=* 6 Hz, 2H), 6.18-6.27 (m, 2H), 4.38-4.42 (m, 2H), 4.23 (s, 1H), 3.88 (d, *J=* 2.0 Hz, 2H), 3.82 (d, *J*= 2.4 Hz, 1H), 3.72 (d, *J=* 2.0 Hz, 1H), 3.21-3.60 (m, 2H).

### Step 3: Synthesis of compound 035-1C

Compound **035-1B** (1.00 g) was dissolved in tetrahydrofuran (10.0 mL), and then di-tert-butyl dicarbonate (764 mg, 3.50 mmol, 804 µL, 1.20 *eq*)*,* and triethylamine (885 mg, 8.75 mmol, 1.22 mL, 3.00 *eq*) were added. The mixture was stirred at 25 °C for 1 h. After the reaction was completed, the mixture was extracted with 10 mL of ethyl acetate and 10 mL of water. The organic phase was washed with 15 mL of saturated brine, dried with anhydrous sodium sulfate, and concentrated. The crude product was purified by column (petroleum ether : ethyl acetate = 3:1) to give compound **035-1C.** LCMS:(ESI)m/z = 433.2[M+H]⁺.

### Step 4: Synthesis of compound 035-1D

Compound **035-1C** (5.69 g) was dissolved in ethanol (60.0 mL), and then dimethylamine (34.4 g, 251.8 mmol, 33% content) was added. The mixture was stirred at 25 °C for 3 h. After the reaction was completed, the mixture was extracted with 40 mL of ethyl acetate and 40 mL of citric acid. The aqueous phase was adjusted to pH 9, and filtered. The filtrate was extracted with 40 mL of ethyl acetate. The organic phases were combined, dried over anhydrous sodium sulfate, and filtered. The filtrate was concentrated to give compound **035-1D.** LCMS:(ESI)m/z = 211.2 [M+H]⁺; ¹H NMR: (400 MHz, CDCl₃) δ = 6.22 (d, *J* = 10 Hz, 2H), 4.40 (d, *J=* 38.8 Hz, 2H), 2.89-3.01 (m, 2H), 2.40 (d, *J=* 13.2 Hz, 2H), 1.49 (s, 9H).

### Step 5: Synthesis of compound 035-1

Compound **005-3** (0.5 g, 1.69 mmol, 1 eq) was dissolved in anhydrous dichloromethane (5 mL), and triethylamine (512.92 mg, 5.07 mmol, 705.53 µL, 3 eq) and **035-1D** (426.34 mg, 2.03 mmol, 1.2 eq) were added. The mixture was stirred at 15°C for 1 h. After the reaction was completed, the mixture was concentrated. The residue was dissolved with 20 mL of dichloromethane, washed once with 10 mL of water, dried over anhydrous sodium sulfate, and filtered. The filtrate was concentrated to give compound **035-1.** LCMS:(ESI)m/z = 468.9[M+H]⁺; ¹H NMR (400 MHz, CDCl₃) δ ppm 7.55-7.39 (m, 2H), 6.17 (s, 2H), 4.80-4.60 (m, 2H), 4.51- 4.25 (m, 2H), 3.88-3.58 (m, 2H), 1.57-1.51 (m, 9 H).

### Step 6: Synthesis of compound 035-2

Compound **035-1** (0.9 g, 1.92 mmol, 1 eq) was dissolved in N,N-dimethylformamide (9 mL) and anhydrous tetrahydrofuran (9 mL), and then compound **001-2A** (366.03 mg, 2.30 mmol, 1.2 eq), cesium carbonate (1.87 g, 5.75 mmol, 3 eq), and triethylenediamine ( 64.47 mg, 574.79 µmol, 63.21 µL, 0.3 eq) were added. The mixture was stirred at 30 °C for 12 h. After the reaction was completed, 10 mL of water and 15 mL of ethyl acetate were added to the reaction solution. The mixture was stirred for 5 min, and then left to stand. The layers were separated. The aqueous phase was extracted once with 15 mL of ethyl acetate. The organic phases were combined, washed with saturated brine (20 mL*2), dried with anhydrous sodium sulfate, and filtered. The filtrate was concentrated and purified by silica gel column chromatography (gradient elution: petroleum ether : ethyl acetate = 100:0-50:50, with 5 parts per thousand of triethylamine in ethyl acetate) to give compound **035-2.** LCMS:(ESI)m/z = 592.1[M+H]⁺. ¹H NMR (400 MHz, CDCl₃) δ ppm 7.37 (d, J = 9.2 Hz, 1H), 7.29-7.23 (m, 1H), 6.13 (s, 2H), 5.38-5.17(m, 1H), 4.76-4.56 (m, 2H), 4.45-4.25 (m, 2H), 4.24-4.04 (m, 2H), 3.81-3.53 (m, 2H), 3.30-3.11 (m, 3H), 3.02-2.92 (m, 1H), 2.32-2.09 (m, 3H),2.00-1.81 (m, 3H), 1.56-1.48 (m, 9H).

### Step 7: Synthesis of compound 035-3

To a pre-dried reaction flask were added compound **035-2** (0.8 g, 1.35 mmol, 1 eq), anhydrous tetrahydrofuran (8 mL), anhydrous potassium phosphate (859.87 mg, 4.05 mmol, 3 eq) and water (4 mL). The atmosphere was replaced with nitrogen three times, and [(n-butylbis(1-adamantyl)phosphine)-2-(2-aminobiphenyl)]palladium (III) chloride (98.34 mg, 135.03 µmol, 0.1 eq) was added. The atmosphere was replaced with nitrogen three times, and the mixture was heated to 60 °C. A solution of compound **016-1** (1.19 g, 1.89 mmol, 1.4 eq) in anhydrous tetrahydrofuran (8 mL) was added, and then the mixture was reacted at 60 °C for 3 h. After the reaction was completed, the reaction solution was cooled down to room temperature, and concentrated under reduced pressure to remove tetrahydrofuran. 20 mL of ethyl acetate was added, and the organic phase was washed with saturated brine (20 mL*3), dried over anhydrous sodium sulfate, and filtered. The filtrate was concentrated to give a crude product. The crude product was purified by silica gel column chromatography (gradient elution: petroleum ether : ethyl acetate = 100:0-50:50 with 5 parts per thousand of triethylamine in ethyl acetate) to give compound **035-3.** LCMS:(ESI)m/z = 1017.5[M+H]⁺. ¹H NMR (400 MHz, CDCl₃) δ ppm 7.83-7.71 (m, 2H), 7.65 (dd, J = 9.0, 5.8 Hz, 1H), 7.53-7.40 (m, 4H), 7.36-7.10 (m, 8H), 6.93 (t, J = 7.0 Hz, 1H), 6.89-6.86 (m, 1H), 6.25 (br s, 1 H), 6.18-6.12 (m, 1H), 5.39-5.12 (m, 1 H), 4.85-4.49 (m, 3H), 4.26-4.00 (m, 2H), 3.96- 3.68 (m, 1H), 3.66-3.38 (m, 1H), 3.32-3.11 (m, 3H), 3.05-2.90 (m, 1H), 2.36-2.14 (m, 3H), 2.00-1.80 (m, 3H), 1.59 (s, 9H), 0.90-0.76 (m, 18H), 0.59-0.43 (m, 3H).

### Step 8: Synthesis of compound 035-4

To compound **035-3** (0.8 g, 786.39 µmol, 1 eq) was added hydrochloric acid/methanol (4 M, 8.00 mL, 40.69 eq) and the mixture was stirred at 15 °C for 1 h. After the reaction was completed, the reaction solution was concentrated to dryness under reduced pressure. The residue was then slurried with stirring with a mixed solution (ethyl acetate:dichloromethane = 5:1, 20 mL) for 0.5 h. The mixture was then filtered and the filter cake was dried under vacuum to give compound **035-4.** LCMS:(ESI)m/z = 753.3[M+H]⁺.

### Step 9: Synthesis of compound 035

Compound **035-4** (0.7 g, 847.56 µmol, 1 eq) was dissolved in N,N-dimethylformamide (7 mL), and anhydrous potassium carbonate (2.34 g, 16.95 mmol, 20 eq) and cesium fluoride (643.73 mg, 4.24 mmol, 5 eq) were added. The mixture was stirred at 65°C for 4 h. After the reaction was completed, the reaction solution was cooled down to room temperature, and then filtered. The filter cake was rinsed with 20 mL of ethyl acetate. The mother liquor was washed with saturated brine (20 mL*3), dried with anhydrous sodium sulfate, and filtered. The filtrate was concentrated to give a crude product. The crude product was purified by preparative high performance liquid chromatography with the following method: column: Phenomenex Luna 80*30mm*3µm; mobile phase: [water (0.04% hydrochloric acid)-acetonitrile]; % acetonitrile: 5%-25%, 8min. The fraction was adjusted to a pH of 9 by adding ammonia dropwise, and then concentrated under reduced pressure to remove acetonitrile. The residue was extracted with ethyl acetate (50 mL*2), and concentrated under reduced pressure to give compound **035.** LCMS:(ESI)m/z = 597.2[M+H]⁺; ¹H NMR (400 MHz, CDCl₃) δ ppm 7.65 (dd, J = 9.0, 5.8 Hz, 1H), 7.52 (d, J = 9.2 Hz, 1H), 7.21 (t, J = 8.8 Hz, 1H), 7.12-7.01 (m, 2H), 6.95 (d, J = 2.4 Hz, 1H), 6.29-6.22 (m, 2H), 5.38-5.18 (m, 1H), 4.48-4.38 (m, 2H), 4.29-4.18 (m, 1H), 4.00-3.93 (m, 2H), 3.90 (s, 1H), 3.79-3.72 (m, 2H), 3.31-3.22 (m, 2H), 3.17 (s, 1 H), 3.01-2.94 (m, 1H), 2.72 (d, J = 2.0 Hz, 1H), 2.34-2.15 (m, 3H),2.03-1.84 (m, 3 H).

### Biological assay:

### Assay example 1. Assay of the activity of inhibiting KRAS^{G12D}

### 1. Purpose

Compounds that can effectively inhibit the binding of KRAS to GTP were screened by TR-FRET method.

**2. Consumables and instruments**

**Table 3: Consumables and instruments**

| Name | Suppliers | Item No. |
|---|---|---|
| HEPES (4-(2-hydroxyethyl)-1-piperazineethanesulfonic acid) pH 7.3 | Thermo Fisher Scientific | BP299-500 |
| Sodium chloride | Promega | V4221 |
| EDTA (ethylenediaminetetraacetic acid) | EMD Millipore | 324506 |
| Tween 20 | BIO-RAD | 1706531 |
| Magnesium chloride | MP Biomedicals | 191421 |
| Bodipy GDP (guanosine 5'-diphosphate, BODIPY^{™} FL 2'-(or -3')-O-(N-(2-aminoethyl)urethane), bis(triethylammonium) salt) | Yingjie | G22360 |
| GTP (guanine-5'-triphosphate) | Sigma | G8877 |
| Tb-SA (Terbium-labeled streptavidin) | Yingjie | PV3576 |
| SOS (son of sevenless) protein | | |
| KRAS^{G12D} (Kirsten rat sarcoma viral oncogene) protein | | |
| Compound plate | Labcyte | LP-0200 |
| Assay plate | Perkin Elmer | 6008269 |
| 15 ml centrifuge tube | Corning | 430791 |
| 1.5 ml centrifuge tube | Axygen | MCT-150-C |
| Dragonfly autosampler | TTP | |
| Bravo | Agilent | |
| Echo 550 | Labcyte | |
| Envision | Perkin Elmer | |

### 3. Preparation of reagent

### a. Stock reagents:

### 1) KRAS nucleotide exchange buffer

20 mL of 1000mM HEPES, 20 mL of 500mM EDTA, 10 mL of 5 M sodium chloride, 0.1 mL of 100% Tween 20, and 949.9 mL of water were weighed and formulated to 1 L of solution. The solution was sterilized by filtration and stored at 4 °C.

### 2) KRAS assay buffer

20 mL of 1000 mM HEPES, 10 mL of 1000mM magnesium chloride, 30 mL of 5 M sodium chloride, 0.05 mL of 100% Tween 20, and 939.95mL of water were weighed and formulated to 1 L of solution. The solution was sterilized by filtration and stored at 4 °C.

### 3) KRAS/Bodipy GDP/Tb-SA mixture

9.5 µL of 95 µM KRAS^{G12D} protein and 440.5 µL of KRAS nucleotide exchange buffer were weighed and mixed. The mixture was incubated at room temperature for 1 hour, and then formulated to 1 L of solution together with 8.4 µL of 17.9 µM Tb-SA, 1.8 µL of 5mM Bodipy GDP and 9539.8 µL of KRAS assay buffer. After mixing, the solution was left to stand at room temperature for 6 hours, and stored at -80 °C.

### b. Assay reagents:

### 1) KRAS kinase solution

73.3 µL of KRAS/Bodipy GDP/Tb-SA mixture and 2126.7 µL of KRAS assay buffer were weighed and formulated to 2200 µL of solution.

### 2) SOS/GTP mixture

1.59 µL of 166 µM SOS protein, 198 µL of 100 mM GTP and 2000.41 µL of KRAS assay buffer were weighed and formulated to 2200 µL of solution.

### 4. Assay process

1) The concentration of a stock solution of the control compound was 1 mM, and the concentration of a stock solution of compounds to be assayed was 10 mM. 9 µL of the control compound and compounds to be assayed were transfered to a 384-LDV plate;
2) The compounds on the LDV plate were serially diluted 3-fold with Bravo to 10 concentrations;
3) 9 nL of the compounds on the LDV plate were transfered to an assay plate using ECHO;
4) 3 µL of 3 nM Kras/0.5 nM TB-SA/30 nM BodipyGDP mixture and 3 µL of Ras buffer were sequentially added to each well of the assay plate using a Dragonfly automatic sampler, and the assay plate was centrifuged at 1000rpm/min for 1 minute;
5) The assay plate was incubated at room temperature for 1 hour;
6) 3 µL of 120 nM SOS/9 mM GTP mixture was added to each well of the assay plate using a Dragonfly automatic sampler, and the assay plate was centrifuged at 1000rpm/min for 1 minute;
7) The assay plate was incubated at room temperature for 1 hour;
8) The plate was read with Envision and data were recorded;
9) The data were analysed using Excel and Xlfit, and IC₅₀ of the compounds to be assayed were calculated.

### 5 Assay results

The results are shown in Table 4.

**Table 4. IC₅₀ values of compounds on inhibiting KRAS^{G12D} enzyme**

| **Compound No.** | **KRAS^{G12D} IC₅₀ (nM)** |
|---|---|
| Compound 001 formate | 9.2 |
| Compound 004 | 0.5 |
| Compound 005 hydrochloride | 0.1 |
| Compound 007 hydrochloride | 0.4 |
| Compound 008 hydrochloride | 1.3 |
| Compound 020 formate | 6.6 |
| Compound 027 hydrochloride | 38.6 |

Assay conclusion: The compounds of the present disclosure have significant inhibitory effect on KRAS^{G12D} enzyme.

### Assay example 2. Assay of p-ERK inhibition in AGS cells

### 1. Purpose

Compounds that can effectively inhibit p-ERK in AGS cells were screened out by a HTRF method.

### 2. Assay process

1) AGS cells were inoculated in a transparent 96-well cell culture plate, and each well contained 80µL of cell suspension and 10000 cells. The cell plate was inoculated in a carbon dioxide incubator at 37 °C overnight;
2) After completion of the incubation, the cell supernatant was discarded. 80 µL of medium containing 0.02% serum was added to each well. The cell plate was incubated in a carbon dioxide incubator at 37 °C overnight;
3) 2 µL of the compound was weighed and added to 78 µL of the cell medium. After the mixture was mixed thoroughly, 20 µL of the solution of compound was weighed and added to the corresponding well of the cell plate. The cell plate was placed back to the carbon dioxide incubator and incubated for another 3 hours;
4) After completion of the incubation, the cell supernatant was discarded. 50 µL of 1X cell lysate was added to each well. The mixture was incubated with shaking at room temperature for 30 minutes;
5) Phospho-ERK1/2 Eu Cryptate antibody and Phospho-ERK1/2 d2 antibody were diluted 20-fold with detection buffer;
6) 16 µL of cell lysate supernatant was weighed and added into each well of a new 384 white microwell plate, and then 2 µL of the diluted solution of Phospho-ERK1/2 Eu Cryptate antibody and 2 µL of the diluted solution of Phospho-ERK1/2 d2 antibody dilution were added. The mixture was incubated at room temperature for at least 4 hours;
7) After completion of the incubation, HTRF was read with multi-label analyzer: excitation: 320nm, emission: 615nm, 665nm;
8). IC₅₀ of the compounds to be assayed was calculated.

### 3. Assay results

The results are shown in Table 5.

**Table 5. IC₅₀ value of compounds on inhibiting p-ERK in AGS cells**

| **Compound No.** | **AGS p-ERK IC₅₀ (nM)** |
|---|---|
| Compound 001 formate | 291.8 |
| Compound 004 | 31.4 |
| Compound 005 hydrochloride | 147.3 |
| Compound 006 hydrochloride | 67.1 |

Assay conclusion: The compounds of the present disclosure have significant inhibitory effect on p-ERK in AGS cells.

### Assay example 3. Assay of p-ERK inhibition in GP2D cells

### 1. Purpose

Compounds that can effectively inhibit p-ERK in GP2D cells were screened out by a HTRF method.

### 2. Assay process

1) GP2D cells were inoculated in a transparent 96-well cell culture plate, and each well contained 80µL of cell suspension and 8000 cells. The cell plate was inoculated in a carbon dioxide incubator at 37 °C overnight;
2) 2 µL of the compound was weighed and added to 78 µL of the cell medium. After the mixture was mixed thoroughly, 20 µL of the solution of compound was weighed and added to the corresponding well of the cell plate. The cell plate was placed back to the carbon dioxide incubator and incubated for another 1 hour;
3) After completion of the incubation, the cell supernatant was discarded. 50 µL of 1X cell lysate was added to each well. The mixture was incubated with shaking at room temperature for 30 minutes;
4) Phospho-ERK1/2 Eu Cryptate antibody and Phospho-ERK1/2 d2 antibody were diluted 20-fold with detection buffer;
5) 16 µL of cell lysate supernatant was weighed and added into each well of a new 384 white microwell plate, and then 2 µL of the diluted solution of Phospho-ERK1/2 Eu Cryptate antibody and 2 µL of the diluted solution of Phospho-ERK1/2 d2 antibody dilution were added. The mixture was incubated at room temperature for at least 4 hours;
6) After completion of the incubation, HTRF was read with multi-label analyzer: excitation: 320nm, emission: 615nm, 665nm;
7). IC₅₀ of the compounds to be assayed was calculated.

### 3. Assay results

The results are shown in Table 6.

**Table 6. IC₅₀ values of compounds on inhibiting p-ERK in GP2D cells**

| **Compound No.** | **GP2D p-ERK IC₅₀ (nM)** |
|---|---|
| Compound 010 hydrochloride | 0.055 |
| Compound 011 hydrochloride | 0.016 |
| Compound 012 formate | 0.71 |
| Compound 013 hydrochloride | 0.88 |
| Compound 014 | 0.43 |
| Compound 016B | 0.67 |
| Compound 018 trifluoroacetate | 1.6 |
| Compound 019 hydrochloride | 21.4 |
| Compound 022 formate | 11.6 |
| Compound 024 formate | 4.4 |

Assay conclusion: The compounds of the present disclosure have significant inhibitory effect on p-ERK in GP2D cells.

### Assay example 4. Assay of inhibition of GP2D cell proliferation

### 1. Purpose of Assay:

The aim of this assay was to verify the inhibitory effect of the compounds of the present disclosure on the proliferation of KRAS G12D mutated GP2D human pancreatic cancer cells.

### 2. Assay material:

Cell line GP2D, DMEM medium, and penicillin/streptomycin antibiotics were purchased from Wisent; fetal bovine serum was purchased from Biosera; and CellTiter-Glo^{®} 3D Cell Viability Assay (3D cell viability chemiluminescence assay reagent) reagent was purchased from Promega.

### 3. Assay methods:

GP2D cells were seeded in a 96-well U-bottom cell culture plate. Each well contained 80 µL of cell suspension containing 2000 GP2D cells. The cell plate was incubated overnight in a CO₂ incubator. The compound to be assayed was serially diluted 5-fold with a pipette to the 8th concentration, i.e. diluted from 200 µM to 2.56 nM, to set up a double replicate well assay. 78 µL of medium was added to the middle plate, and then 2 µL per well of the gradient dilution compound was transferred to the middle plate according to the corresponding position. The mixture was mixed well and 20 µL per well was transferred to the cell plate. The concentration of compound transferred to the cell plate ranged from 1 µM to 0.0128 nM. The cell plate was incubated in a CO₂ incubator for 5 days. At the end of the incubation of the plate with compounds added, 100 µL of cell viability chemiluminescence detection reagent per well was added to the plate and the plate was incubated at room temperature for 10 minutes to stabilize the luminescence signal. Reading was performed using a multi-label analyzer.

### 4. Data analysis:

The raw data were converted into an inhibition rate by using an equation (Sample-Min)/(Max-Min)*100%. IC50 value can be obtained by curve fitting with four parameters ("log(inhibitor) vs. response -- Variable slope" model in GraphPad Prism).

### 5. Assay results

The results are shown in Table 7.

**Table 7. IC₅₀ value of compounds on the inhibition of GP2D cell proliferation**

| **Compound No.** | **GP2D IC₅₀ (nM)** |
|---|---|
| Compound 006 hydrochloride | 2.04 |
| Compound 010 hydrochloride | 3.6 |
| Compound 011 hydrochloride | 6.2 |
| Compound 012 formate | 0.94 |
| Compound 024 formate | 16 |

Assay conclusion: The compounds of the present disclosure have a significant inhibitory effect on the proliferation of GP2D cells.

### Assay example 5: Assay of SOS1-mediated binding ability of KRAS^{G12D} to effector protein c-Raf

### 1. Purpose

Compounds that can effectively inhibit the binding of KRAS^{G12D} to c-Raf, a downstream effector protein of the MAPK pathway, were screened by a TR-FRET method.

### 2. Assay procedure

### 2.1 Preparation of 1× enzyme reaction buffer:

**Table 8: Reaction buffers**

| **1×buffer** | **Stock concentration** | **Dilution folds** | **Assay concentration.** |
|---|---|---|---|
| Hepes pH7.4 | 1M | 40 | 25 mM |
| NaCl₂ | 5 M | 40 | 125 mM |
| MgCl₂ | 1M | 200 | 5 mM |
| Tween20 | 1% | 100 | 0.01% |
| BSA | 7.50% | 75 | 0.10% |
| DTT | 1M | 1000 | 1 mM |
| H₂O | / | | |
| Total | / | | |

### 2.2 Preparation of KRAS G12D enzyme (2×):

**Table 9 KRAS G12D enzyme**

| **KRAS^{G12D} Solution** | | | |
|---|---|---|---|
| **Reagent** | **Stock concentration** | **2Assay concentration** | **Assay concentration** |
| KRAS G12D | 41.667 µM | 200 nM | 100 nM |
| 1× Assay buffer | / | / | / |

### 2.3 Preparation of substrate and antibody mixture (2×):

**Table 10. Substrate and antibody mixture**

| **2× c-Raf/SOS1/GTP/ MAb Anti 6HIS-d2/ MAb Anti GST-Eu** | | | |
|---|---|---|---|
| **Reagent** | **Stock concentration** | **2×assay concentration** | **Assay concentration** |
| SOS1 | 14 µM | 50 nM | 25 nM |
| c-Raf | 13 µM | 50 nM | 25 nM |
| GTP | 50 mM | 100 µm | 50 µM |
| MAb Anti 6HIS-d2 | 200× | / | / |
| MAb Anti GST-Eu cryptate | 200× | / | / |
| 1× Detection buffer | / | | |

### 3. Compound screening:

1) The compounds were serially diluted 5-fold with DMSO in a dilution plate, wherein the final starting concentration of compounds was 10 µM.
2) The compounds were transferred with Echo to a 384 reaction plate with 100 µL per well.
3) 5 µL of KRAS G12D enzyme was added to each well of the reaction plate.
4) The plates were sealed with a sealing film and centrifuged at 1000g for 30 seconds, and then incubated at room temperature for 15 minutes.
5) 2x cRaf/SOS 1/GTP/MAb Anti 6HIS-d2/MAb Anti GST-Eu mixture was prepared with 1X enzyme reaction buffer and 5 µL of the mixture was added to the reaction plate.
6) The mixture was centrifuged at 1000g for 30 seconds and reacted at room temperature for 2 hours.
7) The fluorescence signals at 615 nm (Cryptate) and 665 nm (XL665) were read with a BMG microplate reader.
8) Data analysis was performed using Graphpad 7.0 software to give IC₅₀.

### 4. Assay results

The results are shown in Table 11:

**Table 11. IC₅₀ values of compounds for KRAS^{G12D} activity**

| **Compound No.** | **IC₅₀ (nM)** |
|---|---|
| Compound 014 | 11.97 |
| Compound 016B | 6.23 |
| Compound 034 | 22.49 |

Assay conclusion: The compounds of the present disclosure can significantly inhibit KRAS^{G12D} activity.

### Assay example 6: Assay of plasma protein binding (PPB)

Assay procedure: 995 µL of blank plasma of various genera were weighed, and 5 µL of assay compound working solution (400 µM) or warfarin working solution (400 µM) was added so that the final concentrations of the assay compound and warfarin in plasma sample were each 2 µM. The samples were mixed thoroughly. The final concentration of DMSO (the organic phase) was 0.5%. 50 µL of the plasma samples of the assay compound and warfarin were pipetted to a sample receiving plate (in triplicate), and the corresponding volume of corresponding blank plasma or buffer was added immediately, so that the final volume in each sample well was 100 µL, wherein the volume ratio of plasma:dialysis buffer was 1: 1. 500 µL of stop solution was then added to these samples. These samples were used as T₀ samples for determination of recovery and stability. The T₀ samples were stored at 2-8°C, waiting for subsequent processing together with other dialyzed samples. 150 µL of the plasma samples of the assay compound and warfarin were added to the dosing side of each dialysis well, and 150 µL of blank dialysis buffer was added to the corresponding receiving side of the dialysis well. The dialysis plate was then placed in a wet 5% CO₂ incubator, and incubated with shaking at about 100 rpm at 37°C for 4 hr. After the dialysis was over, 50 µL of dialyzed buffer sample and dialyzed plasma sample were pipetted to a new sample receiving plate. The corresponding volume of the corresponding blank plasma or buffer solution was added to the samples, so that the final volume in each sample well was 100 µL, wherein the volume ratio of plasma:dialysis buffer was 1:1. All samples were subjected to protein precipitation, and then analyzed by LC/MS/MS. Bound rate and recovery rate of protein were calculated using the following formulae: % *Unbound* = *100* * *F* / *T, % Bound* = *100* - % *Unbound,* % *Recovery* = *100* * (*F* + *T)* / *T₀* (wherein F is the peak area ratio of the compound in the dialysate after 4h of dialysis; *T* is the peak area ratio of the compound in the plasma after 4h of dialysis; *T₀* is the peak area ratio of the compound in the plasma sample at time zero). The assay results are shown in Table 12:

**Table. 12 Results of PPB assay**

| Compound No. | Unbound PPB H/D/C/R/M |
|---|---|
| **010** | 2.9%/2.2%/1.9%/1.3%/2.0% |
| **014** | 18.5%/3.0%/2.8%/2.0%/4.5% |

**Conclusion:** The compounds of the present disclosure have strong binding to plasma protein.

### Assay example 7: Pharmacokinetic evaluation of compounds in mice

Purpose of assay: To assay the pharmacokinetic data of the compounds in CD-1 mice in vivo.

Compounds were mixed with vehicle 5% DMSO/95% (10% HP-β-CD aqueous solution), and the mixture was vortexed and sonicated to give 0.2 mg/mL-0.3 mg/mL clear solutions. CD-1 male mice aged 7 to 10 weeks were selected, and the candidate compound solution was administered intravenously. Whole blood was collected for a certain period of time to prepare the plasma. The drug concentration was analyzed by LC-MS/MS method and pharmacokinetic parameters were calculated by Phoenix WinNonlin software (Pharsight, USA). The results are shown in Table 13.

**Table 13 Intravenous (IV) PK data**

| **Assay compound** | **010** | **014** |
|---|---|---|
| Dosage (mg/kg) | 1.03 | 1.28 |
| C₀ (nM) | 712 | 953 |
| T_{1/2} (h) | 8.1 | 5.7 |
| Vd (L/kg) | 25.0 | 25.6 |
| Cl (mL/Kg/min) | 61.4 | 77.0 |
| AUC_{0-inf} (nM.h) | 457 | 565 |

Compounds were mixed with vehicle 5% DMSO/95% (10% HP-β-CD in water), and the mixture was vortexed and sonicated to give 1.6 mg/mL to 4.0 mg/mL clear solutions. CD-1 male mice aged 7 to 10 weeks were selected, and the candidate compound solution was orally administered. Whole blood was collected for a certain period of time to prepare plasma. The drug concentration was analyzed by LC-MS/MS method, and pharmacokinetic parameters were calculated by Phoenix WinNonlin software (Pharsight, USA). The results are shown in Table 14.

**Table 14 Oral (PO) PK data**

| Assay compound | **010 hydrochloride** | **014** |
|---|---|---|
| Dosage (mg/kg) | 5.54 | 16.0 |
| Cₘₐₓ (nM) | 54 | 524 |
| Tₘₐₓ | 1.5 | 2.5 |
| T_{1/2} (h) | 1.6 | 6.0 |
| AUC_{0-inf} (nM.h) | 125 | 1491 |
| AUCᵤ(nM.h) | 2.5 | 67.1 |
| F% | 5.1 | 20.5 |

| | | |
|---|---|---|
| Note: AUC_{u =} AUC_{0-inf} *Unbound PPB (Mouse) | | |

**Conclusion:** The compounds of the present disclosure have good PK properties in mice.

### Assay example 8: Pharmacokinetic evaluation of compounds in rats

Purpose of assay: To assay the pharmacokinetic data of the compounds in SD rats in vivo.

Compounds were mixed with vehicle 5% DMSO/95% (10% HP-β-CD in water), and the mixture was vortexed and sonicated to give a clear solution of 0.3 mg/mL. The candidate compound solutions were administered intravenously. Whole blood was collected for a certain period of time to prepare plasma. The drug concentration was analyzed by LC-MS/MS method, and pharmacokinetic parameters were calculated. The results are shown in Table 15.

**Table 15. Intravenous (IV) PK data**

| **Assay compound** | **014** |
|---|---|
| Dosage (mg/kg) | 1.56 |
| C₀ (nM) | 971 |
| T_{1/2} (h) | 0.56 |
| Vd (L/kg) | 5.89 |
| Cl (mL/Kg/min) | 271 |
| AUC_{0-inf} (nM.h) | 150 |

Compounds were mixed with vehicle 5% DMSO/95% (10% HP-β-CD in water), and the mixture was vortexed and sonicated to give a solution of 7 mg/mL. The candidate compound solutions were administered orally. Whole blood was collected for a certain period of time to prepare plasma. The drug concentration was analyzed by LC-MS/MS method, and pharmacokinetic parameters were calculated. The results are shown in Table 16.

**Table 16. Oral (PO) PK data**

| Assay compound | **014** |
|---|---|
| Dosage (mg/kg) | 31.7 |
| Cₘₐₓ (nM) | 182 |
| Tₘₐₓ | 2.0 |
| T_{1/2} (h) | 6.8 |
| AUC_{0-inf} (nM.h) | 427 |
| F% | 12.5 |

**Conclusion:** The compounds of the present disclosure have good PK properties in rats.

### Assay example 9: Pharmacokinetic evaluation of compounds in dogs

Purpose of assay: To assay the pharmacokinetic data of the compounds in Beagle dogs in vivo.

Compound 014 was mixed with vehicle 20% DMSO/60% PEG400/20% (10% HP-β-CD in water), and the mixture was vortexed and sonicated to give a clear solution of 4 mg/mL. The candidate compound solutions were administered intravenously. Whole blood was collected for a certain period of time to prepare plasma. The drug concentration was analyzed by LC-MS/MS method, and pharmacokinetic parameters were calculated. The results are shown in Table 17.

**Table 17. Intravenous (IV) PK data**

| **Assay compound** | **014** |
|---|---|
| Dosage (mg/kg) | 1.56 |
| C₀ (nM) | 971 |
| T_{1/2} (h) | 0.56 |
| Vd (L/kg) | 5.89 |
| Cl (mL/Kg/min) | 271 |
| AUC_{0-inf} (nM.h) | 150 |

Compound 014 was mixed (15 mg/mL) with vehicle 20% DMSO/60% PEG400/20% (10% HP-β-CD in water), and the mixture was vortexed and sonicated to give solutions of 6 mg/mL to 15 mg/mL. The candidate compound solutions were administered orally. Whole blood was collected for a certain period of time to prepare plasma. The drug concentration was analyzed by LC-MS/MS method, and pharmacokinetic parameters were calculated. The results are shown in Table 18.

**Table 18. Oral (PO) PK data**

| **Assay compound** | **014** |
|---|---|
| Dosage (mg/kg) | 32.1 |
| Cₘₐₓ (nM) | 439 |
| Tₘₐₓ | 1.0 |
| T_{1/2} (h) | 10.8 |
| AUC_{0-inf} (nM.h) | 969 |
| F% | 16.9 |

**Conclusion:** The compound of the present disclosure has good PK properties in dogs.

### Assay example 10: In vivo pharmacodynamic study

An xenograft tumor model in GP2D Balb/c nude mouse:

Assay method: A Balb/c nude mouse model of subcutaneously xenograft tumor of human colon cancer GP2D cells was established. 0.2 mL (2×10⁶) of GP2D cells (Matrigel was added, and volume ratio was 1:1) were inoculated subcutaneously on the right back of each mouse. When the average tumor volume reached 149 mm³, the administration was started by group, with 6 mice in each group. On the day of the assay, the animals were administered the corresponding drugs according to the corresponding groups. The first group G1 was set as a negative control group, which was given 5%DMSO+95%(10%HP-β-CD) alone by gavage administration. The second group G2 to the fifth group G5 were given compound **014,** and the dosage and protocol of administration were shown in Table 19.

**Table 19: Pharmacodynamic study of the assay compound on transplanted tumor of human diffuse large B lymphoma TMD8 in mouse**

| **Group** | **Number of animals** | **Assay compound** | **Dosage (mg/kg)** | **Administration concentration (mg/mL)** | **Administration Volume (mL/kg)** | **Route and frequency of administration** |
|---|---|---|---|---|---|---|
| G1 | 6 | Negative control | (N/A) | (N/A) | (N/A) | PO,BID*22 |
| G2 | 6 | Compound 014 | 25 | 2.5 | 10 | PO,BID*22 |
| G3 | 6 | Compound 014 | 50 | 5 | 10 | PO,BID*22 |
| G4 | 6 | Compound 014 | 150 | 15 | 10 | PO,BID*22 |
| G5 | 6 | Compound 014 | 150 | 15 | 10 | PO,QD*22 |

| | | | | | | |
|---|---|---|---|---|---|---|
| Note: PO means oral administration, QD means once daily, BID means once daily. | | | | | | |

During the assay, the body weight of animals and the tumor size were measured twice a week. Meanwhile, clinical symptoms of animals were observed and recorded every day. Each administration was referenced to the most recent body weight of animals.

The length (a) and width (b) of a tumor were measured with a digital vernier caliper. The calculation formula of tumor volume (TV) was: TV=a×b²/2.

### Assay results:

Compound 014 showed a significant inhibitory effect on xenograft tumors of human colon cancer GP2D in mouse. After 22 days of administration, the second group G2 (25 mg/kg, PO, BID) had a tumor volume inhibition rate TGI (%) of 84.2% at day 14; the third group G3 (50 mg/kg, PO, BID) and the fourth group G4 (150 mg/kg, PO, BID) had a tumor volume inhibition rate TGI (%) of 89.4% and 97.3%, respectively, at day 14; in addition, the fifth group G5 (150 mg/kg, PO, QD) had a tumor volume inhibition rate TGI (%) of 91.4%. The detailed results are shown in Table 20.

**Table 20: Effect of the assay compound on animal tumor size in xenograft tumor model of human colon cancer GP2D in mice**

| Group | Number of animals | Assay compound | Administration frequency | Dosage mg/kg | Tumor volume inhibition rate TGI (%) |
|---|---|---|---|---|---|
| G1 | 6 | Negative control | PO,BID*22 | NA | N/A |
| G2 | 6 | Compound 014 | PO,BID*22 | 25 | 84.2% |
| G3 | 6 | Compound 014 | PO,BID*22 | 50 | 89.4% |
| G4 | 6 | Compound 014 | PO,BID*22 | 150 | 97.3% |
| G5 | 6 | Compound 014 | PO,QD*22 | 150 | 91.4% |

| | | | | | |
|---|---|---|---|---|---|
| Note: N/A means not detected. | | | | | |

**Assay conclusion:** In the aspect of in vivo efficacy, the compound of the present disclosure has a significant inhibitory effect on tumors.

## Claims

1. A compound represented by formula (II), or a pharmaceutically acceptable salt thereof 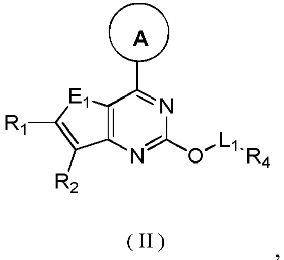 wherein
E₁ is selected from S and -CR₃ = CH-;
L₁ is selected from -CH₂- and a bond;
Ring A is selected from 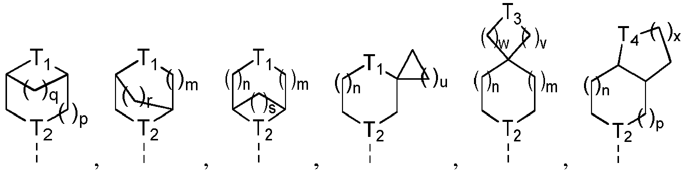 and 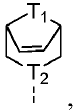 wherein the 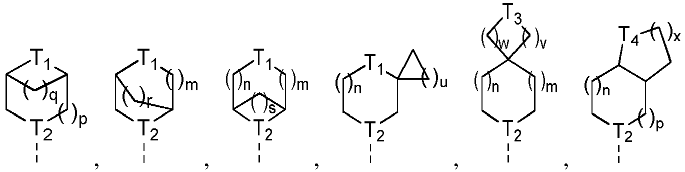 and 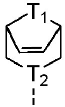 are optionally substituted with 1, 2 or 3 Rₐ;
T₁ is selected from CH₂, NH and O;
T₂ is selected from CH and N;
T₃ and T₄ are each independently selected from CH₂ and NH;
m, n, p and x are each independently selected from 0, 1 and 2;
r, v and w are each independently selected from 1 and 2;
q, s and u are each independently selected from 1, 2 and 3;
R₁ is selected from C₆₋₁₀ aryl and 5- to 10-membered heteroaryl, wherein the C₆₋₁₀ aryl and 5-to 10-membered heteroaryl are optionally substituted with 1, 2, 3, 4 or 5 R_{b};
R₂ is selected from H, F, Cl, CN, NH₂, C₁₋₃ alkyl and C₁₋₃ alkoxy, wherein the C₁₋₃ alkyl and C₁₋₃ alkoxy are optionally substituted with 1, 2 or 3 halogens;
R₃ is selected from H, F, Cl, C₁₋₃ alkyl, C₁₋₃ alkoxy, C₂₋₄ alkenyl and cyclopropyl, wherein the C₁₋₃ alkyl, C₁₋₃ alkoxy, C₂₋₄ alkenyl and cyclopropyl are optionally substituted with 1, 2 or 3 halogens;
R₄ is selected from 4- to 8-membered heterocycloalkyl and 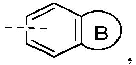 wherein the 4- to 8-membered heterocycloalkyl and 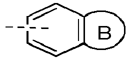 are optionally substituted with 1 2 or 3 Rₑ;
the structural moiety 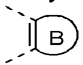 is 5- to 6-membered heterocycloalkenyl;
each Rₐ is independently selected from F, Cl, Br, I and CH₃;
each R_{b} is independently selected from F, Cl, Br, I, OH, NH₂, CN, C₁₋₃ alkyl, C₁₋₃ alkoxy, C₂₋₄ alkenyl and C₂₋₄ alkynyl, wherein the C₁₋₃ alkyl, C₁₋₃ alkoxy, C₂₋₄ alkenyl and C₂₋₄ alkynyl are optionally substituted with 1, 2 or 3 halogens;
each Rₑ is independently selected from H, F, Cl, Br, OH, CN, C₁₋₃ alkyl, C₁₋₃ alkoxy and -C₁₋₃ alkyl-O-C( = O)-C₁₋₃ alkylamino.

2. The compound of claim 1, or a pharmaceutically acceptable salt thereof, wherein Ring A is selected from 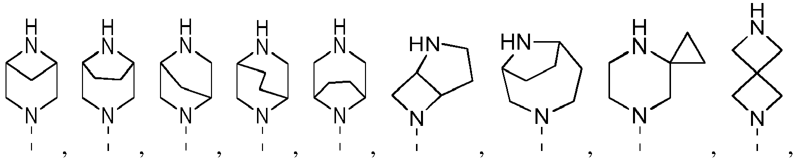 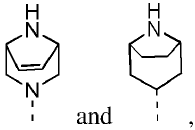 wherein the 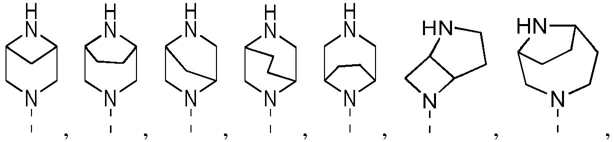 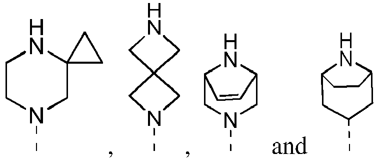 are optionally substituted with 1, 2 or 3 Rₐ.

3. The compound of claim 1 or 2, or a pharmaceutically acceptable salt thereof, wherein Ring A is selected from

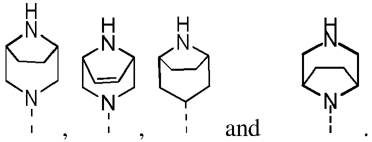

4. The compound of claim 1, or a pharmaceutically acceptable salt thereof, wherein each R_{b} is independently selected from F, Cl, Br, I, OH, NH₂, CN, CH₃, CH₂CH₃, OCH₃, OCH₂CH₃, - CH = CH₂, -CH₂-CH = CH₂ and -C≡CH, wherein the CH₃, CH₂CH₃, OCH₃, OCH₂CH₃, -CH = CH₂, -CH₂-CH = CH₂ and -C≡CH are optionally substituted with 1, 2 or 3 halogens.

5. The compound of claim 4, or a pharmaceutically acceptable salt thereof, wherein each R_{b} is independently selected from F, Cl, OH, NH₂, CN, CH₃, CF₃, CH₂CH₃ and -C≡CH.

6. The compound of claim 1, or a pharmaceutically acceptable salt thereof, wherein R₁ is selected from phenyl, pyridyl, naphthyl, quinolyl, benzothiazolyl and benzothienyl, wherein the phenyl, pyridyl, naphthyl, quinolyl, benzothiazolyl and benzothienyl are optionally substituted with 1, 2, 3, 4 or 5 R_{b}.

7. The compound of any one of claims 1, 4, 5 and 6, or a pharmaceutically acceptable salt thereof, wherein R₁ is selected from

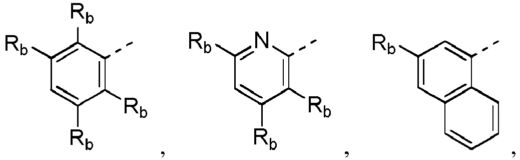

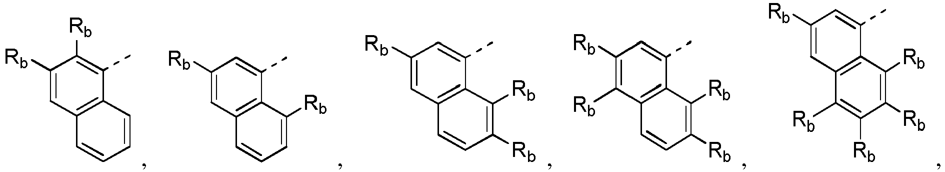

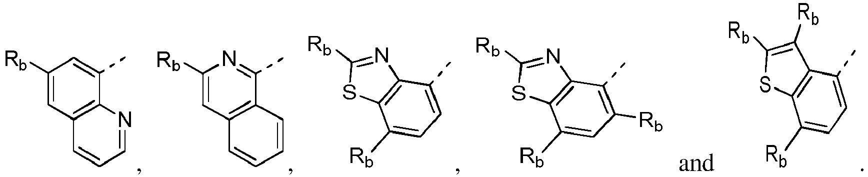

8. The compound of claim 7, or a pharmaceutically acceptable salt thereof, wherein R₁ is selected from

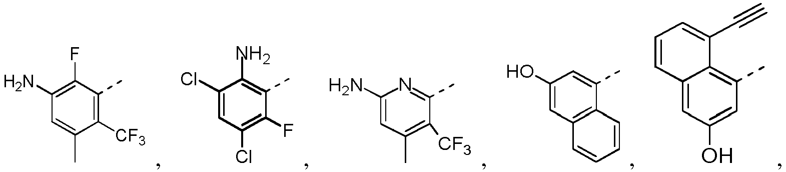

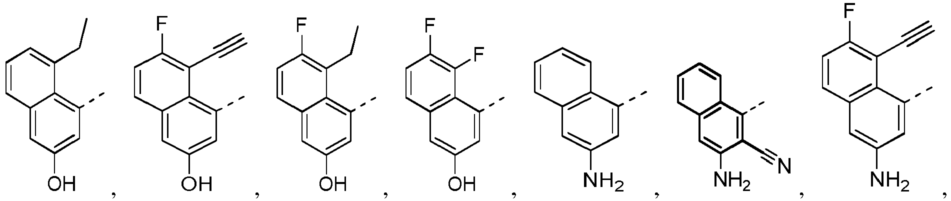

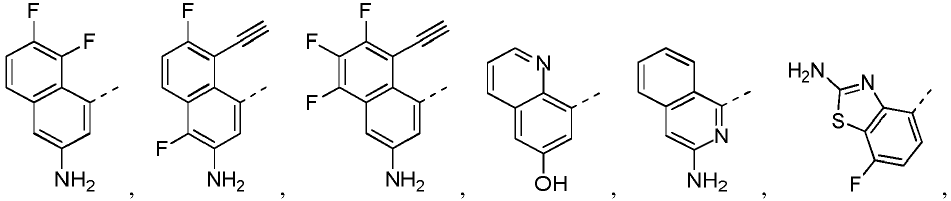

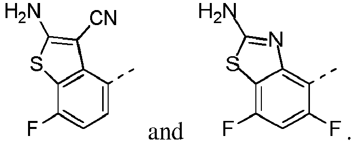

9. The compound of claim 1, or a pharmaceutically acceptable salt thereof, wherein R₂ is selected from H, F, Cl, CH₃ and OCH₃, wherein the CH₃ and OCH₃ are optionally substituted with 1, 2 or 3 halogens.

10. The compound of claim 1 or 9, or a pharmaceutically acceptable salt thereof, wherein R₂ is selected from H, F, Cl, OCH₃ and OCHF₂.

11. The compound of claim 1, or a pharmaceutically acceptable salt thereof, wherein R₃ is selected from H, F, Cl, CH₃, OCH₃, -CH = CH₂ and cyclopropyl, wherein the CH₃, OCH₃, -CH = CH₂ and cyclopropyl are optionally substituted with 1, 2 or 3 halogens.

12. The compound of claim 1 or 11, or a pharmaceutically acceptable salt thereof, wherein R₃ is selected from H, F, Cl, OCHF₂, -CH = CH₂ and cyclopropyl.

13. The compound of claim 1, or a pharmaceutically acceptable salt thereof, wherein each Rₑ is independently selected from H, F, Cl, Br, OH, CN, CH₃, CH₂CH₃, OCH₃ and

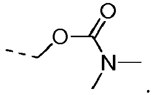

14. The compound of claim 1, or a pharmaceutically acceptable salt thereof, wherein R₄ is selected from tetrahydropyrrolyl and hexahydro-1H-pyrrolizinyl, wherein the tetrahydropyrrolyl and hexahydro-1H-pyrrolizinyl are substituted with 1, 2 or 3 Rₑ.

15. The compound of any one of claims 1, 13 and 14, or a pharmaceutically acceptable salt thereof, wherein R₄ is selected from

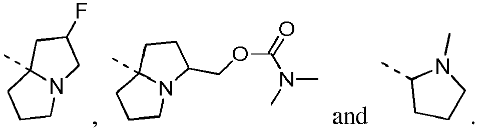

16. A compound or a pharmaceutically acceptable salt thereof, wherein the compound is selected from

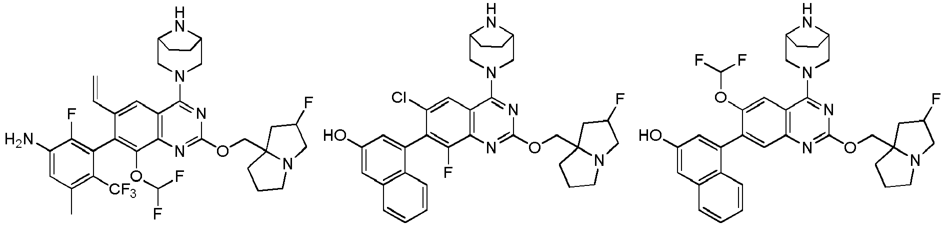

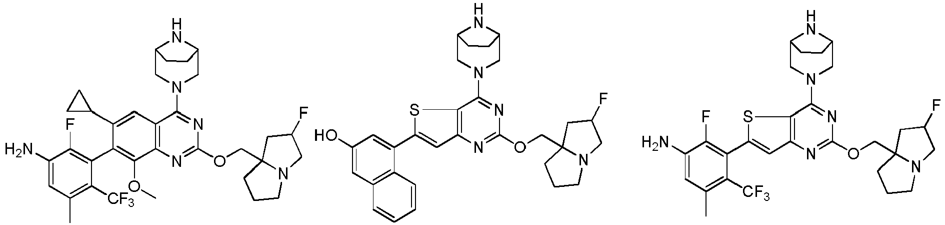

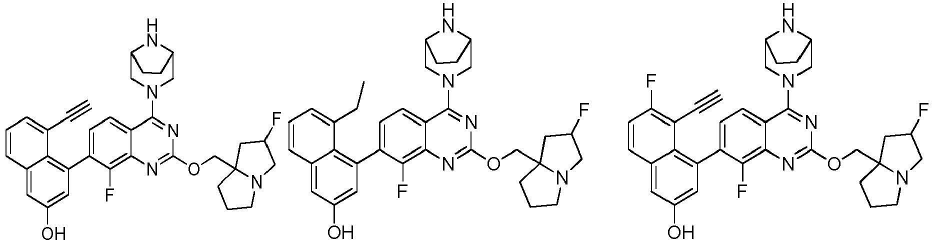

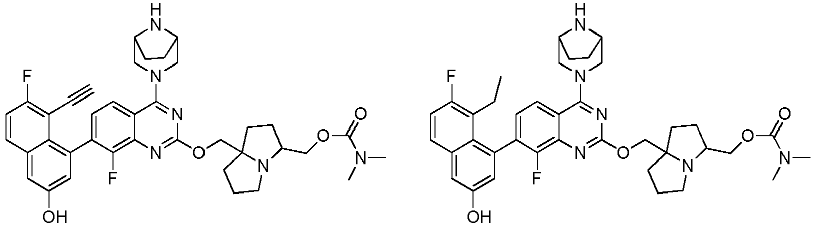

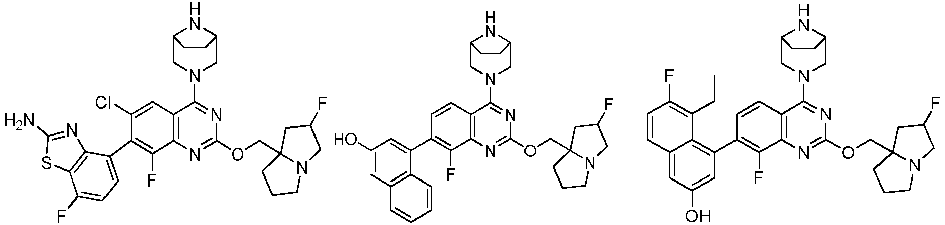

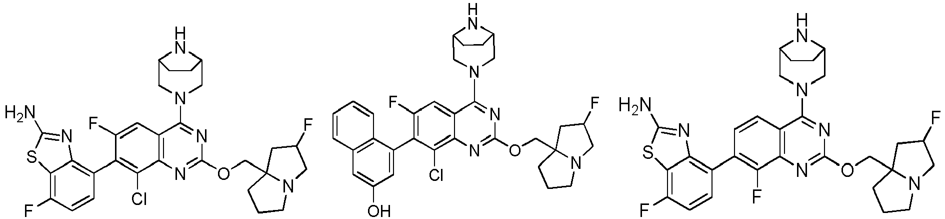

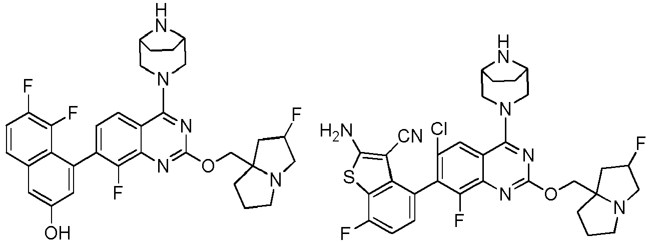

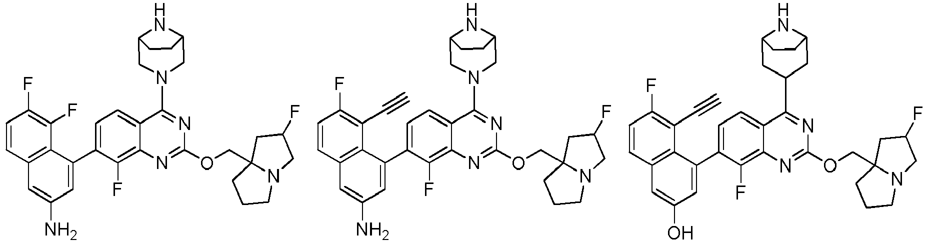

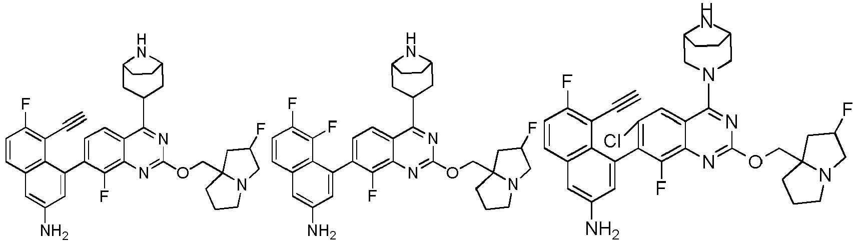

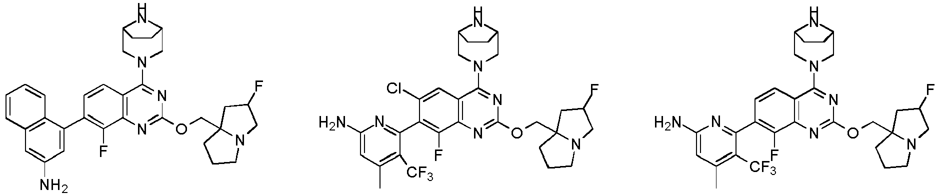

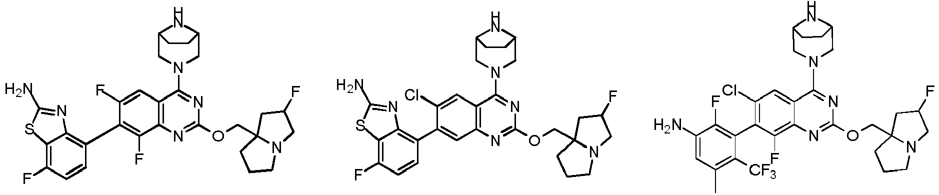

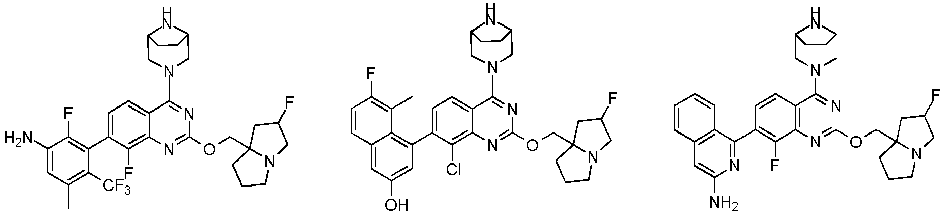

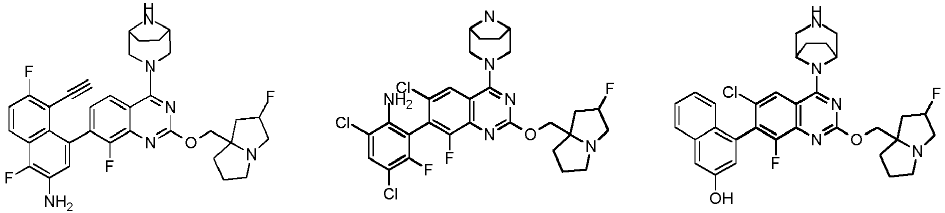

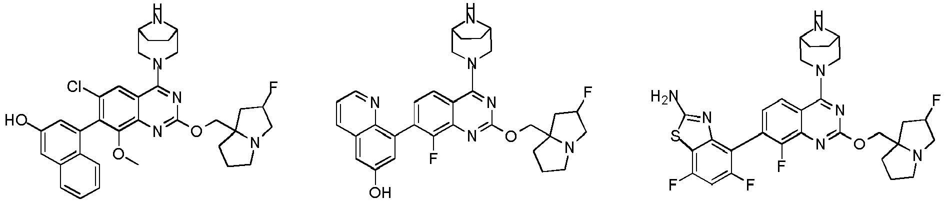

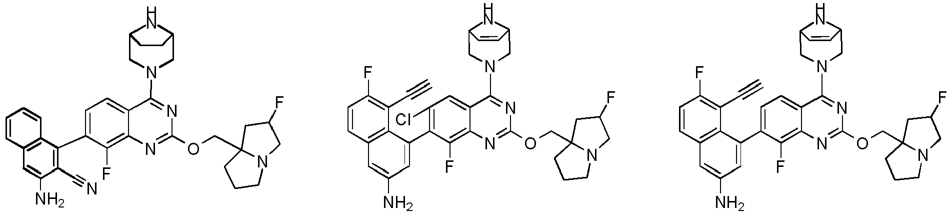

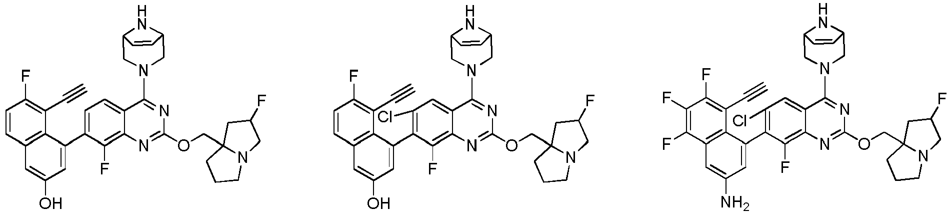

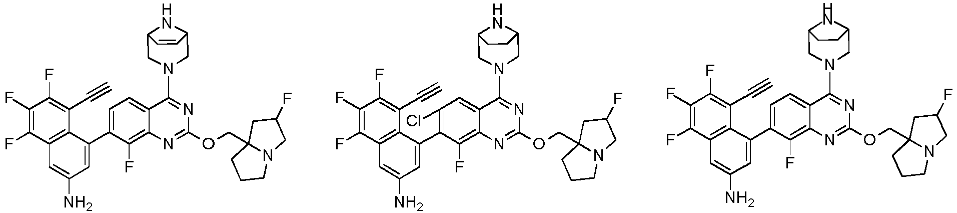

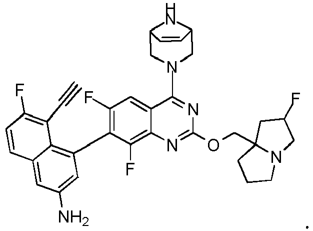

17. The compound of claim 16, or a pharmaceutically acceptable salt thereof, wherein the compound is selected from

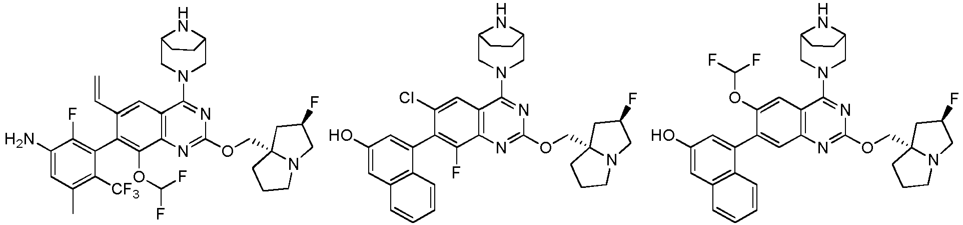

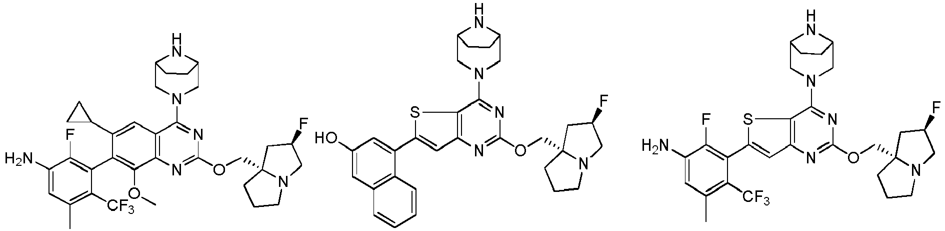

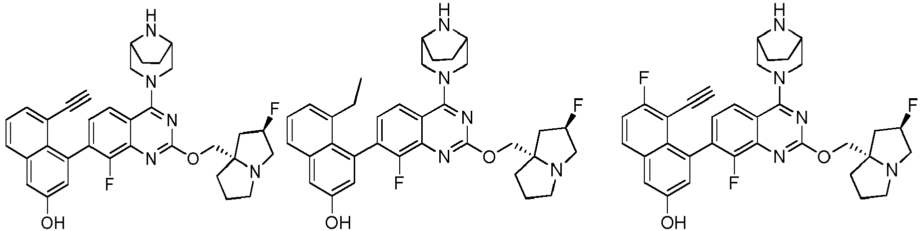

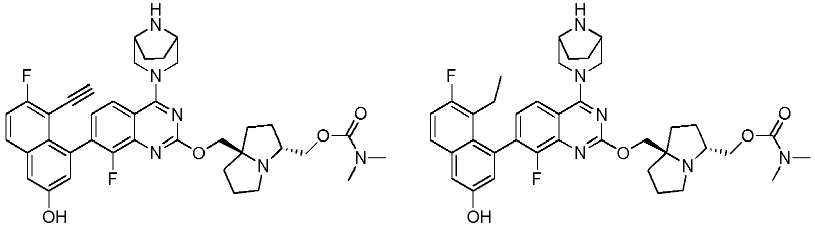

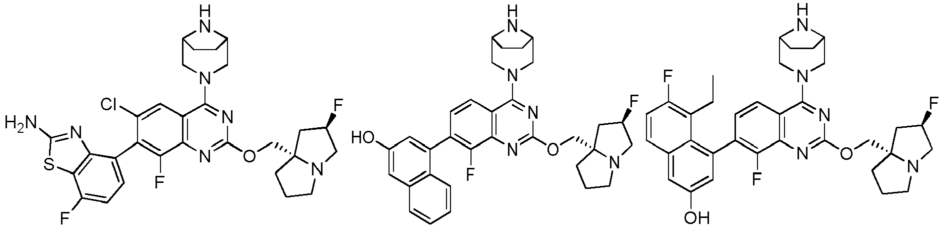

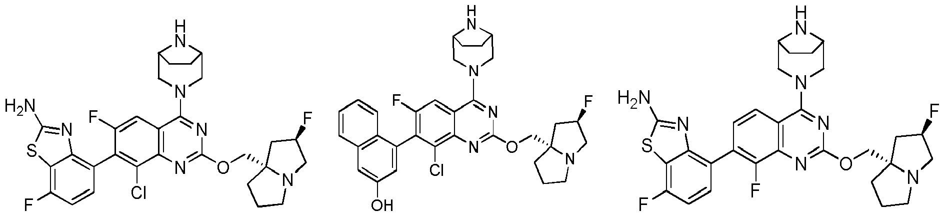

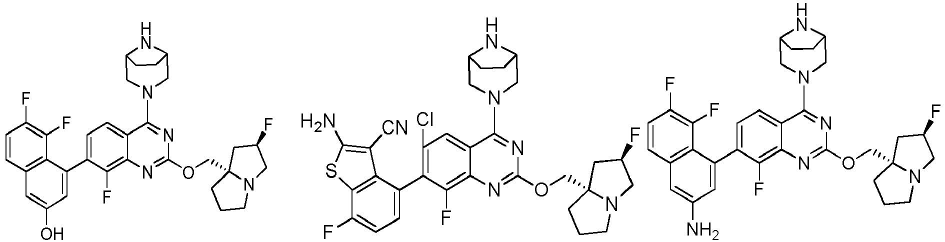

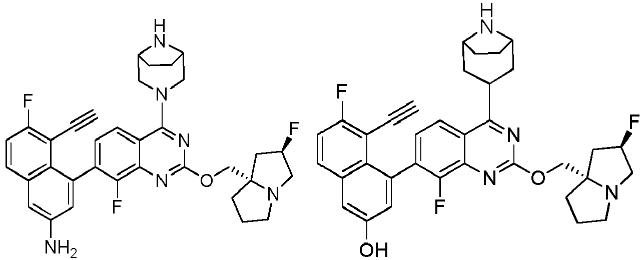

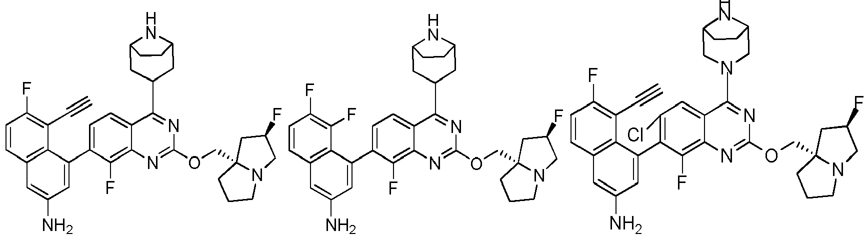

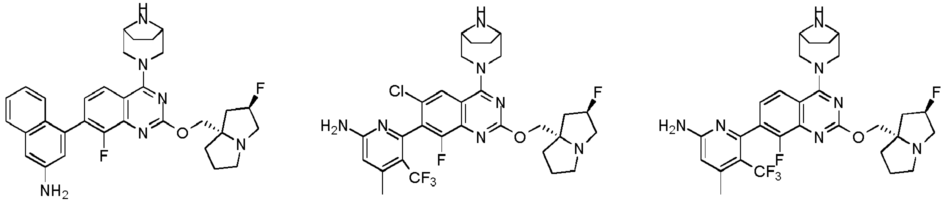

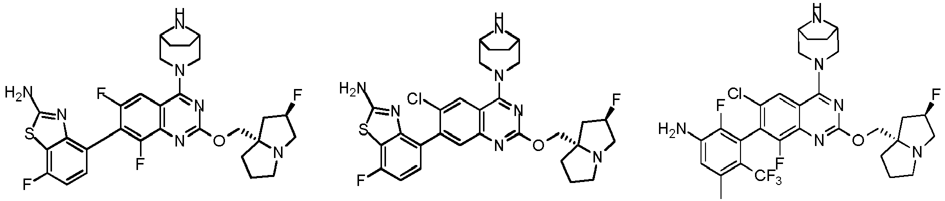

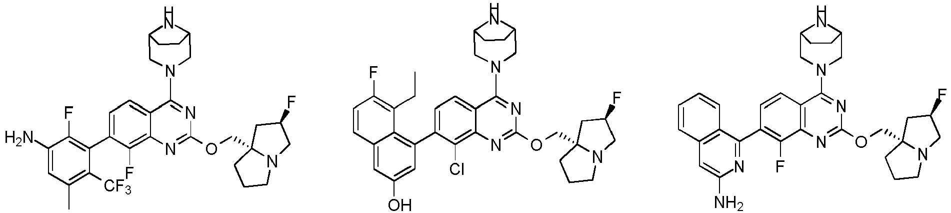

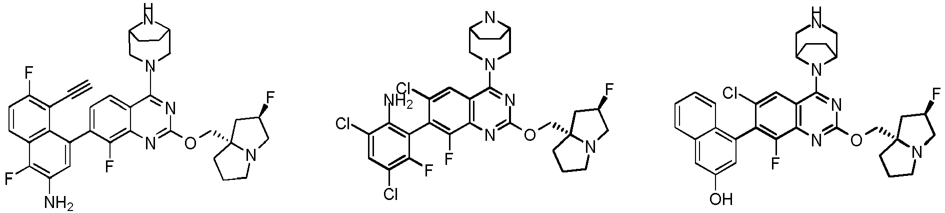

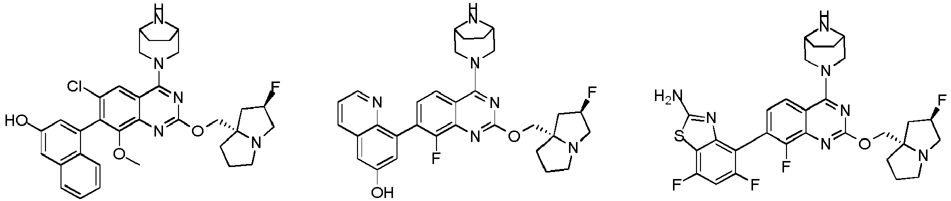

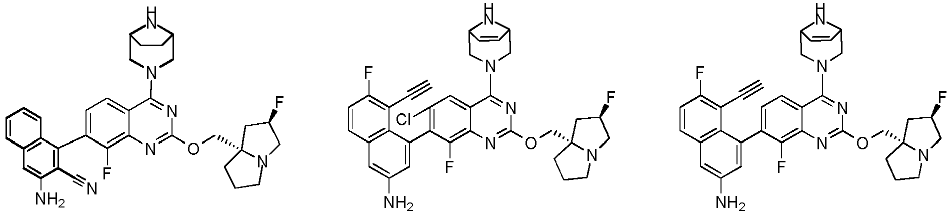

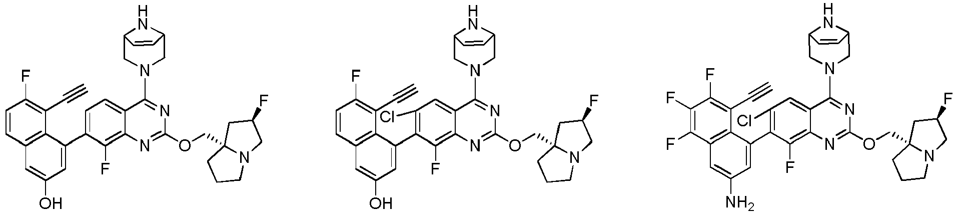

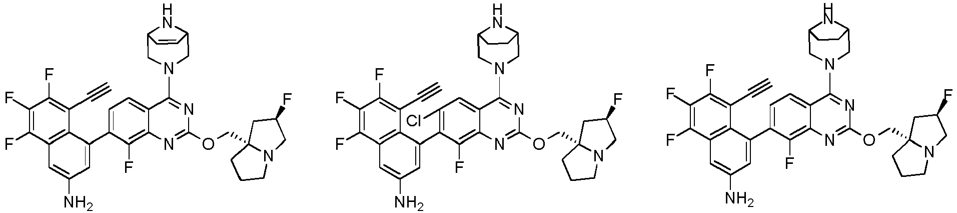

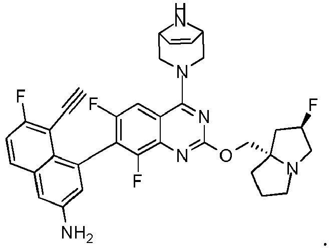

18. The compound of claim 16, or a pharmaceutically acceptable salt thereof, wherein the compound is selected from

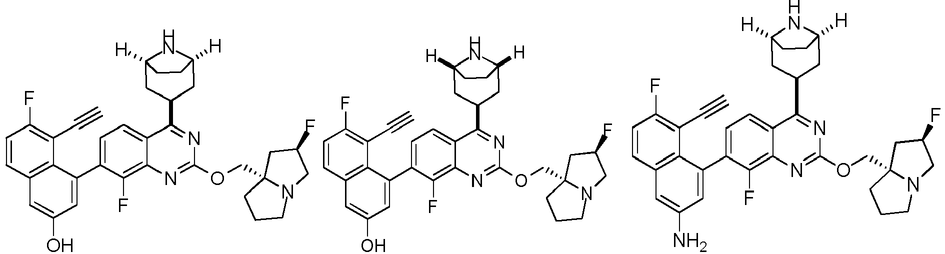

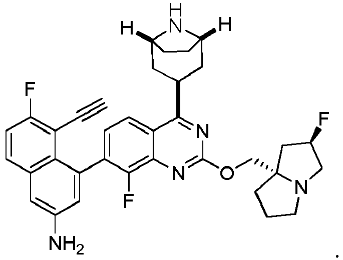

19. Use of the compound of any one of claims 1-18, or a pharmaceutically acceptable salt thereof in the manufacture of a medicament for treating KRAS^{G12D} mutation-related tumors.

20. The use of claim 19, wherein the tumors refer to colorectal cancer and pancreatic cancer.
